# EUROPEAN PATENT APPLICATION

(11) **EP 4 534 134 A1**
(43) Date of publication of application: **09.04.2025**
(21) Application number: 23811158.7
(22) Date of filing: 26.05.2023
(51) Int. Cl.: A61M 25/14

(54) **INTERVENTIONAL CATHETER, INTERVENTIONAL DEVICE, ATOMIZATION DRUG DELIVERY SYSTEM, CONTROL METHOD, ATOMIZATION METHOD, AND TREATING MEDIUM SPRAY SYSTEM AND METHOD**

(30) Priority: 26.05.2022 CN 202210590562
(71) Applicant: Hangzhou Broncus Medical Co., Ltd., Hangzhou, Zhejiang 310051 (CN)
(72) Inventor: XU, Hong, Hangzhou, Zhejiang 310051 (CN); WANG, Maoqiang, Hangzhou, Zhejiang 310051 (CN); XU, Pengwei, Hangzhou, Zhejiang 310051 (CN)
(74) Representative: Zaboliene, Reda
(86) International application number: PCT/CN2023/096430
(87) International publication number: WO 2023/227088

(57) **Abstract**

The present application relates to an interventional catheter, an interventional device, a nebulized medication delivery system, a control method, a nebulization method, and spraying system and method of treating medium. The interventional catheter includes a catheter body, wherein one end of the catheter body is a proximal end, and another end is a distal end that can extend into the bronchus. A flow channel that can deliver a fluid from the proximal end to the distal end is provided in the catheter body. A flow mixing structure is provided at the distal end of the catheter body. A microfluidic chip is provided in the flow mixing structure. A chip channel structure that inter connects an outlet with each fluid inlets is provided in the microfluidic chip. The chip channel structure includes: a main channel with one end connected to with a first fluid inlet at a proximal end side and another end connected to the outlet, and a lateral channel with one end connected to a second fluid inlet on a lateral side and another end connected to the main channel; a distribution structure that can act on at least a portion of the fluid being provided in the chip channel structure. The distribution structure that can act on at least a portion of the fluid is provided in the microfluidic chip. **In** the present application, the delivered fluid is nebulized by the microfluidic chip, which improves the nebulization effect.

## Description

### TECHNICAL FIELD

This application relates to the technical field of nebulization, and in particular to an interventional catheter, an interventional device, a nebulized medication delivery system, a control method, a nebulization method, and spraying system and method of treating medium.

### BACKGROUND

Generally, a nebulizer converts liquid into micron-sized liquid particles. During clinical endoscopic treatment, it is usually necessary to cooperate with nebulization administration to ensure that the injected medication can be evenly distributed, thereby improving the uniformity of the combination of related tissues and the nebulized medication. Liquid input by the nebulizer may include liquids for treatment or recovery. Currently, the nebulizer is configured for patients to administer medication by inhalation, wherein the liquid is broken down into an aerosol of tiny particles or droplets, so that the patients using the medication can obtain more efficient inhalation and absorption. However, during the inhalation administration process, it is difficult for the medication to fully reach the lesions, resulting in unnecessary losses. Not only that, there is another problem with inhalation administration: before reach the lesions the medication may cause damage to the ductal organs along the way due to its own side effects, posing a potential safety risk.

The existing nebulizer has poor nebulization effect, its structure needs to be modified, and its applicability is limited.

### SUMMARY

Based on this, for solving the above technical problem, this application discloses an interventional catheter including a catheter body, one end of the catheter body being configured as a proximal end, another end of the catheter body being configured as a distal end that is capable of extending into a bronchus, an interior of the catheter body being configured with a flow channel that is capable of transporting a fluid from the proximal end to the distal end, a mixing structure being provided at the distal end of the catheter body, wherein the fluid in the flow channel is output after being mixed by the mixing structure;
the fluid mixing structure at least including a first fluid inlet, a second fluid inlet and an outlet, wherein each fluid inlet is independently configured with a flow channel in the catheter body;
a microfluidic chip being provided in the fluid mixing structure, wherein the first fluid inlet is located at a proximal end side of the microfluidic chip, the second fluid inlet is located at a lateral side of the microfluidic chip, an interior of the microfluidic chip is configured with a chip channel structure which interconnects the outlet and each fluid inlet, and wherein the chip channel structure includes:
   a main channel with one end thereof being connected to the first fluid inlet at the proximal end side and another end thereof being connected to the outlet;
   a lateral channel with one end thereof being connected to the second fluid inlet at the lateral side and another end thereof being connected to the main channel; and
   a distribution structure capable of acting on at least a portion of the fluid being arranged in the chip channel structure.

Optionally, the distribution structure is located at an intersection position of the main channel and the lateral channel.

Optionally, a channel width of the lateral channel includes a narrowing part.

Optionally, the lateral channel has an extension trend line, and a channel width of the lateral channel gradually narrows along the extension trend line.

Optionally, a width of the lateral channel decreases continuously from upstream to downstream.

Optionally, intersection positions of the lateral channel and the main channel are configured as junctions, and an orientation of at least one junction is perpendicular to an extending direction of the main channel.

Optionally, a distribution structure which can acts on at least a portion of the fluid to pre-disperse this portion of fluid is provided in chip channel structure.

Optionally, the distribution structure is configured as convex columns, which are remaining solid parts after etching to form the microfluidic chip.

Optionally, the gas-phase fluid is pre-dispersed by the distribution structure and then mixed with the liquid-phase fluid.

Optionally, the main channel and the lateral channel supply fluids in different phases, respectively, and a feed pressure of each fluid is greater than 0.1MPa.

Optionally, all of the chip channels are arranged in a coplanar manner.

Optionally, only one outlet is configured for outputting nebulized fluid.

Optionally, intersection positions of the lateral channel and the main channel are configured as junctions, and an orientation of at least one junction is perpendicular to an extending direction of the main channel.

Optionally, there are two lateral channels arranged at two sides of the main channel, respectively, at least a portion of each lateral channel intersects with the main channel at a position adjacent to the outlet, and an obtuse angle is formed between extension trend lines of the two lateral channels at intersection positions thereof.

Optionally, the two lateral channels are configured with inlets on different sides of the microfluidic chip, respectively.

Optionally, there are two second fluid inlets which are symmetrically distributed on two opposite lateral sides of the microfluidic chip.

Optionally, the distribution structure is configured as at least one of the following manners:
Manner a: a plurality of branch channels connected to the lateral channel, wherein the lateral channel intersects with the main channel via the plurality of branch channels;
Manner b: a plurality of distribution members which are arranged at intervals or are separately provided in the main channel and/or the lateral channel.

Optionally, the distribution structure is configured as the distribution members located in the main channel and/or the lateral channel, and the distribution members guide a flowing direction of the fluid of one of the main channel and lateral channel in which the distribution members are located to be perpendicular to a flowing direction of the fluid in another one of the main channel and lateral channel.

Optionally, a portion of the main channel is configured as a widened expansion section, and at least a portion of the lateral channel intersects with the main channel at the expansion section, the distribution members are located in the expansion section and include at least a first distribution surface and a second distribution surface facing to a flowing direction of the fluid in the main channel.

Optionally, a fluid acceleration structure is provided in the main channel and/or the lateral channel, the fluid acceleration structure is located upstream of at least one intersection position of the main channel and the lateral channel in which at least one narrow opening is provided, and the main channel intersects with the lateral channel through the narrow opening.

Optionally, a sudden expansion section is provided in the main channel and/or the lateral channel and downstream of the fluid acceleration structure, an internal volume of the sudden expansion section is at least 15% larger than that of the fluid acceleration structure, and the intersection position with the narrow opening is arranged in the sudden expansion section.

Optionally, the sudden expansion section is arranged close to the fluid acceleration structure, and a chamber diameter of the sudden expansion section is at least 10% larger than that at an outlet of the fluid acceleration structure.

Optionally, there are two lateral channels arranged at two sides of the main channel, respectively, the fluid acceleration structure and the sudden expansion section are configured in groups and arranged in corresponding lateral channels, respectively, and two of the sudden expansion sections are configured to intersect in the main channel.

Optionally, a distribution member is provided in the main channel, and at least two of the narrow openings are formed by the distribution member and side walls of the main channel and correspond to two expanded sections, respectively.

The present application further discloses a high-viscosity fluid spraying method for natural cavity of human body, wherein the fluid includes a first fluid and a second fluid, one of the first fluid and the second fluid is a liquid-phase fluid, and the other one of the first fluid and the second fluid is a gas-phase fluid, and the high-viscosity fluid is in the liquid-phase fluid;
wherein the first fluid and the second fluid are nebulized into droplets with a particle size less than or equal to 60µm after at least two collisions, and the droplets contain inactivated viruses and move to a lesion location to infiltrate the lesion.

Optionally, the particle size of nebulized droplets is larger than or equal to 20µm, a pressure of the liquid-phase fluid is 0.2~0.76MPa, and a pressure of the gas-phase fluid is 0.1~0.4MPa.

Optionally, the particle size of nebulized droplets is in range of 20~60µm.

The present application further discloses a high-viscosity fluid spraying system for natural cavity of human body, including an interventional catheter and a perfusion device for supplying a first fluid and a second fluid into the interventional catheter, respectively, wherein
the interventional catheter includes a catheter body, one end of the catheter body is configured as a proximal end and another end of the catheter body is configured as a distal end that can extend into a bronchus, an interior of the catheter body is provided with a flow channel that is capable of transporting fluid from the proximal end to the distal end, a mixing structure is provided at the distal end of the catheter body, the fluid in the flow channel is output after being mixed by the mixing structure;
a flow mixing structure at least includes a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure includes:
   a main channel, one end of which is connected to the first fluid inlet at the proximal end side and another end of which is connected to the outlet;
   a lateral channel, one end of which is connected to a second fluid inlet at the lateral side and another end of which is connected to the main channel; and
   a distribution structure capable of acting on at least a portion of the fluid being arranged in the chip channel structure.

Optionally, a droplets size of the nebulized fluid is larger than or equal to 20µm, one of the first fluid and the second fluid is a liquid-phase fluid, and the other one of the first fluid and the second fluid is a gas-phase fluid, a pressure of the liquid-phase fluid is 0.2~0.76MPa, and a pressure of the gas-phase fluid is 0.1~0.4MPa.

Optionally, the lateral channel is configured with a fluid acceleration structure which is located upstream of at least one intersection position of the main channel and the lateral channel.

Optionally, the fluid in the lateral channel is gas, and the fluid acceleration structure is a Laval nozzle that is configured to converge gradually and expand gradually.

Optionally, a portion of the main channel is a widened expansion section, at least a portion of the lateral channel intersects with the expansion section, a distribution member is provided in the expansion section, and the distribution member is provided with at least a first distribution surface and a second distribution surface facing towards a flowing direction of the fluid in the main channel.

Optionally, the main channel is provided with a throat that is narrower than other portion of the main channel at the upstream and downstream of the throat, and a width W7 of the throat of the main channel is 0.1mm to 0.2mm.

Optionally, a width W8 of a droplets outlet is 0.15mm to 0.3mm.

Optionally, an etched depth H3 is 0.1mm to 0.25mm.

Optionally, a thickness H1 of a unit chip with etched flow channel is 0.25mm to 0.55mm.

Optionally, the high-viscosity fluid includes a hydrogel.

The present application further discloses a cell spraying system for natural cavity of human body, including an interventional catheter and a perfusion device for supplying a first fluid and a second fluid to the interventional catheter, respectively, wherein
the interventional catheter includes a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least includes a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure includes:
   a main channel, one end of which is connected to the first fluid inlet at the proximal end side and another end of which is connected to the outlet;
   a lateral channel, one end of which is connected to a second fluid inlet at the lateral side and another end of which is connected to the main channel; and
   a distribution structure capable of acting on at least a portion of the fluid being arranged in the chip channel structure.

Optionally, a particle size of the cells is 15~120µm, one of the first fluid and the second fluid is a liquid-phase fluid and the other one of the first fluid and the second fluid is a gas-phase fluid, a pressure of the liquid-phase fluid is 0.2~0.76MPa, and a pressure of the gas-phase fluid is 0.1~0.4MPa.

Optionally, a fluid acceleration structure and a sudden expansion section are provided in the main channel and/or the lateral channel and arranged in sequence along a flowing direction of the fluid, an internal volume of the sudden expansion section is at least 15% larger than that of the fluid acceleration structure, and the fluids in the main channel and lateral channel meet and are nebulized in the sudden expansion section.

Optionally, the lateral channel is configured for transporting liquid, a distribution structure is provided at an end of the lateral channel close to the main channel, the distribution structure is configured as distribution members arranged in an array, and a cross-sectional size of each distribution member gradually increases along the flowing direction of the fluid.

Optionally, an etched depth H3 is 0.08mm to 0.12mm.

Optionally, a width W8 of a droplets outlet is 0.1mm to 0.2mm.

Optionally, a width W1 of the main channel is 0.2mm to 0.4mm.

Optionally, a particle size of droplets ranges from 5µm to 50µm.

Optionally, the cells include exosomes.

The present application further discloses a medication spraying method for natural cavity of human body, including a first fluid and a second fluid, wherein one of the first fluid and the second fluid is a liquid-phase fluid and the other one of the first fluid and the second fluid is a gas-phase fluid, the medication is in the liquid-phase fluid, and a particle size of the medication is less than or equal to 69000nm;
wherein the first fluid and the second fluid are nebulized into droplets with a particle size less than or equal to 300µm after at least two collisions, and the droplets contain inactivated viruses and move to a lesion location to infiltrate the lesion.

Optionally, a particle size of the medication is less than 69µm, a pressure of the liquid-phase fluid is 0.2~0.76MPa, and a pressure of the gas-phase fluid is 0.2~0.6MPa.

Optionally, the particle size of the medication is less than 600nm.

Optionally, the particle size of nebulized droplets is in range of 8~30µm.

The present application further discloses a medication spraying system for natural cavity of human body, including an interventional catheter and a perfusion device for supplying a first fluid and a second fluid to the interventional catheter, respectively, wherein
the interventional catheter includes a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least includes a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure includes:
   a main channel, one end of which is connected to the first fluid inlet at the proximal end side and another end of which is connected to the outlet;
   a lateral channel, one end of which is connected to a second fluid inlet at the lateral side and another end of which is connected to the main channel; and
   a distribution structure capable of acting on at least a portion of the fluid being arranged in the chip channel structure.

Optionally, one of the first fluid and the second fluid is a liquid-phase fluid, and the other one of the first fluid and the second fluid is a gas-phase fluid, the medication is in the liquid-phase fluid, a particle size of the medication is less than 69µm, a pressure of the liquid-phase fluid is 0.2~0.76MPa, and a pressure of the gas-phase fluid is 0.2~0.6MPa.

Optionally, the lateral channel and the main channel intersect with each other at least twice.

Optionally, a portion of the main channel is configured as a widened expansion section, the lateral channel intersects with the main channel at least once at the expansion section, and the lateral channel intersects with the main channel at least once downstream of the expansion section.

Optionally, a width W1 of the main channel is 0.2 to 0.5mm.

Optionally, the main channel is provided with a throat that is narrower than other portion of the main channel at the upstream and downstream of the throat, and a width W7 of the throat of the main channel is less than the width W1 of the main channel, and the width W7 of the throat of the main channel is preferably 0.05~0.15mm.

Optionally, a width W8 of a droplets outlet deviates from the width W7 of the throat of the main channel by 5%~15%.

The present application further discloses an inactivated viruses spraying method for natural cavity of human body, including a first fluid and a second fluid, wherein one of the first fluid and the second fluid is a liquid-phase fluid and the other one of the first fluid and the second fluid is a gas-phase fluid, the inactivated virus is in the liquid-phase fluid, and a particle size of the inactivated virus is less than or equal to 21000nm;
the first fluid and the second fluid are nebulized into droplets with a particle size less than or equal to 300µm after at least two collisions, and the droplets contain inactivated viruses and move to a lesion location to infiltrate the lesion.

Optionally, a particle size of the inactivated virus is less than 21µm, a pressure of the liquid-phase fluid is 0.2~0.76MPa, and a pressure of the gas-phase fluid is 0.2~0.6MPa.

Optionally, the particle size of the inactivated virus is less than 600nm.

Optionally, a particle size of nebulized droplets is in range of 5~40µm.

The present application further discloses an inactivated virus spraying system for natural cavity of human body, including an interventional catheter and a perfusion device for supplying a first fluid and a second fluid, to the interventional catheter, respectively, wherein
the interventional catheter includes a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least includes a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure includes:
   a main channel, one end of which is connected to the first fluid inlet at the proximal end side and another end of which is connected to the outlet;
   a lateral channel, one end of which is connected to a second fluid inlet at the lateral side and another end of which is connected to the main channel; and
   a distribution structure capable of acting on at least a portion of the fluid being arranged in the chip channel structure.

Optionally, one of the first fluid and the second fluid is a liquid-phase fluid and the other one of the first fluid and the second fluid is a liquid-phase fluid is a gas-phase fluid, the inactivated virus is in the liquid-phase fluid, a particle size of the inactivated virus is less than 21µm, a pressure of the liquid-phase fluid is 0.2~0.76MPa, and a pressure of the gas-phase fluid is 0.2~0.6MPa.

Optionally, there are two lateral channels provided at two sides of the main channel, respectively, each lateral channel is configured with an independent branch channel which includes multiple sub-channels arranged in an array, the multiple sub-channels of two branch channels are arranged opposite to each other and connected to the main channel, and wherein the multiple sub-channels of the two branch channels are arranged at intervals and staggered.

Optionally, a width W8 of a droplets outlet is 0.1~0.2mm.

The present application further discloses a nebulization method based on interventional catheter, wherein a microfluidic chip is provided at a distal end of the interventional catheter, and the nebulization method includes:
supplying a first fluid and a second fluid into the interventional catheter respectively, wherein at least one of the first fluid and second fluid is loaded with a therapeutic substance;
flowing of the first fluid and second fluid into the microfluidic chip from the proximal end side and the lateral side of the microfluidic chip, respectively, wherein at least one of the first fluid and second fluid is pre-dispersed in the microfluidic chip and then mixed with the other one of the first fluid and second fluid and nebulized, and then output to a lesion position;
obtaining current state parameters of the fluid during the nebulization process and generating a corresponding control signal based on the state parameters;
regulating a supply of the fluid accordingly to the control signal.

The present application further discloses a nebulized medication delivery system, including an interventional catheter and a perfusion device for supplying a first fluid and a second fluid to the interventional catheter, respectively, wherein
the interventional catheter includes a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least includes a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure includes:
   a main channel, one end of which is connected to the first fluid inlet at the proximal end side and another end of which is connected to the outlet;
   a lateral channel, one end of which is connected to a second fluid inlet at the lateral side and another end of which is connected to the main channel; and
   a distribution structure capable of acting on at least a portion of the fluid being arranged in the chip channel structure.

The present application further discloses a control method for nebulized medication delivery system, wherein the nebulized medication delivery system includes an interventional catheter, a microfluidic chip is provided at a distal end of the interventional catheter, and the control method includes:
Step S510, supplying a gas-phase fluid first when the nebulized medication delivery system is started, wherein an adjustment target of a pressure of the gas-phase fluid is divided into at least two stages, each stage is independently configured with a PID parameter; collecting a first real-time pressure of the gas-phase fluid when the gas-phase fluid is supplied, determining the adjustment target stage to which the first real-time pressure belongs, and adjusting the pressure of the gas-phase fluid based on the PID parameter corresponding to the adjustment target stage to which the first real-time pressure belongs;
Step S520, supplying a gas-phase fluid with a therapeutic substance, wherein the liquid-phase fluid and the gas-phase fluid enter the microfluidic chip from a proximal end side and a lateral side of the microfluidic chip, respectively, at least one of the liquid-phase fluid and gas-phase fluid is pre-dispersed in the microfluidic chip and then mixed with another one of the liquid-phase fluid and gas-phase fluid and nebulized, and then output to a lesion position;
Step S530, collecting a second real-time pressure of the gas-phase fluid during nebulization and output of the fluid, determining the adjustment target stage to which the second real-time pressure belongs, and adjusting the pressure of the gas-phase fluid based on the PID parameter corresponding to the adjustment target stage to which the second real-time pressure belongs; and
Step S540, looping step S530 at a preset interval.

The present application further discloses a control method for nebulized medication delivery system, including:
pre-setting delivery parameters of a liquid medium and generating a liquid medium delivery map according to the preset delivery parameters of the liquid medium;
generating a corresponding driving signal according to the liquid medium delivery map for driving a motor, wherein the motor is used to drive a piston of a syringe to move to realize liquid delivery;
collecting an encoder signal set on the motor, obtaining a running position of the motor, and calculating the moving position of the piston through the running position of the motor;
recording the moving position of the piston and calculating an actual delivery map based on the moving position information of a plurality of pistons;
comparing the liquid medium delivery map with the actual delivery map according to a preset period: when the difference is less than a preset value, retaining the original liquid medium delivery map; when the difference is greater than or equal to the preset value, correcting the original liquid medium delivery map according to preset rules and generating a new liquid medium delivery map to replace the original liquid medium delivery map;
updating the liquid medium delivery map continuously until the liquid medium delivery is finished or paused.

The present application further discloses an interventional catheter, including a catheter body, wherein one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least includes a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure includes:
   a main channel, one end of which is connected to the first fluid inlet at the proximal end side and another end of which is connected to the outlet;
   a lateral channel, one end of which is connected to a second fluid inlet at the lateral side and another end of which is connected to the main channel; and
   a fluid acceleration structure and a sudden expansion section being provided in the main channel and/or the lateral channel and arranged in sequence along a flowing direction of the fluid, an internal volume of the sudden expansion section being at least 15% larger than that of the fluid acceleration structure, and the fluids in the main channel and lateral channel meeting and nebulized in the sudden expansion section.

Optionally, the sudden expansion section is formed by an expansion of a flow channel in which the sudden expansion section is located, and a smooth transition or a stepped transition is formed between the sudden expansion section and an outlet of the fluid acceleration structure.

Optionally, in a flowing direction of a corresponding flow channel, an expansion trend of the sudden expansion section increases, maintains or shrinks compared to an expansion trend of the fluid acceleration structure.

Optionally, the sudden expansion section is arranged close to the fluid acceleration structure, and a chamber diameter of the sudden expansion section is at least 10% larger than that of the outlet of the fluid acceleration structure.

Optionally, at least one narrow opening is provided at an intersection position of the main channel and the lateral channel, and the main channel and the lateral channel intersect with each other through the narrow opening.

Optionally, there are two lateral channels arranged at two sides of the main channel, respectively, the fluid acceleration structure and the sudden expansion section are configured in groups and arranged in corresponding lateral channels, respectively, and two of the sudden expansion sections are configured to intersect in the main channel.

Optionally, a distribution structure capable of acting on at least a portion of the fluid is provided in the chip channel structure, wherein the distribution structure is configured as distribution members located in the main channel and/or the lateral channel, and the distribution members guide a flowing direction of the fluid of one of the main channel and lateral channel in which the distribution members are located to be perpendicular to a flowing direction of the fluid in another one of the main channel and lateral channel.

Optionally, distribution members are provided in the main channel, and at least two of the narrow openings are formed by the distribution members and side walls of the main channel and correspond to two expanded sections, respectively.

Optionally, there are two lateral channels provided at two sides of the main channel, respectively, each lateral channel is configured with an independent branch channel which includes multiple sub-channels arranged in an array.

Optionally, the multiple sub-channels of two branch channels are arranged opposite to each other and connected to the main channel.

Optionally, the multiple sub-channels of two branch channels are arranged in a staggered manner.

The present application further discloses an interventional catheter, including:
a catheter body with one end being configured as a proximal end and another end being configured as a distal end that is capable of extending into a bronchus;
a microfluidic chip being configured with a chip channel structure therein, wherein the chip channel structure includes a main channel and a lateral channel, and fluids in the main channel and the lateral channel cooperate with each other to output nebulized fluid;
a heating module, located in the catheter body and at a distal end of the microfluidic chip, for adjusting a temperature of the nebulized fluid;
a balloon being deformably arranged outside the catheter body.

Optionally, the balloon and the heating module are positioned correspondingly in an axial direction of the catheter body.

Optionally, the distal end of the microfluidic chip is configured with an outlet for outputting the nebulized fluid, and the heating module is a heating tube, wherein an interior of the heating tube is a temperature control channel connected to the outlet.

Optionally, a ratio of an inner diameter and an axial length of the heating tube is in range of 0.05 to 0.3.

Optionally, a fluid acceleration structure and a sudden expansion section are provided in the main channel and/or the lateral channel and arranged in sequence along a flowing direction of the fluid, and fluids in the main channel and the lateral channel interact with each other in the sudden expansion section to output nebulized fluid.

Optionally, a liquid medium configured for nebulization in the microfluidic chip is water or saline and without any therapeutic medium.

Optionally, a fluid acceleration structure and a sudden expansion section are provided in the main channel and/or the lateral channel and arranged in sequence along a flowing direction of the fluid, and fluids in the main channel and the lateral channel interact with each other in the sudden expansion section to output nebulized fluid.

Optionally, the microfluidic chip includes:
a first fluid inlet located at a proximal end side of the microfluidic chip;
a second fluid inlet located at a lateral side the microfluidic chip; and
an outlet located at a distal end side of the microfluidic chip;
wherein one end of the main channel is connected to the first fluid inlet and another end of the main channel is connected to the outlet; one end of the lateral channel is connected to the second fluid inlet and another end of the lateral channel is connected to the main channel; and a distribution structure which is capable of acting on at least a portion of the fluid to pre-disperse this portion of the fluid is provided in the chip channel structure.

Optionally, an internal volume of the sudden expansion section is at least 15% larger than that of the fluid acceleration structure, and the main channel and the lateral channel are connected in the sudden expansion section with a connection position being adjacent to the fluid acceleration structure.

Optionally, the fluid acceleration structure is formed by a gradually contracting and expanding of a flow channel in which the fluid acceleration structure is located.

Optionally, the sudden expansion section is formed by an expansion of the flow channel in which the sudden expansion section is located, and the sudden expansion section and an outlet of the fluid acceleration structure have a smooth transition or a step transition.

Optionally, the sudden expansion section is arranged close to the fluid acceleration structure, and a chamber diameter of the sudden expansion section is at least 10% larger than that of the outlet of the fluid acceleration structure.

Optionally, the main channel and the lateral channel intersect with each other at least twice along an extending direction of the main channel, wherein a first intersection is at the middle and upper reaches of the main channel for implementing turbulence, and a second intersection is adjacent to the outlet for implementing nebulization.

Optionally, there are two lateral channels respectively provided at two sides of the main channel, and a distribution structure is provided in the main channel, and the distribution structure and the side wall of the main channel form two narrow openings, and each narrow opening corresponds to the expanded section connecting the two lateral channels to form the second intersection.

The present application further discloses that there are two lateral channels arranged at two sides of the main channel, respectively, the fluid acceleration structure and the sudden expansion section are configured in groups and arranged in corresponding lateral channels, respectively, and two of the sudden expansion sections are configured to intersect in the main channel.

The present application further discloses a nebulized medication delivery system, including:
a perfusion device for supplying a fluid containing therapeutic substance, wherein the fluid includes a liquid-phase fluid and a gas-phase fluid, and at least one of the liquid-phase fluid and gas-phase fluid contains the therapeutic substance;
an interventional catheter mentioned above, wherein a proximal end of the interventional catheter is connected to a perfusion device for receiving the gas-phase fluid and the liquid-phase fluid, respectively;
a sampling device for collecting state parameters of the fluids;
a controlling device connected to the sampling device, configured for receiving the state parameters and controlling the perfusion device according to the state parameters.

The present application further discloses a gas-liquid interventional device, including:
a housing, on which a gas path connector and a liquid path connector are installed, a pump chamber being provided at a bottom of the housing;
an air pump installed in the pump chamber, wherein the air pump includes a plurality of pumps connected in parallel and is connected to the gas path connector through a main pipe;
a cooling assembly thermally coupled to the air pump;
a syringe including a cylinder installed in a housing and a piston slidably installed in the cylinder, wherein the cylinder has an outlet and is connected to the liquid path connector;
a drive mechanism, linked to the piston of the syringe;
an interventional catheter mentioned above, wherein a proximal end of the interventional catheter is connected to the gas path connector and the liquid path connector, respectively, for receiving the gas-phase fluid and the liquid-phase fluid, respectively.

The present application further discloses a interventional catheter, including a catheter body, wherein one end of the catheter body is configured as a proximal end, and another end of the catheter body is configured as a distal end that is capable of extending into a bronchus, an interior of the catheter body is provided with a microfluidic chip, wherein the microfluidic chip includes:
a first fluid inlet located at a proximal end side of the microfluidic chip;
a second fluid inlet located at a lateral side of the microfluidic chip; and
an outlet located at a distal end side of the microfluidic chip;
wherein an interior of the microfluidic chip is configured as a chip channel structure which interconnects the outlet and each fluid inlet, and the chip channel structure includes:
   a main channel, one end of which is connected to the first fluid inlet and another end of which is connected to the outlet; and
   a lateral channel with one end thereof being connected to the second fluid inlet and another end thereof being connected to the main channel, wherein the lateral channel is configured with a branch channel and intersects with the main channel via the branch channel.

Optionally, the lateral channel includes:
a trunk channel extending from the second fluid inlet towards the distal end until it intersects with the main channel;
the branch channel with one end thereof being connected to the trunk channel at a position being adjacent to the second fluid inlet and another end thereof extending toward the proximal end and branching into a plurality of sub-channels, distal ends of the sub-channels intersecting with each other at the middle and upper reaches of the main channel.

Optionally, the branch channel has a bending portion at the second fluid inlet and extends toward the distal end through the bending portion, and one end of the branch channel is connected to a proximal end side of the bending portion.

Optionally, the branch channel is configured as a comb in whole, and distal ends of the sub-channels are vertically connected to the main channel.

Optionally, there are two lateral channels arranged at two sides of the main channel, respectively, and distal ends of the sub-channels of the two lateral channels are staggered.

Optionally, there are two lateral channels arranged at two sides of the main channel, respectively, the trunk channels of the two lateral channels each intersect with the main channel to form a first intersection position and a second intersection position, and the two lateral channels each independently extend from the first intersection position and the second intersection position towards the outlet and intersect with each other at a position adjacent to the outlet.

Optionally, a distribution structure is provided in the main channel and adjacent to an outlet portion, and the distribution structure closes a middle portion of the main channel along a width direction of the main channel, narrow openings are formed on two sides between the distribution structure and side walls of the main channel, and the first intersection position and the second intersection position are located at corresponding narrow openings, respectively.

Optionally, the lateral channel is configured with a fluid acceleration structure and a sudden expansion section which are arranged in sequence along a flowing direction of the fluid, wherein the narrow opening is connected to the sudden expansion section at a position adjacent to the fluid acceleration structure.

Optionally, a cross-section of the distribution member is triangular-shaped.

Optionally, the main channel extends straightly from the first fluid inlet to the distribution member, with a constant cross-section.

Optionally, an obtuse angle is formed between trend lines of the two lateral channels extending from the first intersection position and the second intersection position towards the outlet.

Optionally, the outlet has a tendency to expand, and an expansion angle corresponds to an angle between the trend lines.

The present application further discloses a nebulization method based on microfluidic chip, including:
receiving a liquid-phase fluid through the microfluidic chip;
receiving a gas-phase fluid through the microfluidic chip, wherein the gas-phase fluid is divided into two paths and each path is independently guided to mix with the liquid-phase fluid to form two nebulized flows;
guiding the two nebulized flows to mix inside the microfluidic chip and then output.

Optionally, at least a portion of each path of the gas-phase fluid is pre-diverted and mixed with the liquid-phase fluid at a relatively upstream location to cause turbulence; and a remaining portion of each path of the gas-phase fluid is mixed with the liquid-phase fluid at a relatively downstream location to cause nebulization.

Optionally, an angle between flow trend lines of two nebulized flows before intersection is an obtuse angle.

The present application further discloses a nebulized medication delivery system, including:
a perfusion device for supplying a fluid containing therapeutic substance, wherein the fluid includes a liquid-phase fluid and a gas-phase fluid, and at least one of the liquid-phase fluid and gas-phase fluid contains the therapeutic substance;
an interventional catheter mentioned above, wherein a proximal end of the interventional catheter is connected to the perfusion device for receiving the gas-phase fluid and the liquid-phase fluid respectively;
a sampling device for collecting state parameters of the fluids;
a controlling device connected to the sampling device, configured for receiving the state parameters and controlling the perfusion device according to the state parameters.

The present application further discloses a gas-liquid interventional device, including:
a housing, on which a gas path connector and a liquid path connector are installed, a pump chamber being provided at a bottom of the housing;
an air pump is installed in the pump chamber, wherein the air pump includes a plurality of pumps connected in parallel and is connected to the gas path connector through a main pipe;
a cooling assembly thermally coupled to the air pump;
a syringe including a cylinder installed in a housing and a piston slidably installed in the cylinder, wherein the cylinder has an outlet and is connected to the liquid path connector;
a drive mechanism, linked to the piston of the syringe;
an interventional catheter mentioned above, wherein a proximal end of the interventional catheter is respectively connected to the gas path connector and the liquid path connector, for receiving the gas-phase fluid and the liquid-phase fluid, respectively.

The present application further discloses a interventional catheter, including a catheter body, wherein one end of the catheter body is configured as a proximal end, and another end of the catheter body is configured as a distal end that is capable of extending into a bronchus, an interior of the catheter body is provided with a microfluidic chip, wherein the microfluidic chip includes:
a first fluid inlet located at a proximal end side of the microfluidic chip;
two second fluid inlets arranged at two opposite sides of the microfluidic chip, respectively; and
an outlet located at a distal end side of the microfluidic chip;
wherein an interior of the microfluidic chip is configured with a chip channel structure connecting the outlet and each fluid inlet, wherein the chip channel structure includes:
   a main channel, one end of which is connected to the first fluid inlet and another end of which is connected to the outlet; and
   a lateral channel with one end thereof being connected to the second fluid inlet and another end thereof being connected to the main channel, wherein a plurality of spaced distribution members are provided in the lateral channel.

Optionally, there are two lateral channels arranged at two sides of the main channel, respectively, and each lateral channel is connected to the middle and downstream of the main channel.

Optionally, the lateral channel has a bending portion pointing to the distal end at the second fluid inlet, and a side at an end of the bending portion facing the main channel is connected to the main channel through an intersection section; the distribution members are sequentially arranged in the intersection section along a length direction of the main channel.

Optionally, a fluid acceleration structure and a sudden expansion section are provided in the main channel and arranged in sequence along a flowing direction of the fluid, wherein the intersection section is connected to the sudden expansion section at a position adjacent to the fluid acceleration structure.

Optionally, the intersection section includes multiple stages of narrowing parts in sequence, and the distribution structure is located between two adjacent stages of narrowing parts.

Optionally, the narrowing parts include three stages, and the distribution member is located between the first and second stages of narrowing parts.

Optionally, an overall extending direction of the intersection section is along a width of the main channel, each narrowing part has two opposite side walls, and at least one of the side walls is inclined relative to the extending direction, so that the narrowing part gradually shrinks.

Optionally, one of the two side walls is parallel to the extending direction, and the other one of the two side walls is inclined relative to the extending direction.

Optionally, in the two most downstream stages, the inclined side walls are on different sides of the flow channel.

Optionally, a variation in the width of the flow channel between the front and rear of each narrowing part is 20~60%.

Optionally, the distribution members are configured in shape of pointed teeth, and a cross-sectional size of each distribution member gradually increases along the flowing direction of the fluid.

Optionally, the distribution members occupy 30~70% of the width of the flow channel at a downstream side of the distribution members.

The present application further discloses a nebulized medication delivery system, including:
a perfusion device for supplying a fluid containing therapeutic substance, wherein the fluid includes a liquid-phase fluid and a gas-phase fluid, and at least one of the liquid-phase fluid and gas-phase fluid contains the therapeutic substance;
an interventional catheter mentioned above, wherein a proximal end of the interventional catheter is connected to a perfusion device for receiving the gas-phase fluid and the liquid-phase fluid respectively;
a sampling device for collecting state parameters of the fluids;
a controlling device connected to the sampling device, configured for receiving the state parameters and controlling the perfusion device according to the state parameters.

The present application further discloses a gas-liquid interventional device, including:
a housing, on which a gas path connector and a liquid path connector are installed, and a pump chamber being provided at a bottom of the housing;
an air pump installed in the pump chamber, wherein the air pump includes a plurality of pumps connected in parallel and is connected to the gas path connector through a main pipe;
a cooling assembly thermally coupled to the air pump;
a syringe including a cylinder installed in a housing and a piston slidably installed in the cylinder, wherein the cylinder has an outlet and is connected to the liquid path connector;
a drive mechanism, linked to the piston of the syringe;
an interventional catheter mentioned above, wherein a proximal end of the interventional catheter is respectively connected to the gas path connector and the liquid path connector, for receiving the gas-phase fluid and the liquid-phase fluid, respectively.

The present application further discloses a nebulized medication delivery system, including:
a perfusion device for supplying a fluid containing therapeutic substance, wherein the fluid includes a liquid-phase fluid and a gas-phase fluid, at least one of the liquid-phase fluid and gas-phase fluid contains the therapeutic substance, and the perfusion device includes a primary heating device for heating at least one of the liquid-phase fluid and gas-phase fluid;
an interventional catheter, a proximal end of which is in connected to the perfusion device for receiving the gas-phase fluid and the liquid-phase fluid respectively, and the interventional catheter is provided with a secondary heating device at a distal end thereof;
a sampling device for collecting state parameters of the fluids;
a controlling device connected to the sampling device, configured for receiving the state parameters and controlling the perfusion device according to the state parameters.

Optionally, the interventional catheter further includes a tertiary heating device located at the proximal end thereof.

Specific beneficial effects of this application will be described below in combination with specific structures, which will not be repeated here.

### BRIEF DESCRIPTION OF THE DRAWINGS

FIG. 1a is a schematic view of an interventional catheter according to an embodiment of the present application.
FIG. 1b is an exploded view of a distal end of a catheter body of an interventional catheter according to an embodiment of the present application.
FIG. 1c is an exploded view of a flow mixing structure according to an embodiment of the present application.
FIG. 1d is a schematic view of FIG. 1c from another aspect.
FIG. 1e is a cross-sectional view of a flow mixing structure according to an embodiment of the present application.
FIG. 1f is an exploded view of a microfluidic chip according to an embodiment of the present application.
FIG. 2a is a schematic view of a microfluidic chip according to an embodiment of the present application.
FIG. 2b is a schematic view of an internal structure of a microfluidic chip according to a first embodiment of the present application.
FIG. 2c is a cross-sectional view taken along line A-A of FIG. 2b.
FIG. 3a is a schematic view of an internal structure of a microfluidic chip according to a second embodiment of the present application.
FIG. 3b is a schematic view of an internal structure of a microfluidic chip according to a third embodiment of the present application.
FIG. 3c is a cross-sectional view taken along line B-B of FIG. 3b.
FIG. 3d is an enlarged view of a portion A1 of FIG. 3b.
FIG. 4a is a schematic view of an internal structure of a microfluidic chip according to a fourth embodiment of the present application.
FIG. 4b is a cross-sectional view taken along line D-D of FIG. 4a.
FIG. 5a is a schematic view of an internal structure of a microfluidic chip according to a fifth embodiment of the present application.
FIG. 5b is a schematic view of an internal structure of a microfluidic chip according to a sixth embodiment of the present application.
FIG. 5c is an enlarged view of a portion A2 of FIG. 5b.
FIG. 6a is a schematic view of an internal structure of a microfluidic chip according to a seventh embodiment of the present application.
FIG. 6b is a cross-sectional view taken along line E-E of FIG. 6a.
FIG. 7a is a schematic view of an internal structure of a microfluidic chip according to an eighth embodiment of the present application.
FIG. 8a is a structural block diagram of a nebulized medication delivery system according to an embodiment of the present application.
FIG. 8b is a structural block diagram of a nebulized medication delivery system according to an embodiment of the present application.
FIG. 8c is a structural block diagram of a nebulized medication delivery system according to an embodiment of the present application.
FIG. 8d is a circuit diagram of a switch circuit according to an embodiment of the present application.
FIG. 8e is a circuit diagram of a speed control circuit of an air pump according to an embodiment of the present application.
FIG. 8f is a block diagram of a control unit according to an embodiment of the present application.
FIG. 8g is a schematic, block diagram of an interventional device according to an embodiment of the present application.
FIG. 8h is a schematic, block diagram of a control method of an interventional device according to an embodiment of the present application.
FIG. 9 is a flow chart of a control method of a nebulized medication delivery system according to an embodiment of the present application.
FIG. 10 is a structural block diagram of a computer according to an embodiment of the present application.
FIG. 11 is a schematic view of an interventional device according to an embodiment of the present application.
FIG. 12 is a side view of the interventional device of FIG. 11.
FIG. 13 is an axonometric view from a back side of the interventional device of FIG. 11.
FIG. 14 is a back view of the interventional device of FIG. 11.
FIG. 15 is a schematic, assembled view of gas-phase pipelines of an interventional device according to an embodiment of the present application.
FIG. 16 is a schematic view showing connection of gas-phase pipelines of an embodiment of the present application.
FIG. 17 is a schematic view of the gas-phase pipelines of FIG. 16 from another aspect.
FIG. 18 is a schematic, assembled view of an air pump according to an embodiment of the present application.
FIG. 19 is a schematic view of the air pump of FIG. 18 from another aspect.
FIG. 20 is a schematic view of the air pump of FIG. 18 from another aspect.
FIG. 20a is a schematic view of a two-stage shock absorption according to an embodiment.
FIG. 21 is a schematic view showing an assembly relationship between an air pump and a cooling assembly according to an embodiment of the present application.
FIG. 22 is a schematic view of the air pump and cooling assembly of FIG. 21 from another aspect.
FIG. 23 is a schematic view showing a cooperation of the air pump, cooling assembly of FIG. 21 and a pump chamber.
FIG. 24 is a schematic, front view of an internal assembly of an interventional device according to an embodiment of the present application.
FIG. 25 is a front view of an interventional device according to an embodiment of the present application.
FIG. 26 is a schematic view of a liquid-phase portion of the interventional device of FIG. 25.
FIG. 27 and FIG. 28 are schematic views showing different states of a plate relative to a fixing seat according to an embodiment of the present application.
FIG. 29 is a schematic view showing cooperation of an adapter identification circuit and an adapter identification label according to an embodiment of the present application.
FIG. 30 is a schematic view of the adapter identification circuit and the adapter identification label of FIG. 29 from another aspect.
FIG. 31 is a top view of the adapter identification circuit and the adapter identification label of FIG. 30.
FIG. 32 is a schematic view of an adapter identification label according to an embodiment of the present application.
FIG. 33 is a schematic view of an adaptation identification circuit according to an embodiment of the present application.
FIG. 34 is a schematic view of an internal structure of an interventional device according to an embodiment of the present application.
FIG. 35 is a side view of the interventional device of FIG. 34.
FIG. 36 is a schematic view of an interventional device with a movable door according to an embodiment of the present application.
FIG. 37 is a side view of the interventional device of FIG. 36.

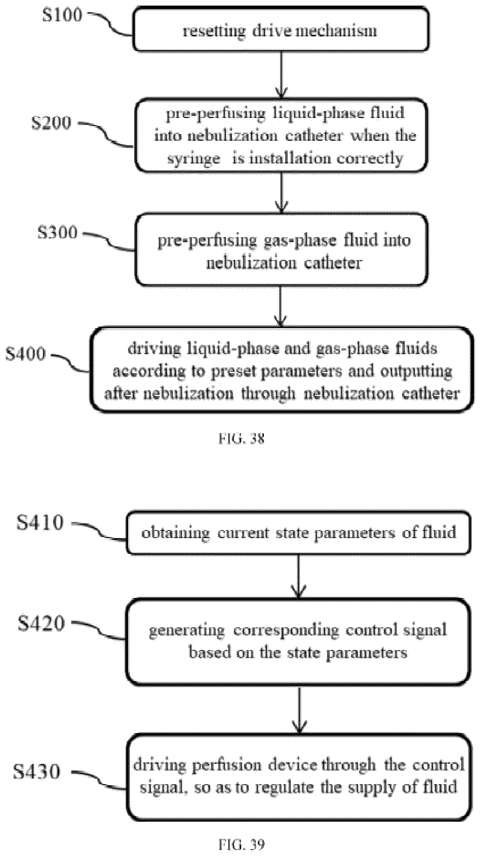

FIG. 38 is a flow chart of a nebulization control method according to an embodiment of the present application.
FIG. 39 is a flow chart of a nebulization method according to an embodiment of the present application.
FIGs. 40-42 are schematic views of a microfluidic chip according to a ninth embodiment of the present application.
FIG. 43 is a schematic view of a microfluidic chip according to a tenth embodiment of the present application.
FIGs. 44-45 are schematic views of a microfluidic chip according to an eleventh embodiment of the present application.
FIGs. 46-47 are schematic views of a microfluidic chip according to a twelfth embodiment of the present application.
FIGs. 48-50b are schematic views of a microfluidic chip according to a thirteenth embodiment of the present application.
FIGs. 51-53 are schematic views of a microfluidic chip according to a fourteenth embodiment of the present application.
FIGs. 54 -56 are schematic views of an overall structure, a distal end and a proximal end of an interventional catheter according to an embodiment of the present application.
FIG. 57 is a schematic, block diagram showing a connection of a catheter handle and a host according to an embodiment of the present application.
FIG. 58 is a schematic, block diagram of a nebulized medication delivery system according to an embodiment of the present application.

### Reference numerals in the drawings are as follows:

900, catheter body; 901, proximal end; 902, distal end; 910, flow mixing structure; 905, tee piece; 906, joint; 9061, communication interface; 907, first joint; 908, second joint; 909, stress dispersion head;
920, microfluidic chip; 921, first unit chip; 922, second unit chip; 930, mark;
940, sheath catheter; 941, balloon; 942, balloon catheter; 943, balloon control interface; 944, heating module; 950, first inner catheter; 951, first connecting tube; 960, second inner catheter; 961, second connecting tube; 970, connecting sleeve; 971, guide chamber; 972, pipe coupling; 973, distal portion; 974, proximal portion;
100, reference plane; 110, inlet; 111, first fluid inlet; 112, second fluid inlet; 120, outlet;
130, main channel; 135, diverging region; 140, lateral channel; 1401/1402/1403/1404, side wall; 141, primary narrowing part; 142, secondary narrowing part; 143, tertiary narrowing part;
210, extension section; 240, narrowing section; 220, expansion section; 231, enlarged section; 232, unchanged section; 233, connecting section; 230, exit section;
300, branch channel;
310, first branch channel; 311, first sub-channel; 312, second sub-channel;
320, second branch channel; 321, first section; 322, second section;
400, distribution member; 401, first side; 402, second side; 403, chamfer; 405, convex column; 450, isolation member; 460, fluid acceleration structure; 461, narrow opening; 462, sudden expansion section;
500, housing; 5001, third buffer block; 501, gas path connector; 5011, second filter; 502, liquid path connector; 503, exhaust port;
510, pump chamber; 511, main pipe; 512, pressure relief valve; 5121, output pipeline; 5122, pressure relief air duct; 5123, four-way joint; 513, soundproof partition; 514, two-stage damping device; 5141, first buffer block; 5142, primary damping platform; 5143, second buffer block; 5144, first mounting groove; 5145, second mounting groove;
520, air pump; 521, air inlet pipe; 5211, first filter; 522, air pressure transmitter; 523, gas cylinder; 524, solenoid valve; 525, pressure regulating valve;
530, cooling assembly; 531, cooling fan; 532, cooling window; 533, heat exchanger; 534, air flow slit;
600, syringe; 610, cylinder; 620, piston; 630, adapter; 631, position limit portion; 632, adapter identification label;
700, drive mechanism; 701, fixing seat; 7011, adapter identification circuit; 702, motor; 703, sliding seat;
7031, pressure sensor; 704, screw rod; 705, pressing plate; 706, locking device; 707, sliding position sensor;
708, sliding trigger member; 709, limit position sensor; 710, limit trigger member;
800, operation panel; 801, shielding cover.

### DESCRIPTION OF THE EMBODIMENTS

Technical solutions of embodiments of the present application will be clearly and completely described below in combination with the drawings. Obviously, the described embodiments are only part of, rather than all of the embodiments of the present application. Based on the embodiments of this application, all other embodiments obtained by those ordinary skilled in the art without any creative work shall fall within the protection scope of this application.

Unless defined otherwise, all technical and scientific terms used herein have the same meaning as commonly understood by one of ordinary skill in the art to which this application belongs. The terms used in the specification of the present application are only for the purpose of describing specific embodiments and are not intended to limit the present application. As used herein, the term "and/or" includes any and all combinations of one or more of the associated listed items.

In this application, the terms "first", "second", and etc. are used for descriptive purposes only and cannot be understood as indicating or implying relative importance or implicitly indicating the number or order of the indicated technical features. Therefore, features defined as "first" or "second" may explicitly or implicitly include one or more of such features. In the description of the present application, "a plurality" means at least two, such as two, three, etc., unless otherwise clearly and specifically defined.

In this application, the terms "comprise/comprises/comprising" and "have/has/having" and any variations thereof are intended to cover non-exclusive inclusions, for example, a system, product or device comprising a series of units is not necessarily limited to those units explicitly listed, but may include other units not explicitly listed or inherent to these products or devices.

This application discloses an interventional catheter including a catheter body, wherein one end of the catheter body is a proximal end, and another end of the catheter body is a distal end being capable of extending into a bronchus, an interior of the catheter body is configured with flow channels for transporting fluid from the proximal end to the distal end, a fluid mixing structure is provided at the distal end of the catheter body, and fluids in the flow channels are mixed in the fluid mixing structure and then output,
the fluid mixing structure at least includes a first fluid inlet, a second fluid inlet and an outlet, and each fluid inlet is configured with the flow channel(s) in the catheter body, respectively;
a microfluidic chip is provided in the fluid mixing structure, the first fluid inlet is located at a proximal end side of the microfluidic chip, the second fluid inlet is located at a lateral side of the microfluidic chip, an interior of the microfluidic chip is configured with a chip channel structure which intercommunicates the outlet and each fluid inlet, wherein the chip channel structure includes:
   a main channel, configured with one end thereof communicating with the first fluid inlet at the proximal end side and another end thereof communicating with to the outlet;
   a lateral channel, configured with one end thereof communicating with the second fluid inlet at the lateral side and another end thereof communicating with the main channel; and
   a distribution structure which is capable of acting on at least a portion of the fluid being set in the chip channel structure. In an embodiment, the distribution structure may pre-disperse a portion of the fluid, so as to avoid direct mixing of all gas-phase fluid and liquid-phase fluid, which can further improve the nebulization effect. For example, the gas-phase fluid is pre-dispersed by the distribution structure and then mixed with the liquid-phase fluid. For one of the gas-phase fluid and liquid-phase fluid, a portion of this fluid (such as 15~60% of the total flow) may be diverted for pre-distribution or flow disturbance, or this fluid may all be dispersed through the distribution structure.

The microfluidic chip may generate vibration during operation, which causes temperature rise in the microfluidic chip. The temperature rise of the microfluidic chip causes cells and inactivated viruses to die or lose their activity. Through nebulization, the present application may effectively reduce the temperature of the microfluidic chip, so as to reduce a probability of death and loss of activity of the cell and inactivated viruses.

In the present application, flow mixing and nebulization of the gas-phase fluid and liquid-phase fluid is different from the application scenarios of fluid transmission or negative pressure transmission in the prior art, both in terms of principle and device requirements. On one hand, the flow mixing and nebulization are carried out inside the microfluidic chip or near the outlet; on the other hand, the main channel and the lateral channel supply fluids in different phases (one is gas-phase and the other is liquid-phase), respectively, wherein each fluid has a working pressure required to be positive pressure, for example, a feed pressure is required to be greater than 0.1MPa.

In the present application, the microfluidic chip is processed by etching, for example, the etched portion of the microfluidic chip forms the chip channel structure, and the distribution structure is convex columns formed by the remaining solid portion of the etched microfluidic chip. Based on this, all channels of the chip channel structure are set in a coplanar manner. In order to ensure the nebulization effect, the microfluidic chip is configured with only one outlet at the distal end thereof for outputting the nebulization fluid.

Since sufficient mixing and nebulization need to be achieved in the narrow space of the chip channel structure, the setting of the lateral channel and main channel as well as intersection angles of themselves or each other are also different from conventional flow mixing. At least one or a combination of the following manners may be used to ensure the nebulization effect: for example, an intersection position of the lateral channel and the main channel is a junction, and at least one of the junctions is configured with an orientation thereof perpendicular to an extension direction of the main channel. For another example, there are two lateral channels distributed on two sides of the main channel, respectively, at least a portion of each lateral channel intersects with the main channel at a position adjacent to the outlet, and an obtuse angle is formed between extension trend lines of the two lateral channels at their intersection position. For another example, two lateral channels are provided at different sides of the microfluidic chip and each is configured with one inlet, respectively, that is, there are two second fluid inlets distributed symmetrically on two opposite sides, respectively.

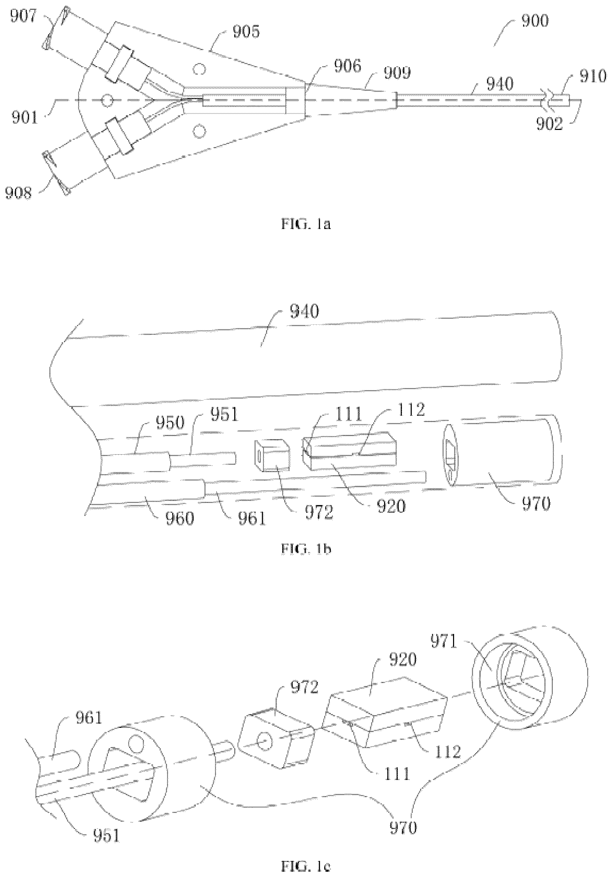

Referring to FIG. 1a and FIG. 1b, an embodiment of the present application provides an interventional catheter which includes a catheter body 900. One end of the catheter body 900 is a proximal end 901, and another end of the catheter body 900 is a distal end 902 that can extend into a bronchus. An interior of the catheter body 900 is configured with flow channels for transporting fluid from the proximal end 901 to the distal end 902. The distal end 902 of the catheter body 900 is provided with a flow mixing structure 910. That is, for the whole interventional catheter, its distal end 902 is configured as the flow mixing structure 910, in which a microfluidic chip 920 is provided. Fluids in the flow channels are mixed in the microfluidic chip 920 and then output. A distribution structure that can act on at least a portion of the fluid is provided inside the microfluidic chip 920.

The interventional catheter of this embodiment can extend into the bronchus, so as to reach the lesion site. Depending on the treatment purpose, therapeutic substances may be pre-dispersed in the fluid. The therapeutic substances may be medications, chemical agents, solutions containing biological cell tissues, or solutions containing cell secretions. The treatment purpose may be, for example, to repair damage to the lungs caused by pneumonia or asthma.

Depending on the nature of the therapeutic substances, the fluid itself may be in liquid-phase or gas-phase, or a complex mixture of liquid and gas. The purpose of nebulization is to further disperse the fluid into smaller particles, so as to promote absorption and uniform administration. Flow channels, configured for transporting the fluids, extend from the proximal end 901 to the distal end 902 of the catheter body 900 and intersect and communicate with each other at the flow mixing structure 910. That is, the flow channels, especially for fluids in different phases, are independent before reaching the flow mixing structure 910, which can avoid weakening the nebulization effect due to premature mixing. In the embodiments of the present application, the distal end or distal end side refers to an end or a side relatively close to the outlet of the fluid mixing structure, and the proximal end or proximal end side refers to an end or a side relatively far away from the outlet of the fluid mixing structure, along an extending direction of the catheter body of the interventional catheter. At various positions of the interventional catheter, the relative upstream and downstream are divided according to the flowing direction of the fluid.

The catheter body 900 may be made of metal or synthetic material, so as to provide the necessary mechanical properties and interventional safety. According to the needs of fluid transportation, the catheter body 900 may be in the form of a single tube, multiple tubes, and etc. When the catheter body 900 is configured with multiple tubes, at least two of the tubes may be arranged side by side or one inside another, and the tubes each may be configured independently or may be an integrated multi-lumen tube.

Referring to FIG. 1a to FIG. 1f, in one embodiment, the catheter body 900 includes a sheath catheter 940, a first inner catheter 950 and a second inner catheter 960. The microfluidic chip 920 includes a first fluid inlet 111, a second fluid inlet 112 and an outlet 120. Fluids flow into the microfluidic chip through the inlets and are nebulized in the microfluidic chip. The first inner catheter 950 and the second inner catheter 960 both extend in the sheath catheter 940. A distal end of the first inner catheter 950 communicates with the first fluid inlet 111, and a distal end of the second inner catheter 960 communicates with the second fluid inlet 112. The sheath catheter 940 provides protection and necessary mechanical support for each inner catheter. The dotted line in FIG. 1b indicates an assembly position of the sheath catheter 940.

Taking an extension direction of the catheter body 900 as a reference, an orientation of the outlet 120 of the microfluidic chip 920 may face toward the distal end in the axial direction. In order to facilitate extension in thinner bronchus, an outer diameter of the sheath catheter 940 is generally not greater than 1.2mm to 2.0mm. For example, an outer diameter of the interventional catheter is 1.8mm, which can reach the lungs that the endoscope cannot reach, so as to achieve precise nebulization medication delivery. Since the interventional catheter of the present application can extend into the bronchus, it accordingly has a matched length. For example, the length of the sheath catheter 940 is 800mm to 1200mm.

The interventional catheter further includes a tee piece 905, and the proximal end of the sheath catheter 940 is connected to one joint of the tee piece 905, such as the joint 906 shown in FIG. 1a. After extending out of the sheath catheter 940, the proximal ends of the first inner catheter 950 and second inner catheter 960 extend into and are connected to the other two joints of the tee piece 905, respectively, so as to realize separate connections of the input fluid for the first inner catheter and the second inner catheter.

A stress dispersing head 909 for relieving stress may be provided at the joint 906 to reduce the damage of the sheath catheter 940. The other two joints of the tee piece 905 may be, for example, first joint 907 and second joint 908. The first joint 907 and second joint 908 may be Luer tapers. The first joint 907 and second joint 908 may be connected to an external perfusion device, so as to transport fluid into each inner catheter.

Referring to FIG. 1b to FIG. 1e, the flow mixing structure 910 includes a microfluidic chip 920 and a connecting sleeve 970 fixed in and located at the distal end of the sheath catheter 940. The connecting sleeve 970 is set around the microfluidic chip 920, with both ends thereof being sealed relative to an outer circumference of the microfluidic chip 920. A guide chamber 971 is defined between an inner wall of the connecting sleeve 970 and the outer circumference of the microfluidic chip 920, and the second fluid inlet 112 and the distal end of the second inner catheter 960 both are communicated with the guide chamber 971. The flow guide chamber 971 may be understood as an annular space formed around the microfluidic chip 920. After the second fluid of the second inner catheter 960 enters the annular space, it is transported into the microfluidic chip 920 through the second fluid inlet 112. The sheath catheter provides necessary structural stability for the first inner catheter and the second inner catheter. The first inner catheter communicates with the first fluid inlet, allowing the first fluid to flow into the microfluidic chip. The guide chamber surrounds the microfluidic chip, and the microfluidic chip may be provided with multiple second fluid inlets at a portion thereof being surrounded and contacted.

The proximal end of the microfluidic chip 920 is fixedly attached to a pipe coupling 972, the proximal end of the connecting sleeve 970 is mounted around and sealed with an outer circumference of the pipe coupling 972. A first connecting tube 951 is inserted into the pipe coupling 972, wherein a distal end of the first connecting tube 951 communicates with the first fluid inlet 111, and a proximal end of the first connecting tube 951 is mounted around and sealed with the distal end of the first inner catheter 950. A second connecting tube 961 is inserted into the proximal end side of the connecting sleeve 970, wherein a distal end of the second connecting tube 961 extends into the connecting sleeve 970 and communicates with the guide chamber 971, and a proximal end of the second connecting tube 961 is mounted around and sealed with the distal end of the second inner catheter 960.

The microfluidic chip 920 protrudes outwardly relative to the distal end of the connecting sleeve 970, and an axial protruding size of the microfluidic chip is 20%~30% of an axial length of the entire microfluidic chip 920. The first inner catheter 950 and the second inner catheter 960 may be, for example, made of plastic, and the first connecting tube 951 and the second connecting tube 961 may be, for example, made of metal, so as to ensure the flexibility and stability of the catheter body and avoid bending at the connecting position. The pipe coupling 972 is attached to the microfluidic chip 920, with the proximal end thereof being sealed and surrounded by the connecting sleeve 970, forming an abutment position limit against the microfluidic chip 920 in the axial direction of the catheter body. Along the axial direction of the sheath catheter 940, the connecting sleeve 970 is a divided structure, and includes a distal portion 973 that is sealed and fixed and a proximal portion 974, and the guide chamber 971 is located between the distal portion and the outer circumference of the microfluidic chip. The connecting sleeve in whole has a small size and is configured as a divided structure, which is conducive to the processing of the guide chamber. The nebulizer may use a gas source as a driving member to achieve nebulization. The gas source may be, for example, air, a mixed gas of hydrogen, oxygen, and carbon dioxide in different proportions, or other gases that promote lung recovery.

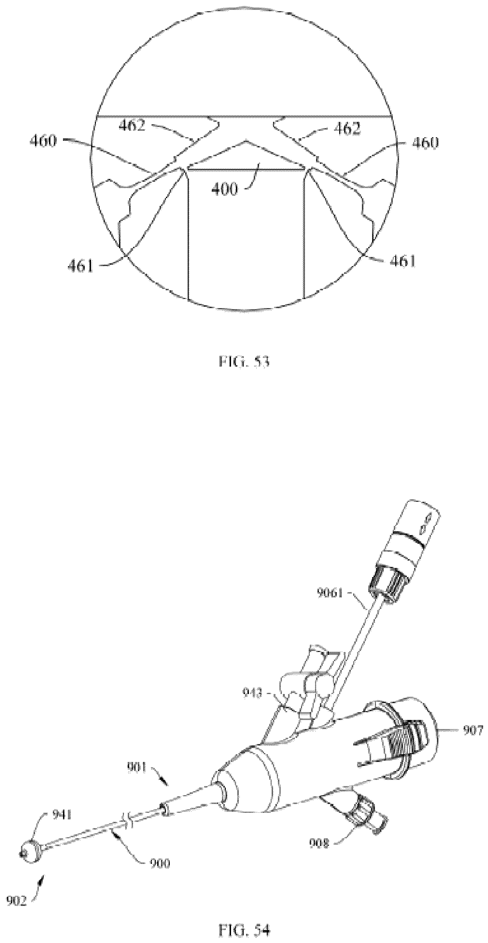

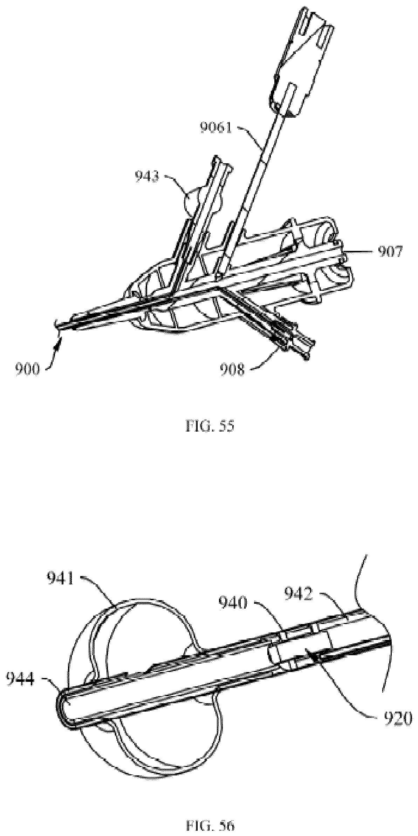

In addition to the above embodiments, with reference to FIG. 54 to FIG. 56, the present application further provides another interventional catheter, including:
a catheter body 900, wherein one end of the catheter body 900 is a proximal end 901 and another end of the catheter body 900 is a distal end 902 that can extend into a bronchus,
a microfluidic chip 920 with a chip channel structure formed therein, wherein the chip channel structure includes a main channel 130 and a lateral channel 140, the main channel 130 and/or the lateral channel 140 are configured with a fluid acceleration structure 460 and a sudden expansion section 462 arranged in sequence along a flowing direction of the fluids, and the fluids in the main channel 130 and the lateral channel 140 interact with each other in the sudden expansion section 462 and thus output nebulized fluid;
a heating module 944 set in the catheter body 900 and located at a distal end 902 of the microfluidic chip 920, configured for adjusting a temperature of the nebulized fluid; and
a balloon 941 being disposed outside the catheter body 900 and deformable.

The balloon 941 is capable of changing its shape, such as expansion or contraction, under the action of a balloon catheter 942, a balloon control interface 943 and a corresponding fluid delivery, so as to achieve blocking, positioning and corresponding operations for a preset position, improving the stability and adaptability of the operation of the interventional catheter.

In terms of setting details, in the axial direction of the catheter body 900, the balloon 941 and the heating module 944 are positioned correspondingly. The distal end 902 of the microfluidic chip 920 is configured with an outlet 120 for outputting the nebulized fluid. The heating module 944 is a heating tube. An interior of the heating tube is configured as a temperature control channel, connected to and communicating with the outlet 120. A ratio of an inner diameter to an axial length of the heating tube is in a range of 0.05 to 0.3.

The microfluidic chip 920 includes:
a first fluid inlet 111, located at a proximal end side of the microfluidic chip 920;
a second fluid inlet 112, located at a lateral side of the microfluidic chip 920;
an outlet 120, located at a distal end side of the microfluidic chip 920;
wherein one end of the main channel 130 communicates with the first fluid inlet 111, and another end of the main channel 130 communicates with the outlet 120; one end of the lateral channel 140 communicates with the second fluid inlet 112, and another end of the lateral channel 140 communicates with the main channel 130; a distribution structure capable of acting on at least a portion of the fluid to pre-disperse this portion of the fluid is provided in the chip channel structure. An internal volume of the sudden expansion section 462 is at least 15% larger than an internal volume of the fluid acceleration structure 460. The main channel 130 is connected to the lateral channel 140 at the sudden expansion section 462, and a connection position is adjacent to the fluid acceleration structure 460. The fluid acceleration structure 460 is formed by expansion and contraction of the flow channel where the sudden expansion section 462 is located. The sudden expansion section 462 is formed by expansion of the flow channel where the sudden expansion section 462 is located, and the sudden expansion section 462 and the outlet 120 of the fluid acceleration structure 460 have a smooth transition or a stepped transition. The sudden expansion section 462 is attached to the fluid acceleration structure 460, and a chamber diameter of the sudden expansion section 462 is at least 10% larger than that of the outlet 120 of the fluid acceleration structure 460.

Along an extension direction of the main channel 130, the main channel 130 intersects with the lateral channel 140 at least twice, wherein the first intersection is at the middle and upper reaches of the main channel 130 and implements turbulence, and the second intersection is adjacent to the outlet 120 and implements nebulization. The first intersection can play a role of pre-dispersion, effectively controlling the particle size of the nebulized liquid and the distribution range of the particle size of the nebulized liquid. There are two lateral channels 140 being arranged on two sides of the main channel 130, respectively. The distribution structure 400 is provided in the main channel 130, and two narrow openings 461 are formed between the distribution structure 400 and sidewalls of the main channel 130. The narrow openings 461 communicate with the corresponding sudden expansion sections 462 of the two lateral channels 140, respectively, so as to form the second intersection. Two lateral channels 140 are provided and are respectively arranged on two sides of the main channel 130, the fluid acceleration structure 460 and the sudden expansion section 462 are arranged in groups and are respectively arranged in the corresponding lateral channel 140, wherein the two sudden expansion sections 462 are configured to intersect with each other in the main channel 130.

For the embodiment shown in the drawings, the microfluidic chip 920 is arranged at a proximal end side of the balloon 941, and a heating module 944 is provided inside the sheath catheter 940 corresponding to the position of the balloon 941. The heating module 944 is a hollow heating tube in the drawings. The droplets passing through the microfluidic chip 920 leave the sheath catheter 940 through an internal space of the heating module 944 to complete the transportation. The heating module 944 is able to form a temperature-controllable transportation environment, thereby realizing operations such as insulation, heating of the droplets. A heating temperature of the heating module 944 is preferably 43-60 °C., at which time the lesions (cancer cells) can be killed and normal cells can recover. The interventional catheter further includes a first joint 907 and a second joint 908, which can be connected to an external perfusion device for conveying fluid into each inner catheter. The interventional catheter further includes a communication interface 9061, through which the transmission of signals and energy can be realized and connected.

On this basis, this embodiment further discloses =a nebulized medication delivery system, including:
a perfusion device for supplying a fluid containing a therapeutic substance, wherein the fluid comprises a liquid-phase fluid and a gas-phase fluid, and at least one of the liquid-phase fluid and a gas-phase fluid contains the therapeutic substance;
an interventional catheter, using the above-mentioned interventional catheter, wherein a proximal end 901 of the interventional catheter communicates with the perfusion device for input the gas-phase fluid and liquid-phase fluid, respectively;
a sampling device, configured for collecting state parameters of the fluid;
a controlling device connected to the sampling device, configured for receiving the state parameters and controlling the perfusion device according to the state parameters.

Similarly, this embodiment further discloses a two-phase interventional device, wherein the interventional device includes:
a housing 500, on which a gas path connector 501 and a liquid path connector 502 are mounted, a pump chamber 510 being provided at a bottom of the housing 500;
an air pump 520 installed in the pump chamber 510, wherein the air pump 520 includes a plurality of pumps connected in parallel and is connected to the gas path connector 501 through a main pipe 511.
a cooling assembly 530, thermally coupled to the air pump 520;
a syringe 600 including a cylinder 610 installed in the housing 500 and a piston 620 slidably installed in the cylinder 610, wherein the cylinder 610 has an outlet 120 communicating with the liquid path connector 502;
a drive mechanism 700 linked to the piston 620 of the syringe 600.

The interventional catheter, uses the above-mentioned interventional catheter, with the proximal end 901 thereof connected to the gas path connector 501 and the liquid path connector 502 for receiving gas-phase fluid and liquid-phase fluid, respectively. The specific settings of the interventional device are described below and will not be repeated here.

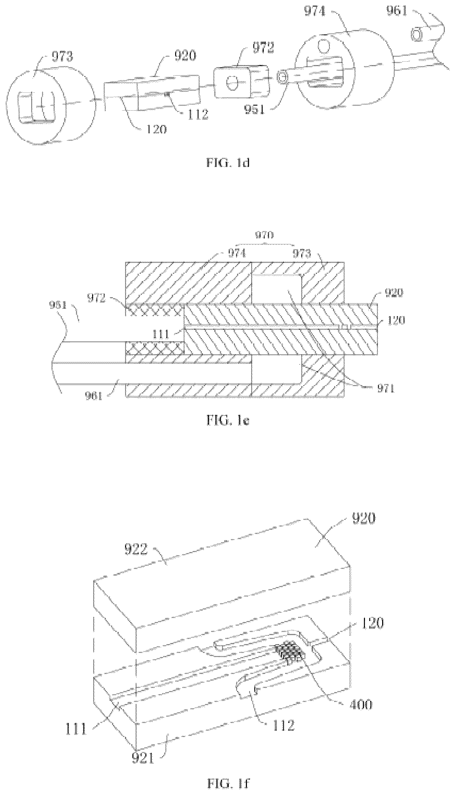

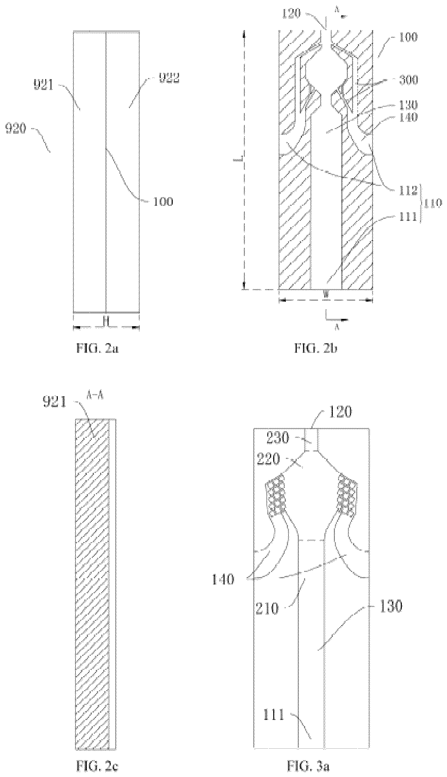

Referring to FIG. 1e and FIG. 2a to FIG. 2c, in one embodiment, the microfluidic chip 920 includes a first unit chip 921 and a second unit chip 922 which are stacked and fixed, and at least one of the two unit chips, on sides facing towards each other, is etched with a microflow channel for guiding fluid to pass through. The two unit chips are processed separately, which is convenient for adjusting and changing the depth of the microflow channel and the shape of the microflow channel.

Taking the overlapping surfaces of the two unit chips as a reference plane 100, that is to say, the reference plane 100 is a side of the etched unit chip facing to the other unit chip. In the direction perpendicular to the reference plane 100, the depth of the microflow channel is 30~300µm, and more preferably 75±25µm. The overlapping surfaces of the two unit chips is the contact surfaces of the first unit chip 921 and the second unit chip 922. The depth of the microflow channel is preferably the same everywhere, for example, it may be 50µm. Referring to the cross-sectional views of the following embodiments, the etched depth is represented by the width of the blank zone on the right side of each cross-sectional view. The depth of the chip channel structure affects the flow channel area, which further affects the flow rate and fluid pressure. An appropriate range of values of the depth ensures that molecules or cells with larger particle sizes in the solution pass through and ensures the fluid pressure. When the microflow channel is larger in space, it may allow the transport of larger particles, such as human stem cells, small molecule peptides, and even protein cells, generally having a size in a range of 20~60µm, such as 50µm.

The microfluidic chip 920 may be configured as a cuboid in whole, and is consisted of a first unit chip 921 and a second unit chip 922 having substantially the same three-dimensional dimensions. One of the first unit chip 921 and the second unit chip 922 may be made of silicon, and the other may be made of glass. The unit chip made of silicon is used to etch a microflow channel on a side thereof facing to the other unit chip. After the etching is completed, the two unit chips are stacked, fixed, and bonded into an integrated structure to obtain the microfluidic chip 920. During the etching process, etching is also performed on areas outside the product cutting line to ensure the patency of each flow channel of the microfluidic chip.

In this embodiment, a direction in which the reference plane 100 extends along the catheter body 900 is defined as the length direction L of the microfluidic chip 920. The length of the microfluidic chip 920 may be, for example, 2500µm. A direction in which the reference plane 100 extends perpendicular to the catheter body 900 is defined as the width direction W of the microfluidic chip 920. The width of the microfluidic chip 920 may be, for example, 900µm. In the direction H perpendicular to the reference plane 100, the size of the microfluidic chip 920 may be, for example, 600µm. Of course, the microfluidic chip 920 can be further adjusted according to the size of the catheter body 900 and the requirements of nebulization and mixing effects.

The flow mixing structure 910 has an inlet connected to the flow channel and an outlet for outputting the nebulized fluid. The outlet is located at the distal end of the microfluidic chip 920, and the inlet may be one or multiple. When there is only one inlet, it may be configured at the proximal end face of the microfluidic chip 920.

When there are multiple inlets, at least one inlet is located on the proximal end side of the microfluidic chip 920, and at least one inlet is located at a lateral side of the microfluidic chip 920 along the radial direction of the catheter body 900; the inlet 110 at the lateral side and the inlet at the proximal end side are independently configured with flow channels in the catheter body 900.

Taking multiple inlets as an example, the inlet 110 at least includes a first fluid inlet 111 and a second fluid inlet 112, and each fluid inlet is independently configured with a flow channel in the catheter body 900. The first fluid inlet 111 and the second fluid inlet 112 refer to inlets for flowing two types of fluids, and the specific number of the first fluid inlet or second fluid inlet is not limited. The first fluid inlet 111 is located at the proximal end side of the microfluidic chip 920; and the second fluid inlet 112 is located at the lateral side the microfluidic chip 920 along the radial direction of the catheter body 900. Further, there are two second fluid inlets 112 arranged at two opposite sides of the microfluidic chip 920, respectively. Specifically, the two second fluid inlets 112 are arranged at two opposite sides in the width direction of the reference plane 100. The microfluidic chip 920 acts as a structure for realizing the nebulization function of the flow mixing structure 910, its inlet 110 includes a first fluid inlet 111 and a second fluid inlet 112; its outlet 120 is the outlet of the flow mixing structure 910, and an interior of the microfluidic chip 920 is configured as a chip channel structure intercommunicating the inlet 110 with the outlet 120.

As shown in FIG. 2b, from a viewing angle perpendicular to the reference plane 100, at least one of the flow channels in the chip channel structure has a width greater than or equal to 50µm. The width of the chip channel structure is set with such threshold value, which can ensure that molecules or cells with a particle size greater than or equal to 50µm in the solution pass through, reducing the possibility of damage during passage. That is, there is at least one path from any inlet to the outlet with a width not less than the above-mentioned size at any place, so that therapeutic substances with larger particle sizes can pass through without being blocked or damaged.

Preferably, at least one flow channel in the chip channel structure has a width of 100±10µm; and the width of the outlet 120 is 100±10µm. The nebulization process allows molecules or cells in the solution to pass through the chip channel structure in parallel and reduces cell damage.

In each embodiment of the present application, the numerical value represented by A±B is between (A-B) and (A+B), A is greater than B and both are numerical values. For example, the width of the inlet is 300±100µm, which means that the width of the inlet is 200~400µm.

In one embodiment, the chip channel structure includes a main channel 130 and a lateral channel 140, wherein one end of the main channel 130 communicates with the first fluid inlet 111 at the proximal end side, and another end communicates with the outlet 120. One end of the lateral channel 140 communicates with the second fluid inlet 112 at the lateral side, and another end communicates with the main channel 130. The first fluid inlet 111 is used to deliver a first fluid, and the second fluid inlet 112 is used to deliver a second fluid. For example, the first fluid is in liquid-phase and the second fluid is in gas-phase, or the first fluid is in gas-phase and the second fluid is in liquid-phase. The width of the second fluid inlet 112 may be, for example, 200µm. In the length direction L of the microfluidic chip 920, a distance between the second fluid inlet 112 and the proximal end of the microfluidic chip 920 is 1300±30µm. The main channel 130 may be understood as a flow channel located in a middle portion of the microfluidic chip 920.

The distribution structure is configured as at least one of the following manners. In one embodiment, the distribution structure is configured as a plurality of branch channels 300 at the distal end of the lateral channel 140, through which the lateral channel 140 intersects with the main channel 130, wherein the width of the branch channel 300 is 20±10µm. In another embodiment, the distribution structure is configured as a plurality of distribution members 400 arranged at intervals. Further, the distributed members 400 are arranged in an array with a gap of 10±5µm and a size of 40±20µm. Changing the sizes of different branch channels may adjust the fluid pressure of the different branch channels, and the distribution members arranged in an array may further disperse and release the fluid pressure. The above-mentioned gaps and sizes refer to the dimensions on the reference plane. The size of 40±20µm may be understood as a rectangle with a side length of 40±20µm or a circle within a diameter of 40±20µm. Other shapes may be judged similarly. The distribution member may be convex columns 405, which are remaining solid parts after etching to form the first unit chip 921 or the second unit chip 922.

Specifically, the distribution structure is located at the intersection portion of the main channel 130 and the lateral channel 140, and the fluid is mixed through multiple inlets and multiple flow channels, so as to further enhance the nebulization effect. A position of the intersection portion belongs to the main channel 130 or the lateral channel 140 and is close to the other. For example, the intersection portion is located at a position of the main channel 130 close to the lateral channel 140, or it is not strictly divided and does not affect the realization of the nebulization effect. As an embodiment, the flow channel where the distribution structure is located is gas-phase, and the distribution structure can disperse the gas-phase fluid. When the liquid-phase fluid uses a solution containing biological cells, the cell damage rate may be reduced.

In one embodiment, a portion of the main channel 130 is a widened expansion section, and at least a portion of the lateral channel 140 intersects with the expansion section, wherein intersection positions are multiple and spaced apart from each other. The widening of the main channel will change the state of the fluid. Combined with the multiple intersection positions, the mixing effect of the two fluids and the final nebulization effect can be further optimized. Compared with a single intersection or directly dispersing one phase fluid into another phase fluid, the droplets size after nebulization in this application can be further reduced, thereby improving the distribution of therapeutic substances and saving dosage. The widened range of the expansion section may be 1.45~2 times of the original range, and the widening trend is gradual widening and then gradual narrowing. It can be narrowed directly to the exit, or it can be narrowed and then extended to the exit with the same width. As for the lateral channel 140 as a whole, it may intersect with the main channel only in the expansion section; or, it may intersect with the main channel both in the expansion section and a part other than the expansion section, preferably intersect with the main channel at a downstream of the expansion section.

Further, two of the intersection positions are arranged in sequence along the extension direction of the main channel 130, or two of the intersection positions are distributed on two sides of the main channel 130. The intersection position of the lateral channel 140 and the main channel 130 is a junction, and an orientation of at least one junction is perpendicular or inclined to the extension direction of the main channel 130, wherein an inclined angle is 30~60 degrees and is inclined toward the outlet of the flow mixing structure.

The orientation of the junction may affect the nebulization effect and fluid resistance. If the junctions are all parallel to the extension direction of the main channel, it will not be conducive to the full mixing of the gas-phase fluid and the liquid-phase fluid. When multiple junctions are arranged along the extension direction of the main channel, the orientation of the junctions may vary slightly. That is, the orientations of the multiple junctions arranged in sequence along the extension direction of the main channel are not exactly the same. In some cases, the orientations of all the junctions may be perpendicular or inclined to the extension direction of the main channel.

Referring to FIG. 3a to FIG. 3d, in one embodiment, the main channel 130 includes an extension section 210, an expansion section 220, and an exit section 230 that communicate with each other in sequence. The division of the three sections may refer to dotted lines in FIG. 3a. The extension section 210 is relatively located at the proximal end side of the microfluidic chip, and has a width of 250±50µm; the expansion section 220 is wider than the extension section 210, and has a maximum width of 1.45~2 times the width of the extension section 210, for example, 375±75µm; the exit section 230 is connected to the outlet 120, and has a width of 100±50µm. The change in diameter of the main channel helps to release the fluid pressure. On the basis of the diameter changes of the extension section, expansion section and exit section of the main channel, a narrowing section is added between the extension section and the expansion section to further reduce the difficulty of gas-liquid mixing of the fluid in the expansion section.

The division of the sections of the main channel 130 is determined according to the trend of width change. Along a direction from the proximal end to the distal end, the first time the width of the main channel 130 increases is considered as the beginning of the expansion section 220; and a portion which is adjacent to the outlet and has a constant width is considered as the exit section 230. In the prior art, the calculation formula of flow resistance is: R=ΔP/Q. When the flow rate of the fluid in the main channel remains unchanged per unit time, as the pressure difference between the extension section and the expansion section increases, the flow resistance of the fluid in the expansion section decreases, thereby reducing the difficulty of gas-liquid mixing in the expansion section.

In this embodiment, the lateral channel 140 intersects with the expansion section 220. The maximum width of the lateral channel 140 is 200±50µm. Furthermore, the width of the lateral channel 140 decreases continuously from upstream to downstream.

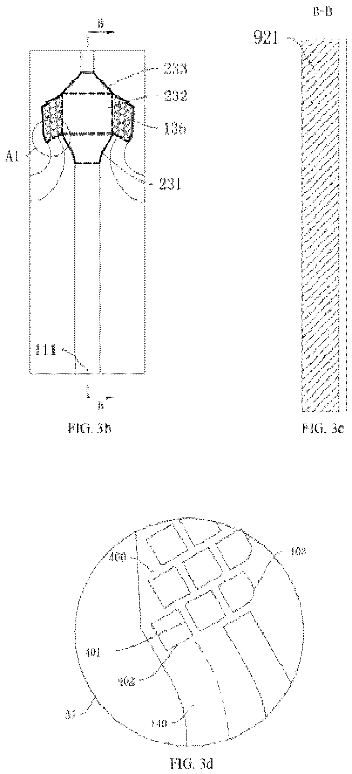

In this embodiment, the expansion section 220 is specifically divided into three parts, which are enlarged section 231, unchanged section 232 and connecting section 233 along the direction from the proximal end to the distal end. The division of the three parts may refer to the dotted lines shown in FIG. 3b. The width of the enlarged section 231 continues to increase relative to the extension section 210. The retaining section 232 maintains the width of the expansion section 220 without significant change, which can be understood as a change of less than 10% relative to the maximum width. The connecting section 233 continues to shrink relative to the expansion section 220 until it docks with the exit section 230. The intersection portions are at a position where the unchanged section 232 is connected to the lateral channel 140, specifically, the dotted lines on two opposite sides of the unchanged section 232 in the width direction in FIG. 3b.

The distribution structure of this embodiment adopts a distribution member 400. The distribution member 400 is located in the lateral channel 140 and adjacent to the intersection portion of the lateral channel 140 and the main channel 130. The distribution member 400 is located at a diverging region 135 for diffusing the fluid, and the distribution member 400 is disposed within the diverging region 135. Accordingly, the diverging region 135 is outside two opposite sides of the expansion section 220.

Furthermore, a cross section of each distribution member on the reference plane is a square which includes a first side 401 and a second side 402 that are perpendicular to each other. The gap between the distribution members 400 is 10µm, and they are arranged in a strip-shaped array of 3×8 on two opposite sides, wherein the strip-shaped array generally extends along the length direction L.

The lateral channel 140 has an extended trend line (dashed line in FIG. 3 d ) for controlling the direction of the fluid at a position adjacent to the diverging region 135, and the distal end of the extended trend line is perpendicular to the second side 402. Furthermore, a chamfer 403 is provided on a side of the distribution member 400 adjacent to the expansion section 220. The extension trend line is arc-shaped, and the flow channel width of the lateral channel 140 along the extension trend line direction gradually narrows.

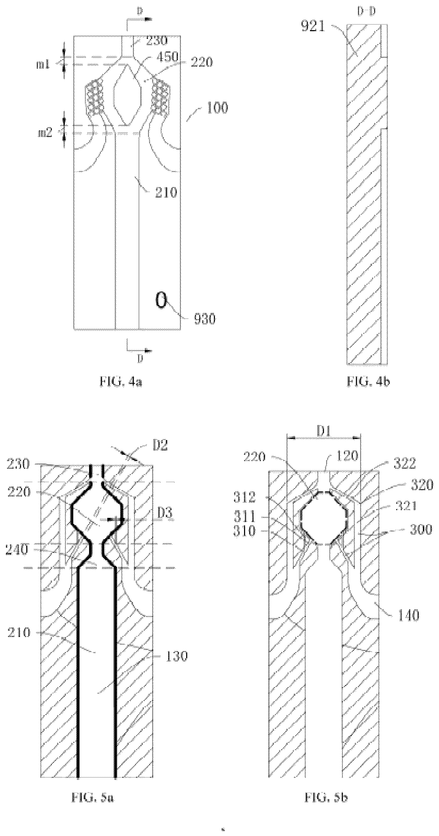

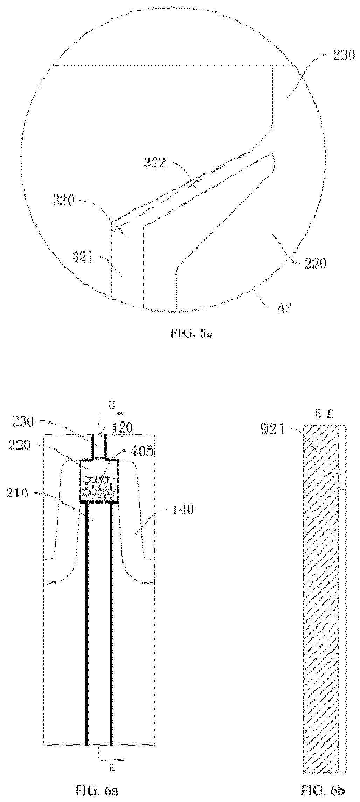

The distal end of the trend line may be directed toward the distal end of the microfluidic chip (as shown in FIG. 5a), or, bend outward relative to the central axis of the microfluidic chip (as shown in FIG. 3b), or bend inward relative to the central axis of the microfluidic chip (as shown in FIG. 6a).

In FIG. 3a, an interior of the expansion section 220 is interconnected as a whole. In another embodiment shown in FIG. 4a, an isolation member 450 is provided inside the expansion section 220. The isolation member 450 divides the interior of the expansion section 220 into a plurality of parallel flow channels, and the width of each flow channel is not less than 90µm.

An outer profile of the isolation member 450 may be, for example, a symmetrical hexagon, wherein an inner angle at the proximal end and an inner angle at the distal end of the hexagon are equal and acute. The isolation member 450 may be shuttle-shaped, and two opposite sides of the shuttle-shaped isolation member in the width direction are divided into two parallel flow channels. The flow channel width may be, for example, 0.5±0.2 times the width of the extension section 210. In this embodiment, the width of the extension section 210 remains unchanged and is 200µm.

In the length direction of the reference plane, the isolation member 450 is shorter than the expansion section 220. A distance between the distal end of the isolation member 450 and the exit section 230 is m1, and a distance between the proximal end of the isolation member 450 and the extension section 210 is m2, wherein m1 is 100±10µm, and m2 is 100±10µm.

Referring to FIG. 5a and FIG. 5b, in one embodiment, the main channel 130 includes an extension section 210, a narrowing section 240, an expansion section 220 and an exit section 230 that are communicated with each other in sequence. Among them, the extension section 210 is relatively located at the proximal end side of the microfluidic chip 920, communicating with the first fluid inlet 111 and with a width of 250±50µm. The narrowing section 240 intercommunicates the extension section 210 with the expansion section 220, and has a width smaller than that of the extension section 210, wherein the minimum width of the narrowing section 240 is 100±50µm. The expansion section 220 becomes wider relative to the extension section 210, and the maximum width thereof is 375±75µm. The exit section 230 is connected to the outlet 120, and has a width of 100±50µm. On the basis of the diameter changes of the extension section, expansion section and outlet section of the main channel, the narrowing section added between the extension section and the expansion section further reduces the difficulty of gas-liquid mixing of the fluid in the expansion section.

The width variation trends and definition principles of the extension section 210, the expansion section 220 and the exit section 230 may refer to the embodiment shown in FIG. 3a, or refer to the dotted line in FIG. 5a. Compared with the embodiment shown in FIG. 3a, the narrowing section 240 is provided in this embodiment. The narrowing section 240 is continuously narrowed along the direction from the proximal end to the distal end until it connects the expansion section 220.

An area enclosed by the expansion section 220 is a hexagon with three pairs of edges, wherein the edges of each pair are parallel to each other. As shown in the dotted box in FIG. 5b, the hexagon is provided with chamfers, and each inner angle is in the range of 110-130 degrees. For example, the area enclosed by the expansion section 220 is a regular hexagon with chamfers, and each inner angle is 120 degrees.

In this embodiment, the distribution structure is realized by the branch channels 300. The lateral channels 140 intersect with the expansion section 220 and/or the exit section 230, and the width of the lateral channel 140 is 200±50µm. Since the sizes of the expansion section and the exit section are different, the nebulization effects produced by the intersection of the lateral channel and the main channel at different locations will be different accordingly. The lateral channel and the main channel intersect at two positions at the same time, and the nebulization effect is better. For the same lateral channel, there are at least two branch channels 300 communicated with the lateral channel, wherein at least one branch channel 300 is connected to the expansion section 220 of the main channel 130, and at least one branch channel 300 is connected to the exit section 230 of the main channel 130.

The lateral channel 140 includes a first branch channel 310 that intersects with the expansion section 220. A connection position of the first branch channel 310 and the expansion section 220 is located at the proximal end side of the expansion section 220. In the drawings, the first branch channel 310 is set to be bilateral symmetry. In this embodiment, the expansion section 220 includes three parts, namely, an enlarged section whose width increases along the direction from the proximal end to the distal end, an unchanged section with unchanged width, and a connecting section whose width is narrowed along the direction from the proximal end to the distal end. The connection position of the first branch channel 310 and the expansion section 220 is located within the enlarged section of the expansion section 220.

Referring to FIG. 5b and FIG. 5c, the lateral channel 140 includes a second branch channel 320 that intersects with the exit section 230. In the drawings, the second branch channel 320 is set to be bilateral symmetry. The connection position of the second branch channel 320 and the exit section 230 is adjacent to the expansion section 220. The second branch channel 320 can be divided into a first section 321 and a second section 322 that are communicated with each other and arranged along the direction from the proximal end to the distal end. The first section 321 extends along the length direction of the reference plane, and the second section 322 converges from the distal end of the first section 321 towards the exit section 230 and directly connects the proximal end of the exit section 230. Furthermore, the width of the first section 321 is greater than the width of the second section 322, and is less than or equal to half of the width of the second section 322. The width of the first section 321 is less than the width of the second fluid inlet, for example may be 50µm. A distance between two opposite side edges of the first section 321 in the width direction W is D1, and D1 may be 600µm, for example. Along the direction from the proximal end to the distal end, the width of the second section 322 gradually decreases or remains unchanged. For example, the dotted line in FIG. 5c may be taken as a boundary of the second section 322, and the width of the second section 322 remains unchanged.

Specifically, the lateral channel 140 is divided into a first branch channel 310 and a second branch channel 320 in an extension path along the direction from the proximal end to the distal end. The two branch channels are symmetrically arranged in the width direction W. The first branch channel 310 is divided into a first sub-channel 311 and a second sub-channel 312 on an extension path along the direction from the proximal end to the distal end. The distal ends of the first sub-channel 311 and the second sub-channel 312 are both connected to the enlarged section of the expansion section 220. The distal end of the first sub-channel 311 is located at an upstream of the distal end of the second sub-channel 312. The width of the first sub-channel 311 is D2, and the width of the second sub-channel 312 is D3. The width D2 is twice the width D3 or equal to D3. For example, D2 may be 20µm, and D3 may be 20µm or 10µm correspondingly.

It can be seen from the drawings that the extension direction of the second sub-channel 312 is consistent with that of the main channel, and the extension direction of the first sub-channel 311 is set at an angle with that of the main channel, for example, the angle is 30~60 degrees. As mentioned above, in FIG. 2a to FIG. 5b, the first fluid inlet is configured to input liquid-phase fluid, and the second fluid inlet is configured to input gas-phase fluid.

Referring to FIG. 6a and FIG. 6b, in a preferred embodiment, the main channel 130 includes an extension section 210, an expansion section 220 and an exit section 230 that are communicated with each other in sequence. The width variation trends and definition principles of the extension section 210, the expansion section 220 and the exit section 230 may refer to the embodiment shown in FIG. 3a, or refer to the dotted line in FIG. 6a.

The first fluid inlet at the proximal end side is used to input the gas-phase fluid, and the two second fluid inlets at the lateral side are used to input the liquid-phase fluid.

The distribution structure is realized by the distribution member 400. Compared to the solution shown in FIG. 3a where the distribution member is disposed in the lateral channel, the distribution member 400 in this embodiment is located in the expansion section 220 and adjacent to the outlet 120 of the extension section 210. The distribution members 400 are centrally arranged on the proximal end side of the expansion section 220. In the length direction of the reference plane, the overall length of the centrally arranged distribution members 400 is smaller than that of the expansion section 220.

The distribution members 400 are arranged in multiple rows along the direction from the proximal end to the distal end at the same interval, for example, 4 rows with an interval may be, for example, 10µm. Each row includes multiple convex columns 405 which are arranged with the same intervals and have a square cross-section on the reference plane. The intra-row intervals of the convex columns 405 may be, for example, 10µm. The multiple rows of convex columns 405 are arranged in a staggered manner, and the intra-row intervals of the multiple rows of convex columns are not communicated in the length direction of the reference plane.

The two second fluid inlets are located at a proximal end of a connection part of the extension section 210 and the expansion section 220. The lateral channel 140 starts from the second fluid inlet and then makes an arc turn, roughly points to the distal end and gradually approaches the expansion section 220 along a straight path until it intersects and communicates with the expansion section 220.

It can be understood that when the main channel 130 is used for flowing gas-phase fluid, the centrally arranged distribution members 400 can better change the direction of the gas-phase fluid, so that the gas-phase fluid interact with the liquid-phase fluid coming from the lateral side. Due to the pre-dispersion of the gas-phase fluid, it can further reduce damage to biological cells serving as therapeutic substances and improve cell survival rate.

The flow mixing structure provided in each embodiment of the present application can take into account both the nebulization effect and the cell survival rate. Taking lung lesions as an example, during nebulization, the lesion area is evenly adhered to the nebulizer (usually an aerosol) to avoid aggregation of droplets. This can save medication dosage, shorten treatment time, and reduce the side effects of nebulized substances on the human body. In addition, a good nebulization effect can make the nebulized substances float a certain distance in the air, solving the problem that the nebulized substances cannot reach the lung lobe lesions.

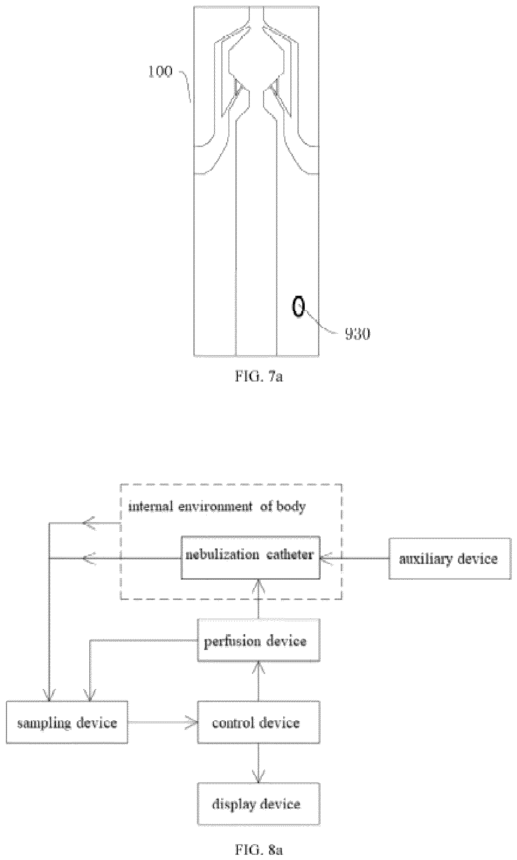

The embodiment shown in FIG. 6a corresponds to the three-dimensional view shown in FIG. 1e. When the nebulization chip shown in FIG. 6a is used, biological cells with a particle size of 50µm are atomized. Under the experimental conditions of a liquid-phase flow rate of 1 mL/min and a pressure of the gas-phase fluid of 0.15MPa, the experimental data of the cell mortality rate of the staining test is better, and the cell survival rate is above 90%. Referring to FIG. 4a and FIG. 7a, the microfluidic chip in each embodiment of the present application may be provided with a mark 930. The mark is provided on a side of the etched unit chip facing another unit chip for easy distinction and identification. The distinguishing method may be, for example, that the numbers or shapes of the marks are different in different embodiments.

Based on the above description, it is not difficult to understand that the present application discloses an interventional catheter, including a catheter body 900, wherein one end of the catheter body 900 is a proximal end 901, and another end of the catheter body 900 is a distal end 902 that can extend into the bronchus, an interior of the catheter body 900 is configured with a flow channel that can transport fluid from the proximal end 901 to the distal end 902, a flow mixing structure 910 is provided at the distal end 902 of the catheter body 900, and fluids in the flow channels are mixed in the flow mixing structure 910 and then output;
the flow mixing structure 910 at least includes a first fluid inlet 111, a second fluid inlet 112 and an outlet 120, and each fluid inlet 110 is independently configured with a flow channel in the catheter body 900;
a microfluidic chip 920 is provided in the fluid mixing structure 910, the first fluid inlet 111 is located at the proximal end side of the microfluidic chip 920, and the second fluid inlet 112 is located at a lateral side of the microfluidic chip 920, an interior of the microfluidic chip 920 is configured with a chip channel structure intercommunicating the outlet 120 with each fluid inlet 110, the chip channel structure includes:
   a main channel 130 with one end thereof communicating with the first fluid inlet 111 at the proximal end side and another end thereof communicating with the outlet 120;
   a lateral channel 140 with one end thereof communicating with the second fluid inlet 112 at the lateral side and another end thereof communicating with the main channel 130; and
   a distribution structure capable of acting on at least a portion of the fluid being arranged in the chip channel structure.

Similarly, the microfluidic chip 920 independently includes the following features: the distribution structure is located at the intersection portion of the main channel 130 and the lateral channel 140; the flow channel width of the lateral channel 140 has a narrowing part; the lateral channel 140 has an extension trend line, and the flow channel width of the lateral channel 140 gradually narrows along the extension trend line; the width of the lateral channel 140 continuously decreases from upstream to downstream; the intersection positions of the lateral channel 140 and the main channel 130 are junctions, and the orientation of at least one junction is perpendicular to the extension direction of the main channel 130. A throat part is provided on the main channel or the lateral channel, and the size of the throat part is smaller than that of the channel at two sides of the throat portion. At least one of the fluids flows to a droplets outlet through the throat part.

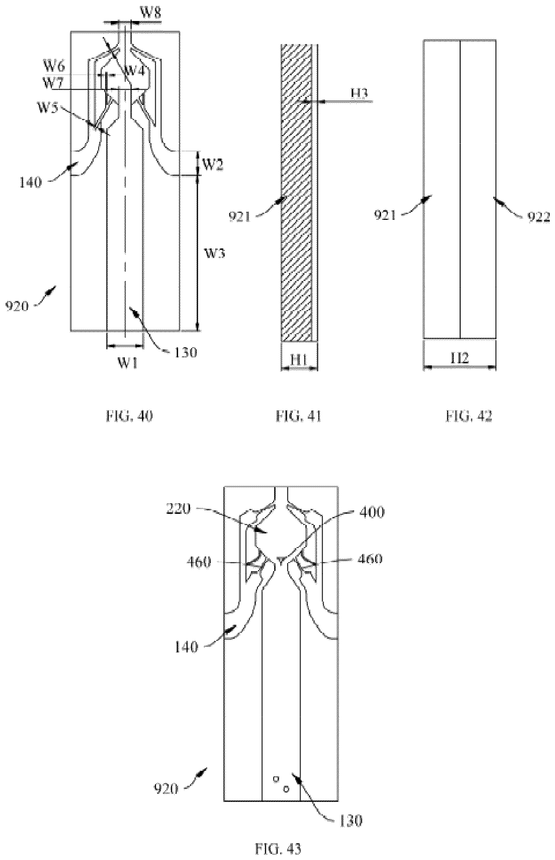

Referring to FIG. 40 to FIG. 42, a microfluidic chip 920 is disclosed in the figures. A length-to-width ratio of this microfluidic chip 920 ranges from 2.5 to 5, preferably ranges from 3 to 4. The ratio shown in the figures is 2.5:0.9. A width W1 of the main channel 130 is preferably 0.2 to 0.5mm. In actual products, the width W1 is preferably 0.25 to 0.45mm. In the drawings, the width W1 is 0.3mm. The throat part of the main channel has a width W7 less than the width W1 of the main channel 130. The width W7 of the throat part of the main channel is preferably 0.05 to 0.15mm. In actual products, the width W7 is preferably 0.08mm to 0.12mm. In the drawings, the width W7 is 0.1mm. The droplets outlet has a width W8 which may be the same as the width W7 of the throat part of the main channel, or may be different from the width W7. For example, the width W8 of the droplets outlet may deviate from the width W7 of the throat part of the main channel by 5% to 15%. A width W2 of the lateral channel 140 is preferably 0.1 to 0.3mm. In actual products, the width W2 is preferably 0.15 to 0.25mm. In the drawings, the width W2 is 0.2mm. A distance W3 between the lateral channel 140 and the proximal end side of the microfluidic chip 920 is preferably 0.5 to 1.8mm. In actual products, the distance W3 is preferably 0.8 to 1.5mm. In the drawings, the distance W3 is 1.3mm. A width W4 of the branch channel is preferably 0.01 to 0.05mm. In actual products, the width W4 is preferably 0.015 to 0.03mm. In the drawings, the width W4 is 0.02mm. A width W5 of the branch channel may be set to have a range the same as that of the width W4 of the branch channel, or may be different from the width W4. For example, the width W5 of the branch channel may deviate from the width W4 of the branch channel by 5% to 15%. A width W6 of the sub-channel is less than the width W5 of the branch channel. The width W6 of the sub-channel is preferably 0.005 to 0.015mm. In actual products, the width W6 is preferably 0.008 to 0.012mm. In the drawings, the width W6 is 0.01mm. In terms of thickness, a thickness H1 of the unit chip etched with the flow channel is preferably 0.2 to 0.5mm. In actual products, the thickness H1 is preferably 0.25 to 0.35mm. In the drawings, H1 is 0.3mm. In the drawings, an etched depth H3 is 0.05mm. An overall thickness H2 of the microfluidic chip 920 is preferably 0.4 to 1.0mm. In actual products, the thickness H2 is preferably 0.55 to 0.85mm. In the drawings, the thickness H2 is 0.6±0.02mm. In this embodiment, the microfluidic chip 920 is designed based on the double nebulization principle, and the lateral channel 140 and the main channel 130 intersect with each other at least twice. A portion of the main channel 130 is a widened expansion section, and the lateral channel 140 intersects with the main channel 130 at least once in the expansion section. Furthermore, the lateral channel 140 intersects with the main channel 130 at least once after the expansion section. In the drawings, the lateral channel 140 includes branch channels, and independently intersects with the main channel twice through the branch channels in the expansion section. Two lateral channels 140 are provided at two sides of the main channel 130, respectively, and each lateral channel 140 independently intersects with the main channel 130. A droplets size after nebulization is in range of 10~30µm, and each liquid-phase flow channel has a size larger than 100µm. The microfluidic chip of this embodiment allows the medication solution containing particles to pass through, and thus can be used in the medication spraying system of the human body cavity mentioned above.

Referring to FIG. 43, a microfluidic chip 920 is disclosed in the figure. This microfluidic chip 920 has an overall size similar to that of the embodiment shown in FIG. 40 to FIG. 42, wherein a portion of the main channel 130 is a widened expansion section 220, at least a portion of the lateral channel 140 intersects with the expansion section 220, and the distribution member 400 is located in the expansion section 220 and provided with at least a first distribution surface and a second distribution surface facing towards a flowing direction of the fluid in the main channel 130. A fluid acceleration structure 460 is provided on the lateral channel 140 and located upstream of at least one junction of the main channel 130 and the lateral channel 140. The fluid acceleration structure 460 is actually a Laval nozzle that is gradually converging and expanding, which can effectively increase the fluid velocity in the lateral channel 140. In this embodiment, the fluid in the lateral channel 140 is gas. Compared with the previous embodiment, the microfluidic chip 920 of this embodiment is designed based on the double nebulization principle and the fluid acceleration structure. The gas is accelerated after flowing through the fluid acceleration structure 460, which is beneficial to gas-liquid mixing. The nebulized particle size is made smaller, so the droplets particle size after nebulization in this embodiment is in a range of 8~25µm.

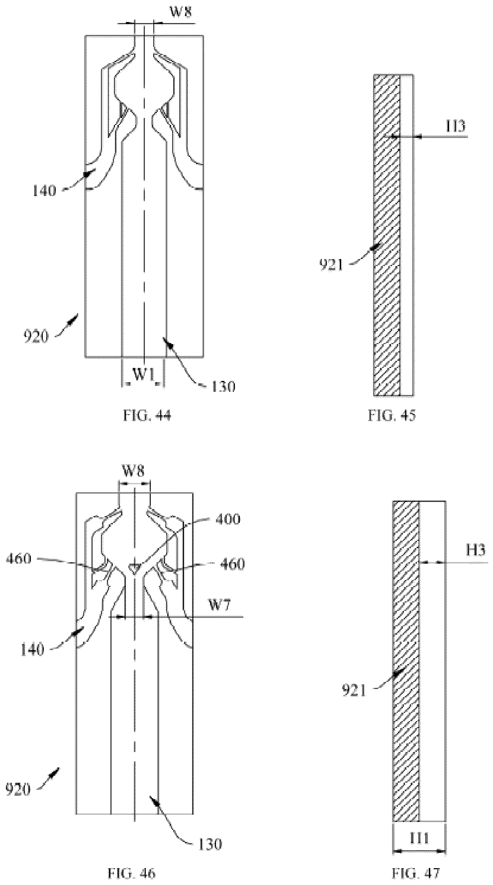

Referring to FIG. 44 to FIG. 45, a microfluidic chip 920 is disclosed in the figures. This microfluidic chip 920 has an overall size similar to that of the embodiment shown in FIG. 40 to FIG. 42, wherein an etched depth H3 is 0.08mm to 0.12mm. A width W8 of the droplets outlet is 0.1mm to 0.2mm. A width W1 of the main channel 130 is in range of 0.2mm to 0.4mm. In the drawings, the etched depth H3 is preferably 0.1mm, the width W8 of the droplets outlet is preferably 0.15mm, and the width W1 of the main channel 130 is preferably 0.32mm. This embodiment is designed based on the principle of double nebulization. The size of the liquid-phase flow channel is larger than that of other embodiments, which can effectively reduce the nebulization pressure of the liquid-phase fluid. Similarly, the microfluidic chip of this embodiment can allow the medication solution containing particles to flow through, so that can be used in the medication spraying system of the human body cavity mentioned above. At the same time, the size of the liquid-phase flow channel is larger than that of other embodiments, which can effectively reduce the nebulization pressure of the liquid-phase, thereby meeting specific usage conditions. For example, when the therapeutic substance is cells, the lower nebulization pressure and the larger flow channel size can effectively reduce the damage to the cells. The droplets diameters after nebulization in this embodiment are in range of 20µm~50µm, so the microfluidic chip of this embodiment may be used in the cell spraying system of the human body cavity mentioned above.

Referring to FIG. 46 to FIG. 47, a microfluidic chip 920 is disclosed in the figures. This microfluidic chip 920 has an overall size similar to that of the embodiment shown in FIG. 40 to FIG. 42, wherein a throat part of the main channel has a width W7 in range of 0.1mm to 0.2mm. A width W8 of the droplets outlet is 0.15mm to 0.3mm. An etched depth H3 is 0.1mm to 0.25mm. A thickness H1 of the unit chip with etched flow channels is 0.25mm to 0.55mm. In the drawings, the width W7 of the throat part of the main channel is preferably 0.14mm, the width W8 of the droplets outlet is preferably 0.24mm, the etched depth H3 is preferably 0.2mm, and the thickness H1 of the unit chip with etched flow channel is preferably 0.4mm. It is not difficult to see that the sizes of the flow channels of this embodiment are increased compared with other embodiments. In addition, a fluid acceleration structure 460 is provided on the lateral channel 140 and located upstream of at least one junction of the main channel 130 and the lateral channel 140. The fluid acceleration structure 460 is actually a Laval nozzle that is gradually converging and expanding, which can effectively increase the fluid velocity in the lateral channel 140. In this embodiment, the fluid in the lateral channel 140 is gas. At the same time, a portion of the main channel 130 is a widened expansion section 220, at least a portion of the lateral channel 140 intersects with the expansion section 220, and the distribution member 400 is located in the expansion section 220 and provided with at least a first distribution surface and a second distribution surface facing towards the flowing direction of the fluid in the main channel 130. This embodiment is designed based on the double nebulization principle and the fluid acceleration structure, which greatly increases the sizes of the flow channels, so that it can atomize liquids with higher viscosity, such as hydrogels. The droplets diameters after nebulization in this embodiment are in range of 20~60µm, so that the microfluidic chip of this embodiment may be used in the high-viscosity fluid spraying system in the human body cavity mentioned above.

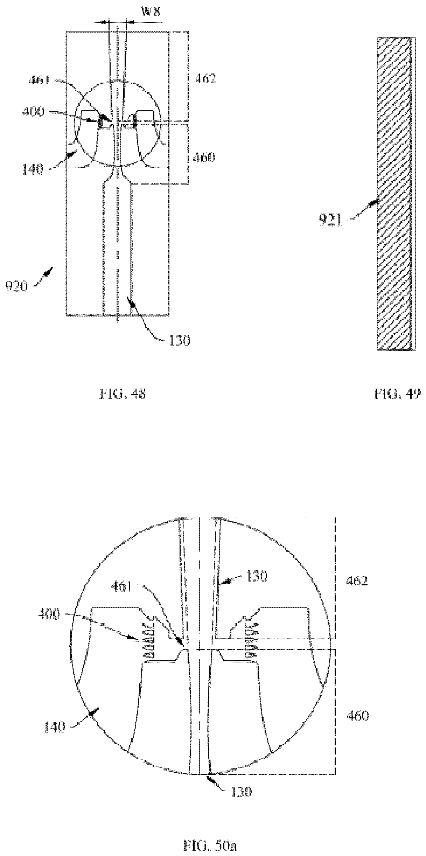

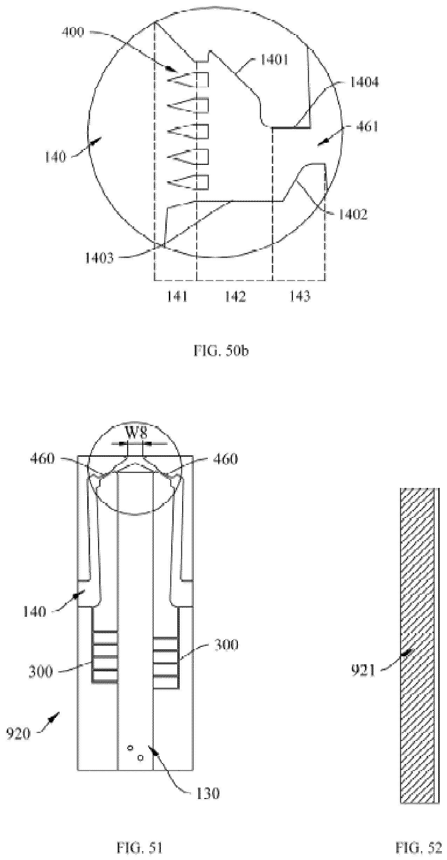

Referring to FIG. 48 to FIG. 50b, a microfluidic chip 920 is disclosed in the figures. This microfluidic chip 920 has an overall size similar to that of the embodiment shown in FIG. 40 to FIG. 42, wherein a width W8 of the droplets outlet is preferably 0.15mm, and an overall thickness H2 of the microfluidic chip 920 is preferably 0.6±0.02mm. Compared with the other embodiments, the main channel 130 in this embodiment transports gas and is provided with a fluid acceleration structure 460. The fluid acceleration structure 460 is located upstream of at least one junction of the main channel 130 and the lateral channel 140, and this junction is provided with at least one narrow opening 461 through which and the main channel 130 or the lateral channel 140 intersects with the other. The lateral channel 140 transports liquid and a distribution structure is provided at one end of the lateral channel 140 close to the main channel 130. The distribution structure is configured as distribution members 400 arranged in an array. Along the flowing direction of the fluid, a size of a cross-section of each distribution member 400 gradually increases. The distribution member can sort out the turbulence in the fluid, which improves the flow stability of the fluid, ensures the stability of the nebulization process, achieves sufficient nebulization effect, and controls the distribution range of the droplets particle size. The main channel 130 is provided with a sudden expansion section 462 downstream of the fluid acceleration structure 460. In FIG. 50a, in order to better show the sudden expansion section 462, the extension trend of the fluid acceleration structure 460 is extended by a dotted line. Compared with the extension trend of the fluid acceleration structure 460, it can be clearly observed that the sudden expansion section 462 has a larger chamber diameter and a larger internal volume. The markings on the sudden expansion section 462 and the fluid acceleration structure 460 in FIG. 50a are for better indicating the boundary of close portions of the sudden expansion section 462 and the fluid acceleration structure 460, and do not limit their axial lengths, especially the ends of the sudden expansion section 462 and the fluid acceleration structure 460that are far away from each other. For example, in the embodiment shown in FIG. 50a, the axial lengths of the sudden expansion section 462 and the fluid acceleration structure 460 are longer than that shown in FIG. 49, wherein the distal end side of the sudden expansion section 462 extends to the distal end side of the microfluidic chip and forms the droplets outlet. In terms of proportion, an internal volume of the sudden expansion section 462 is at least 15% larger than an internal volume of the fluid acceleration structure. The junction with the narrow opening 461 is arranged at the sudden expansion section 462, as shown in the drawings, this junction is arranged at the proximal end side of the sudden expansion section 462. That is, the sudden expansion section 462 is set close to the fluid acceleration structure 460, and a chamber diameter of the sudden expansion section 462 is at least 10% larger than a chamber diameter at the outlet 120 of the fluid acceleration structure 460. In the embodiment of the present application, the design is based on the principle of pressure difference nebulization. The gas is accelerated through the fluid acceleration structure in the middle and interacts with the liquid-phase in the sudden expansion section 462. The suddenly expanded flow channel space of the sudden expansion section 462 and the high-speed gas cooperate to form the pressure difference. Through differential pressure nebulization, the size of small droplets is more concentrated, which can effectively improve the concentration of the distribution range of droplets particle size after nebulization. In this embodiment, the particle sizes of the nebulized droplets are in range of 20~60µm, of which at least 70% are in range of 20~40µm. The microfluidic chip of this embodiment may be used in the cell spraying system of the human body cavity mentioned above. In this embodiment, the cells are preferably exosomes.

Based on the above content, this embodiment discloses an interventional catheter, including a catheter body 900, wherein one end of the catheter body 900 is a proximal end 901, and another end of the catheter body 900 is a distal end 902 that can extend into the bronchus, a microfluidic chip 920 is provided in the catheter body 900, and the microfluidic chip 920 includes:
a first fluid inlet 111 located at a proximal end side of the microfluidic chip 920;
two second fluid inlets 112 being arranged at two opposite lateral sides of the microfluidic chip 920, respectively;
an outlet 120 located at a distal end side of the microfluidic chip 920;
an interior of the microfluidic chip 920 is configured with a chip channel structure intercommunicating the outlet 120 with each fluid inlet 110, wherein the chip channel structure includes:
   a main channel 130 with one end thereof communicating with the first fluid inlet 111 and another end thereof communicating with the outlet 120;
   a lateral channel 140 with one end thereof communicating with the second fluid inlet 112 and another end thereof communicating with the main channel 130, a plurality of distribution members 400 being provided in the lateral channel 140 and arranged at intervals.

In terms of details, there are two lateral channels 140 symmetrically distributed on two sides of the main channel 130, and each lateral channel 140 is connected to the middle and downstream of the main channel 130. The lateral channel 140 has a bending portion pointing to the distal end 902 at the second fluid inlet 112, and a side facing towards the main channel 130 and at a distal end of the bending portion is connected to the main channel through an intersection section.

The distribution members 400 are sequentially arranged in the intersection section along the length direction of the main channel 130.

The main channel 130 is provided with a fluid acceleration structure 460 and a sudden expansion section 462 which are arranged in sequence along the flowing direction of the fluid, wherein the intersection section is connected to the sudden expansion section 462 and a connection portion is adjacent to the fluid acceleration structure 460. As shown in FIG. 2d, the intersection section includes multiple stages of narrowing parts in sequence, and the distribution members 400 are located between two adjacent stages of narrowing parts. In terms of the narrowing form, the width of the flow channel changes by 20 to 60% before and after each stage of narrowing part. It is specifically manifested as a change in the inner diameter of the flow channel. The narrowing process may be uniform narrowing or sudden narrowing. It should be noted that the narrowing comparison is the inner diameter of the flow channel before and after narrowing. During the narrowing process, the width of the flow channel may become larger. For example, as shown in FIG. 2d, the narrowing parts includes three stages, namely, a primary narrowing part 141, a secondary narrowing part 142, and a tertiary narrowing part 143, wherein the inner diameters of the three stages decrease successively. In the drawings, the distribution members 400 are located between the primary narrowing part 141 and the secondary narrowing part 142. In the drawings, the secondary narrowing part 142 first expands and then narrows compared to the outlet of the primary narrowing part 141. A main purpose of the expansion of the secondary narrowing part 142 is to provide a larger space for the rear end of the distribution members 400, so that the fluid can flow through the distribution members 400 more smoothly.

Overall, the setting of the multi-stage narrowing parts can adjust the flow indicators and distribution effects of the fluid in stages. For example, the primary narrowing part 141 in the drawings can better guide the fluid to the distribution members 400, the secondary narrowing part 142 can receive the fluid combed by the distribution members 400 and further compress and transport the fluid, and the tertiary narrowing part 143 can further compress and transport the fluid on the basis of the secondary narrowing part 142 and adjust the intersection angle of the fluid and the main channel, thereby realizing the efficient and accurate nebulization process of the fluids in the main channel 130 and the lateral channel 140.

An extension direction of the whole intersection section is along the width direction of the main channel 130, and each narrowing part has two opposite side walls, wherein at least one side wall is arranged inclined relative to the extension direction so that the narrowing part is gradually narrowed. For the two side walls, one is parallel to the extension direction, and the other is set to be inclined to the extension direction. For example, as shown in FIG. 2d, the side walls 1401, 1402 are inclined to the extension direction, and the side walls 1403, 1404 are parallel to the extension direction. The side walls parallel to the extension direction are for better counteracting nebulization with the fluid in the main channel 130, and the side walls inclined to the extension direction are for better bundling of the fluid in the lateral channel 140. In the two most downstream stages of narrowing parts, the inclined side walls are on different sides of the flow channel. In the drawings, it is specifically shown that the side walls 1401 and 1402 are located on two sides of the flow channel, respectively. Such setting can realize the combing of the flowing direction of the fluid and avoid the deflection of the flowing direction of the fluid due to the narrowing structure. More importantly, the intersection angle of the fluid in the lateral channel 140 and the fluid in the main channel 130 is ensured, which in turn ensures the nebulization effect.

In this embodiment, the distribution members 400 are in the shape of pointed teeth, and the size of the cross-section of the distribution member 400 gradually increases in the flowing direction of the fluid. On the downstream side of the distribution members 400, the distribution members 400 occupy 30~70% of the width of the flow channel. The distribution members can sort out the turbulence in the fluid, thereby improving the flow stability of the fluid, ensuring the stability of the nebulization process, and controlling the distribution range of the droplets particle size.

The fluid in the lateral channel 140 is narrowed at multiple stages of the narrowing parts and combed by the distribution members 400, so that it can smoothly and stably flow into the main channel 130 while ensuring the flow rate, and mix with the fluid in the main channel 130 to achieve a sufficient nebulization effect.

Referring to FIG. 51 to FIG. 52, a microfluidic chip 920 is disclosed in the figures. This microfluidic chip 920 has an overall size similar to that of the embodiment shown in FIG. 48 to FIG. 50b, wherein a width W8 of the droplets outlet is preferably 0.12mm. Compared with the previous embodiment, the lateral channel 140 in this embodiment transports gas, and the lateral channel is configured with an independent branch channel 300. In the drawings, two lateral channels 140 are provided and are respectively arranged on two sides of the main channel 130. Each of the lateral channels 140 is provided with an independent branch channel 300. The independent branch channel 300 includes multiple sub-channels arranged in an array. The multiple sub-channels of the two branch channels 300 are arranged opposite to each other and communicate with the main channel 130, wherein the multiple sub-channels of the two branch channels 300 are arranged at intervals and staggered, which can realize the disturbance of the fluid in the lateral channel to the fluid in the main channel. In the embodiment shown in the drawings, along the extension direction of the main channel 130, the main channel 130 and the lateral channel 140 intersect with each other at least twice, wherein the first intersection is in the middle and upper reaches of the main channel 130 to implement disturbance, and the second intersection is near the outlet 120 to implement nebulization.

The first intersection can play a role of pre-dispersion, and the setting of double mixing can effectively control the particle size of the nebulized liquid and the distribution range of the particle size of the nebulized liquid. The multiple sub-channels, arranged at intervals and staggered, can enable the fluid to be sequentially impacted from two sides of the main channel, thereby achieving better nebulization, improving the premixing effect, and avoiding the blockage of the main channel by sub-channels set in opposite directions. In this embodiment, the fluid transported in the lateral channel is gas, and the fluid transported in the main channel is liquid, wherein the sub-channels make the gas impact the liquid from left and right sides in sequence, allowing the liquid to be better dispersed and mixed.

At the outlet, the fluid acceleration structure and the sudden expansion section are arranged in groups and are respectively arranged in the corresponding lateral channels 140, wherein the two sudden expansion sections are arranged to intersect in the main channel 130. In this embodiment, a fluid acceleration structure 460 is provided in the lateral channel 140. The fluid acceleration structure 460 is located upstream of at least one junction of the main channel 130 and the lateral channel 140. A distribution member 400 is disposed in the main channel 130. The distribution member 400 and the side walls of the main channel 130 form at least two narrow openings 461 corresponding to the two sudden expansion sections 462, respectively. According to the drawings, a portion of a side wall of the sudden expansion section 462 is provided by the distribution member 400. From another aspect, the distribution member 400 divides the main channel into two narrow openings 461 and two sudden expansion sections 462 corresponding to the two narrow openings 461, wherein the two sudden expansion sections 462 correspond to the fluid acceleration structures 460 of the two flow channels 140. The above arrangement can effectively reduce the processing difficulty of the microfluidic chip and improve the flow channel dimensional accuracy. By means of controlling the dimensional parameters of the main channel and the distribution members, the precise dimensional parameters of the narrow openings and the sudden expansion sections can be obtained, and the internal structure is compact and stable. At the same time, compared with small-sized distribution members, the large-sized distribution members in this embodiment have the advantages of excellent structural strength, being able to withstand greater fluid pressure, and being adaptable to meet more design needs. In terms of intersection relationship, one of the main channel 130 and the lateral channel 140 intersects with the other through two narrow openings 461. The lateral channel 140 is provided with a sudden expansion section 462 downstream of the fluid acceleration structure 460. In FIG. 53, it can be clearly observed that a chamber diameter of the expansion section 462 is larger and has a larger internal volume. In terms of proportion, the internal volume of the sudden expansion section 462 is at least 15% larger than the internal volume of the fluid acceleration structure. The junction with the narrow opening 461 is arranged at the sudden expansion section 462. In the drawings, the junction is arranged at the proximal end side of the sudden expansion section 462. That is, the sudden expansion section 462 is disposed close to the fluid acceleration structure 460, and the chamber diameter of the sudden expansion section 462 is at least 10% larger than the chamber diameter at the outlet 120 of the fluid acceleration structure 460. This embodiment is designed based on the principle of pressure difference nebulization. Compared with the previous embodiment, the gas-phase fluid is transferred to the lateral channels on two opposite sides, and the main nebulization structure is concentrated at the outlet. The nebulized droplets caused by two pressure difference will collide at the outlet, forming smaller droplets while ensuring a relatively high concentration of droplets size distribution. In this embodiment, the particle sizes of the nebulized droplets are distributed in range of 5~40µm, of which at least 70% are distributed in range of 5~15µm. The microfluidic chip of this embodiment may be used for the cell spraying system of the human body cavity and the inactivated virus spraying system of the human body cavity mentioned above.

Based on the above embodiment, the present application discloses an interventional catheter, including a catheter body 900, wherein one end of the catheter body 900 is a proximal end 901, and another end of the catheter body 900 is a distal end 902 that can extend into the bronchus, a microfluidic chip 920 is provided in the catheter body 900, wherein the microfluidic chip 920 includes:
a first fluid inlet 111, located at a proximal end side of the microfluidic chip 920;
a second fluid inlet 112, located at a lateral side the microfluidic chip 920;
an outlet 120, located at a distal end side of the microfluidic chip 920;
an interior of the microfluidic chip 920 being configured as a chip channel structure intercommunicating the outlet 120 with each fluid inlet 110, wherein the chip channel structure includes:
   a main channel 130 with one end thereof communicating with the first fluid inlet 111 and another end thereof communicating with the outlet 120;
   a lateral channel 140 with one end thereof communicating with the second fluid inlet 112 and another end thereof communicating with the main channel 130, the lateral channel 140 being configured with branch channels 300 and intersecting with the main channel 130 via the branch channels 300.

In terms of design details, the lateral channel 140 includes:
a trunk channel extending from the second fluid inlet 112 to the distal end 902 until it intersects with the main channel 130;
a plurality of branch channels 300 each having an end communicating with the trunk channel at a communication portion being adjacent to the second fluid inlet 112, and another end extending toward the proximal end 901 and branching into a plurality of sub-channels, distal ends of the sub-channels intersecting at the middle and upper reaches of the main channel 130. The trunk channel has a bending portion at the second fluid inlet 112 and extends to the distal end 902 through the bending portion. One end of the branch channel 300 is connected to the proximal end side of the bending portion. The branch channels 300 are in a comb-like structure as a whole, and distal ends of each sub-channel is vertically connected to the main channel 130. There are two lateral channels 140 arranged on two opposite sides of the main channel 130, respectively, and the distal ends of the sub-channels in the two lateral channels 140 are staggered. There are two lateral channels 140 arranged on two opposite sides of the main channel 130, and the trunk channels of the two lateral channels 140 intersect with the main channel 130 respectively to from a first junction and a second junction. The two lateral channels 140 extend independently from the first junction and the second junction to the outlet 120 and intersect with each other at a position near the outlet 120. In the main channel 130, a distribution structure 400 is provided near the outlet 120. Along the width direction of the main channel 130, the distribution structure 400 closes the middle part of the main channel 130 and narrow openings 461 are formed on two sides between the distribution structure 400 and the side wall of the main channel 130. The first junction and the second junction are located corresponding to the narrow openings 461, respectively. The lateral channel 140 is provided with a fluid acceleration structure 460 and a sudden expansion section 462 which are arranged in sequence along the flowing direction of the fluid, wherein the narrow openings 461 are connected to the sudden expansion section 462, and the connection portions are adjacent to the fluid acceleration structure 460. The distribution member 400 has a triangular cross-sectional shape. The main channel 130 extends from the first fluid inlet 111 to the distribution member 400, extending straightly with a constant cross section. An angle between extension trend lines of the two lateral channels 140 extending from the first intersection and the second intersection to the outlet 120 is an obtuse angle. The outlet 120 has a trend of expansion, wherein an expansion angle corresponding to the angle of the trend lines.

Similarly, it may also be understood that the present application discloses a nebulization method based on the microfluidic chip 920, including:
inputting liquid-phase fluid through the microfluidic chip 920;
inputting gas-phase fluid through the microfluidic chip 920, wherein the gas-phase fluid is divided into two streams and each stream is independently guided to meet with the liquid-phase fluid, so as to form two nebulized flows;
guiding the two nebulized flows to meet the liquid-phase fluid inside the microfluidic chip 920 and then output.

In detail, at least a portion of each stream of gas-phase fluid is pre-divided and merges with the liquid-phase fluid at a relatively upstream position to form a turbulent flow; while the remaining portion of each stream of gas-phase fluid merges with the liquid-phase fluid at a relatively downstream position to form nebulization. An angle between flowing trend lines of the two nebulized flows before they meet is an obtuse angle.

The various embodiments mentioned above have their own characteristics and certain commonalities. According to the above description, the following characteristics may be summarized. Regarding the setting manner of the distribution structure, the distribution structure is in at least one of the following manners: manner a: a plurality of branch channels 300 connected to the lateral channel 140, wherein the lateral channel 140 intersects with the main channel 130 via the plurality of branch channels 300; manner b: a plurality of distribution members 400 arranged at intervals or separately set in the main channel 130 and/or the lateral channel 140.

Regarding the fluid acceleration structure 460 and the sudden expansion section 462, the sudden expansion section 462 is formed by the expansion of the flow channel where the sudden expansion section 462 is located. In terms of transition, the sudden expansion section 462 and the outlet of the fluid acceleration structure 460 have a smooth transition or a step transition. It should be noted that smooth or non-smooth transition emphasized here is based on the fluid dynamics design principle, in order to strengthen or adjust the effect of the sudden expansion section 460 on the flowing of the fluid. For example, structures such as burrs, straight edges, steps and etc. formed due to process limitations in common industrial products cannot be regarded as the step transition as described here. For another example, conventional corners and other settings cannot be regarded as the smooth transition as described here. The key here is to deliberately process and shape it into this form. In the flowing direction of the corresponding flow channel, the expansion trend of the sudden expansion section 462 compared with the fluid acceleration structure 460 is also designed. According to the overall size relationship of the sudden expansion section 462, when the size of the sudden expansion section 462 is significantly larger than the opening size of the main channel, in order to achieve structural compatibility, the expansion trend of the sudden expansion section 462 will be reduced on the distal end side; when the size of the sudden expansion section 462 is significantly smaller than the size of the droplets outlet, the sudden expansion section 462 can maintain its own expansion trend or further increase its expansion trend. In addition to the size relationship, it is also related to the position of the sudden expansion section 462 on the microfluidic chip. When the sudden expansion section 462 is close to the droplets outlet, the axial expansion distance of the sudden expansion section 462 is limited. As the axial and radial expansion trends of the sudden expansion section 462 changes, the sudden expansion section 462 will undergo morphological changes. For example, in an embodiment that the expansion section 220 and the fluid acceleration structure 460 are provided in the main channel 130, when the expansion section 220 is close to the outlet of the fluid acceleration structure 460, the expansion section 220 may also have a certain effect as that of the sudden expansion section 462.

In combination with the above embodiments and referring to FIG. 8a, the present application provides a nebulized medication delivery system, including:
a perfusion device, configured for supplying a fluids containing a therapeutic substance, wherein the fluid includes a liquid-phase fluid and a gas-phase fluid, and at least one of the liquid-phase fluid and gas-phase fluid contains the therapeutic substance;
an interventional catheter, using the interventional catheter in the above technical solutions, wherein a proximal end of the interventional catheter is connected to the perfusion device for input the gas-phase fluid and the liquid-phase fluid, respectively;
a sampling device, configured for collecting state parameters of the fluids;
a controlling device, connected to the sampling device and configured for receiving the state parameters and controlling the perfusion device according to the state parameters.

Cooperating with the following device, it may be understood that the present application discloses a gas-liquid interventional device, wherein the interventional device includes:
a housing, on which a gas path connector and a liquid path connector are mounted, a pump chamber being provided at a bottom of the housing;
an air pump installed in the pump chamber, wherein the air pump includes a plurality of pumps connected in parallel and is connected to the gas path connector through a main pipe;
a cooling assembly, thermally coupled to the air pump;
a syringe including a cylinder installed in a housing and a piston slidably installed in the cylinder, wherein the cylinder has an outlet communicating with the liquid path connector;
a drive mechanism, linked to the piston of the syringe.

The interventional catheter adopts the interventional catheter in the above technical solutions, and the proximal end of the interventional catheter is respectively connected to the gas path connector and the liquid path connector, and is used to receive gas-phase fluid and liquid-phase fluid respectively. The detailed description of the device is given below and will not be repeated here. "Medication delivery" mentioned in each embodiment of the present application refers to the delivery of the therapeutic substance to the lesion site, wherein the therapeutic substance is described in detail in other embodiments. The interventional catheter may refer to the various embodiments of the present application. The fluid containing the therapeutic substance enters the interventional catheter through the perfusion device until it is applied to the lesion site in the human body. The perfusion device mainly provides power for the fluid, so as to obtain a certain flow rate which can be adjusted according to need, such as using a controllable fluid delivery pump, and etc. In terms of the structure and control method of the pump itself, existing technology may also be used. The perfusion device is configured according to the state and type of the fluid, and regulating devices may be set at the parts that need to be controlled. The fluid with the therapeutic substance can be either pre-formulated or mixed in real time during flowing of the fluid. The therapeutic substance itself depends on the purpose of treatment and the condition of the lesion. For example, the therapeutic substance itself is a fluid, or the therapeutic substance is loaded in a gas-phase fluid, or the therapeutic substance is loaded in a liquid-phase fluid. The interventional catheter may transport fluids with different phases, for example, the fluids including gas-phase fluid and liquid-phase fluid. The gas-phase fluid and liquid-phase fluid are preferably mixed and nebulized at the fluid mixing structure after entering the interventional catheter separately. In order to facilitate the operation of the interventional catheter, in one embodiment, the nebulized medication delivery system further includes an auxiliary device, which acts on the interventional catheter to change a relative position of the interventional catheter and the lesion site.

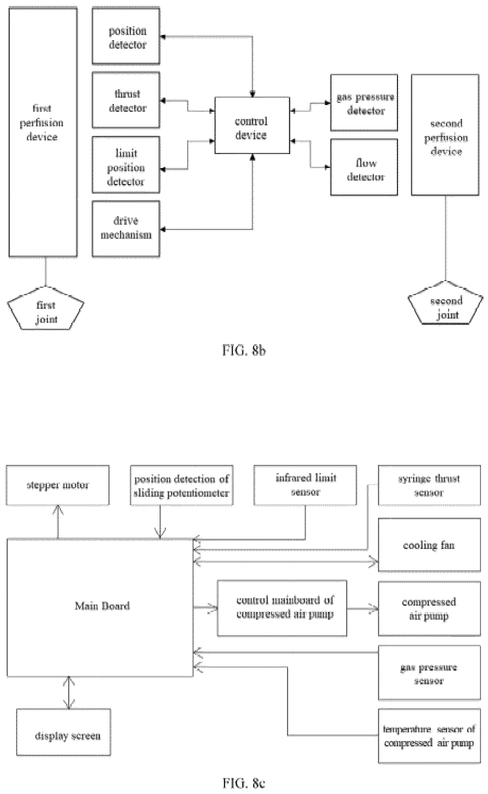

Referring to FIG. 8b, the perfusion device includes a first perfusion device and a second perfusion device. The microfluidic chip includes a plurality of inlets, at least one of the inlets is a gas-phase inlet and at least one of the inlets is a liquid-phase inlet. The first perfusion device includes a cylinder, a piston and a drive mechanism, wherein the cylinder is used to supply liquid-phase fluid and has an output port connected to a liquid-phase inlet; the piston is slidably mounted in the cylinder; and the drive mechanism pushes the piston to slide.

The first perfusion device may be, for example, an injection pump equipped with a syringe; the cylinder may be, for example, a cylinder of the syringe; the piston may be, for example, a push plug of the syringe, and the drive mechanism may be, for example, a stepping motor configured to push the piston to move linearly to achieve medication pushing of the first perfusion device. When in use, the liquid-phase fluid may be maintained with a flow rate of 1~4.5ml/min, for example, 3ml/min. The pushing force applied to the piston is 80~200N. Combined with the cross-sectional area of the piston, a pressure of the liquid-phase fluid is calculated to be 0.2~0.76MPa, for example, 0.4MPa.

The second perfusion device includes an air compressor and/or an air compression bottle. At least one of the air compressor and the air compression bottle is used to supply gas-phase fluid and is connected to the gas-phase inlet. When using the air compressor, the air is compressed to 1.5~2 bar, which is the necessary pressure for the gas-phase fluid. When using the air compressor bottle, it is sufficient to ensure that the pressure in the air compressor bottle is higher than the pressure required for the gas-phase fluid. The pressure of the gas-phase fluid is 0.15~0.4MPa, for example, 0.2MPa.

The first perfusion device is connected to one of the inner catheters, and the second perfusion device is connected to the other inner catheter. Taking the first inner catheter 950 conveying liquid-phase fluid as an example, the first inner catheter 950 of the catheter body is connected to the first perfusion device, and the second inner catheter 960 is connected to the second perfusion device, and vice versa.

The first perfusion device communicates with the first inner catheter 950 via a first joint as shown in FIG. 8b, and the second perfusion device communicates with the second inner catheter 960 via a second joint. A quick-release connector is provided at the interface where the second inner catheter 960 communicates with the second perfusion device. When the second perfusion device adopts the air compression bottle, the air compression bottle can be connected via the quick-release connector, which can enhance portability and facilitate quick replacement. The compressed air bottle, for example, has a volume of 500ml to 1L, a pressure of 0.2MPa, and is made of for example, metal. Correspondingly, a regulating valve may be provided on the pipeline connected to the second inner catheter 960, and the controlling device adjusts the pressure of the gas-phase fluid in the second inner catheter 960 through the regulating valve.

The sampling device may collect relevant parameters according to need, wherein the parameters are taken as a control basis. The state parameters of the fluid include at least one of temperature, pressure, and flow rate. To assist the operation, the sampling device further collects image signals and/or status signals of the lesion site. Image signals facilitate visualization and can be used as real-time reference and comparison, while status signals at the lesion site, for example electrical signals such as current and impedance, or temperature signals, can reflect the progress of treatment. In order to understand the physiological status of patients during the treatment, the sampling device further collects physiological signals from patients, for example, at least one of electrocardiogram signals, blood signals, and etc.

As a nebulization medication delivery system, it may relate to multiple specific devices. In order to obtain the status of each device as a monitoring or control basis, the sampling device further collects the operating status of related devices, such as the perfusion device and/or sampling device. Combined with specific device, it may be speed, working current, working temperature, pressure, and etc.

Referring to FIG. 8b to FIG. 8c, the sampling device includes a liquid-phase sampling device and a gas-phase sampling device, wherein the liquid-phase sampling device includes a position detector and a thrust detector. The controlling device receives signals from the liquid-phase sampling device, which may be used to feedback control the first perfusion device, and receives signals from the gas-phase sampling device, which may be used to feedback control the second perfusion device. The position detector is used to detect the position of the piston. The thrust detector is used to detect the pushing force of the piston, which may be understood as detecting the resistance during the operation of the syringe. The controlling device is used to receive signals from the position detector and thrust detector, so as to control the drive mechanism accordingly. The detector may use the sensors mentioned below, and the same applies to other descriptions.

Considering that the piston resistance values of different syringes are different, the first perfusion device selects several types of syringes with specified configurations, and the controlling device identifies and determines the type of syringe, and controls the drive mechanism accordingly. Specifically, after identifying the model and volume of the syringe, the controlling device adjusts and obtains the flow rate of the liquid-phase fluid in the first perfusion device through the signal feedback of the thrust detector. By means of obtaining the flow rate of the liquid-phase fluid to feedback control the drive mechanism, the adjustment of the flow rate of the liquid-phase fluid can be achieved. By means of obtaining the position of the piston at different times and detecting the moving distance of the syringe, the flow rate delivered by the first perfusion device can be obtained. The thrust detector, for example, can be fixedly installed between the drive mechanism and the piston.

The gas-phase sampling device includes a gas pressure detector for detecting the pressure of the gas-phase fluid. The controlling device is configured for receiving signals from the gas pressure detector and controlling the pressure of the gas-phase fluid accordingly. If the second perfusion device uses an air compressor, the controlling device detects the state parameters of the gas-phase fluid, controls the speed of the air compressor through closed-loop feedback, and adjusts the pressure of the gas-phase fluid delivered by the air compressor. If the second perfusion device uses the air compression bottle, the controlling device controls the pressure of the gas-phase fluid through feedback from the regulating valve. It can be understood that the pressure of gas-phase fluids is only a manifestation, and adjusting the pressure includes adjusting the flow rate of the gas-phase fluid. Furthermore, the gas-phase sampling device may further be equipped with a corresponding flow detector for detecting the flow rate of the output gas-phase fluid.

The nebulized medication delivery system further includes a protection device, which includes a limit position detector, a temperature detector, and a cooling fan. The parts of the protection device prevent the first perfusion device and the second perfusion device from operating abnormally. The limit position detector is used to detect the limit position of the piston in the cylinder. The controlling device is used to receive signals from the limit position detector and control the drive mechanism accordingly to avoid damage to the device. The temperature detector is used to detect the working temperature of the air compressor. The cooling fan is used to adjust the working temperature of the air compressor. The controlling device is used to receive information from the temperature detector and control the cooling fan accordingly. The protection device further includes an alarm. If the second perfusion device uses the air compression bottle, the controlling device monitors the air pressure of the gas-phase fluid through the air pressure detector. If the air pressure is insufficient, the controlling device will give a corresponding alarm.

Referring to FIG. 8c, each detector may choose a corresponding implementation scheme, for example: the position detector is implemented through the position detection of a sliding potentiometer; the limit position detector is implemented through an infrared limit sensor; the thrust detector is implemented through a syringe thrust sensor; the air compressor is achieved through an air compression pump; the temperature detector is implemented through the temperature sensor of the air compression pump; the air pressure detector is implemented through the air pressure sensor. The controlling device includes a motherboard and a compressed air pump control motherboard, wherein the motherboard detects the data of the above-mentioned detectors and controls the working status of the stepper motor, compressed air pump, and cooling fan accordingly.

The controlling device may also be used to convert the detection results of the position detector, thrust detector, limit position detector and air pressure detector into state parameters of the fluid, such as velocity and flow rate of liquid-phase fluid and velocity and flow rate of gas-phase fluid, and output the state parameters directly or after processed through video, audio.

For example, the nebulized medication delivery system further includes a display device as shown in FIG. 8a, wherein the display device may also be provided with a touch function accordingly. The controlling device receives the touch signal from the display device and adjusts the working mode accordingly, for example, adjusting the flow rate of the liquid-phase fluid, the pressure of the gas-phase fluid. The display device may be used, for example, through a capacitive touch screen, for human-machine exchange, system operation status indication, display of current pressure of the gas-phase fluid, current flow rate of the liquid-phase fluid, and flow rate of delivered liquid-phase fluid.

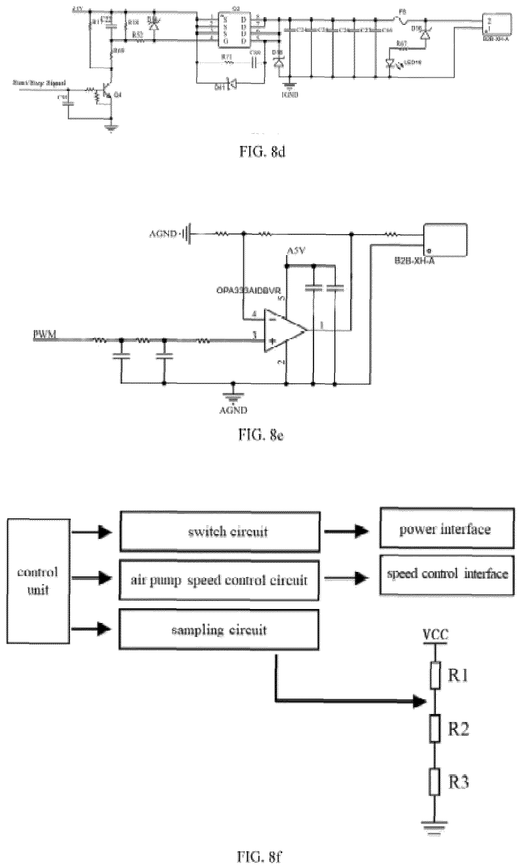

Referring to FIG. 8d, the controlling device includes a control unit and a switch circuit controlled by the control unit, wherein the switch circuit includes a first switch and a second switch. The control unit is internally configured with a microprocessor. For example, the control unit may be a Microcontroller Unit (MCU) inside the mainboard. The high-level signal output by the control unit is the start signal, and the low-level signal output by the control unit is the stop signal, which plays the role of controlling large current with small current.

The first switch has a first driving electrode, a first input electrode, and a first output electrode, wherein the first driving electrode controls and regulates the conduction and cutoff of the first input electrode and the first output electrode. The second switch has a second driving electrode, a second input electrode, and a second output electrode, wherein the second driving electrode controls and regulates the conduction and cutoff of the second input electrode and the second output electrode.

Among them, the first input electrode is electrically connected to an external power supply, which may be, for example, a processed 24V power supply. The first output electrode is electrically connected to a corresponding perfusion device (such as an air compressor) or a cooling fan, which is used to control the start and stop of the air compressor or the cooling fan. The first drive pole is electrically connected to the control unit, which is used to receive the start and stop signal of the control unit. The first driving electrode may be electrically connected to the control unit directly or indirectly, for example, electrically connected to the control unit through the second switch. The second input electrode is electrically connected to an external power supply via a bias resistor R17. The second output electrode is grounded. The second driving electrode is electrically connected to the control unit.

Specifically, the second switch may be, for example, a triode Q4, and the first switch may be, for example, a field effect transistor Q2. The second driving electrode is the base of the triode Q4, the second input electrode is the collector of the triode Q4, and the second output electrode is the emitter of the triode Q4. The first driving electrode is the gate (G) of the field effect transistor Q2, the first input electrode is the source (S) of the field effect transistor Q2, and the first output electrode is the drain (D) of the field effect transistor Q2. After the second driving electrode receives the start signal, the second input electrode and the second output electrode are conducted, the voltage of the second input electrode drops, triggering the first driving electrode to make the first input electrode and the second output electrode conduct, thereby achieving external power supply to the rear circuit.

The external power supply may be, for example, 24V DC power obtained through processing, and the drain (D) is connected to the corresponding air compressor or cooling fan through a board connector (B2B-XH-A), which has an input terminal (pin 2) and an output terminal (pin 1). A resettable fuse F5 is electrically connected between the drain (D) and the perfusion device, specifically between the drain (D) and the input terminal of the board connector to avoid the risk of high current caused by overload or short circuit, and the output terminal (pin 1) is grounded.

The air compressor or the cooling fan may be independently configured with a switch circuit, and the control unit outputs a start/stop signal to the corresponding switch circuit. The field effect transistor Q2 may be, for example, a P-type MOS transistor of with model SM4405PPL. After the field effect transistor Q2 is turned on, the external power supply supplies power to the rear circuit.

The triode Q4 may be, for example, a transistor with model SS8050, which works in a saturation or cut-off state in the circuit and is used to receive high and low level signals from the control unit. After the base of the triode Q4 receives the start signal, the MOS transistor is turned on.

The switch circuit further includes an electric energy storage, a first current limiting element, a second current limiting element, and a third current limiting element, wherein a first end of the electric energy storage is electrically connected between the external power source and a first input electrode (source), and the first current limiting element is connected in parallel with the electric energy storage to prevent false triggering due to charge accumulation. One end of the second current limiting element is electrically connected to a second end of the first current limiting element, and another end of the second current limiting element is electrically connected to the first driving electrode (gate). One end of the third current limiting element is electrically connected to a second end of the electric energy storage, and another end of the third current limiting element is electrically connected to the second input electrode (collector).

Specifically, the electric energy storage is the first capacitor C22, and the first capacitor C22 is used as a soft-start capacitor. The current passing through the field effect transistor Q2 increases linearly and slowly to prevent transient large current from impacting the load. The first current limiting element is a first resistor R18. The first resistor R18 divides the voltage so that the field effect tube Q2 operates within 12V. The second current limiting element is a second resistor R52, and the third current limiting element is a third resistor R69. A third voltage zener diode D14 is electrically connected between the source and the gate. The third voltage zener diode D14 is a clamping diode. The third voltage zener diode D14 limits the voltage between the source and the gate to within 15V, so as to protect the field effect transistor Q2 from being broken down.

Furthermore, a second capacitor C91 is provided between the base and the emitter of the triode Q4. The second capacitor C91 filters the interfering instantaneous peak signal to prevent the triode Q4 from being triggered incorrectly. A first voltage zener diode D41, a fourth resistor R71 and a third capacitor C89 which are connected in series and in parallel with the first voltage zener diode D41 are provided between the source and the drain of the field effect transistor Q2. The first voltage zener diode D41 is used for clamping and the RC buffer circuit is used for protection to prevent overvoltage between the drain and the source. A second voltage zener diode D18 is electrically connected between the drain of the field effect transistor Q2 and the ground, and a capacitor group connected in parallel with the second voltage zener diode D18 is also electrically connected between the drain of the field effect transistor Q2 and the ground. The second voltage zener diode D18 discharges the reverse electromotive force generated when the load stops. The capacitor group includes capacitor C24, capacitor C25, capacitor C26, capacitor C27 and capacitor C66. A reminder device is also electrically connected between the drain of the field effect transistor Q2 and the ground to remind the working condition of the electronic devices controlled by the switch circuit. The reminder device may be, for example, a buzzer or an indicator light LED 18. Specifically, a fourth voltage zener diode D16, a fifth resistor R67, and a reminder device are sequentially connected in series between the drain and the ground.

Referring to FIG. 8c to FIG. 8f, the control unit is electrically connected to the power interface on a control mainboard of the compressed air pump through the switch circuit shown in FIG. 8d, and is electrically connected to an adjustment interface on the control mainboard of the compressed air pump through the air pump speed control circuit shown in FIG. 8e. The switch circuit controls the start and stop of the air compression pump, and adjusts the speed of the air compression pump through the air pump speed control circuit (DAC).

As shown in FIG. 8e, the air pump speed control circuit is controlled by the control unit. The air pump speed control circuit includes a D/A module and an operational amplifier module coupled in sequence. After the air pump speed control circuit receives the PWM signal output by the control unit, the D/A module generates an analog voltage with a voltage range of 0~3.3V. The operational amplifier module converts this voltage to 0~5V. The analog signal of 0~5 V is connected to the mainboard of the compressed air pump shown in FIG. 8c through the B2B-XH-A board connector, thereby being electrically connected to the air compression pump to adjust the speed of the air compression pump and the pressure of the gas-phase fluid. The operational amplifier circuit may be specifically implemented by an integrated operational amplifier OPA333AIDBVR and its peripheral devices, wherein the operational amplifier includes five pins 1 to 5, of which pin 5 is the power supply end. The specific connection method will not be repeated here.

The control unit, switch circuit, or other peripheral circuits may be integrated into one circuit board or distributed on multiple circuit boards. In order to adapt to the upgrade of hardware circuits or software, the circuit board(s) may have multiple versions, wherein different identification marks may be set on different versions of the circuit board(s), respectively. The identification marks may be a simple physical structure or realized through circuits. Furthermore, other mechanical elements or consumables may also be provided with identification marks.

To simplify the identification marks, in one embodiment, a group of circuit elements may be provided, and signals that can be detected by the group of circuit elements may be used as the identification marks. In one embodiment, a replaceable component in the interventional device is provided with a circuit element as an identification mark, wherein the circuit element includes a plurality of resistors cooperatively forming a circuit topology structure, and at least one position in the circuit topology structure serves as a sampling point. Different elements are distinguished based on the electrical signals measured at the sampling points.

**In** order to match the identification marks and generate corresponding electrical signals, at least one position in the circuit topology structure is connected to the power supply, and the sampling point is connected to the judgment circuit through the sampling circuit. The power supply may be uniformly supplied by the main power supply of the interventional device or configured independently. Taking the main board of the control unit as an example, the power supply end of the main board may be used to power the circuit topology structure, or a battery may be configured separately. The judgment circuit may be implemented by the control unit itself or additionally configured with a device with signal processing capability.

For example, 2~5 resistors are connected in series to form the circuit topology structure, and power is supplied at one end. Both ends of each resistor may be used as sampling points, and the resistance values of the resistors may be the same as or different from each other, as long as the electrical signals at different sampling points are easy to distinguish and identify. Furthermore, the external resistance value of the circuit topology structure remains unchanged, and the circuit board has different versions. In each version, the number and/or resistances values of the resistors in the circuit topology structure are not exactly the same. In different versions, the sampling results of the sampling points in the circuit topology structure are different, without affecting the circuit elements in the controlling device other than the circuit topology structure.

Taking circuit boards of version A and version B as examples, sampling points may be set at different positions. When the power supply voltage is the same, the voltage values at different sampling points are different, so as to distinguish different versions of circuit boards and determine whether the circuit boards are installed correctly. As shown in FIG. 8f, the circuit topology structure is electrically connected between the voltage division VCC and the ground, and includes a resistor R1, a resistor R2, and a resistor R3 which are sequentially connected in series to the ground. Among them, the resistors R1 and R2 are configured in a certain ratio, and the voltage division VCC enables the sampling circuit to obtain different voltages. Different voltages detected by the port of the control unit represent different versions. For example, the detection result of 1V is used as a mark for version 1.0; and the detection result of 1.1V is used as a mark for version 1.1. The voltage division VCC may be, for example, 3.3V. The sampling circuit may be implemented, for example, by an ADC sampling circuit.

The control unit may perform machine self-check and corresponding processing according to the sampling result of the sampling circuit. When the control unit is compatible with at least two versions, the control unit automatically selects the control logic. In the case where the control unit can only be used with a single version, an alarm will be given if the versions do not match. It should be understood that the control logic and various control parameters under different versions are not exactly the same.

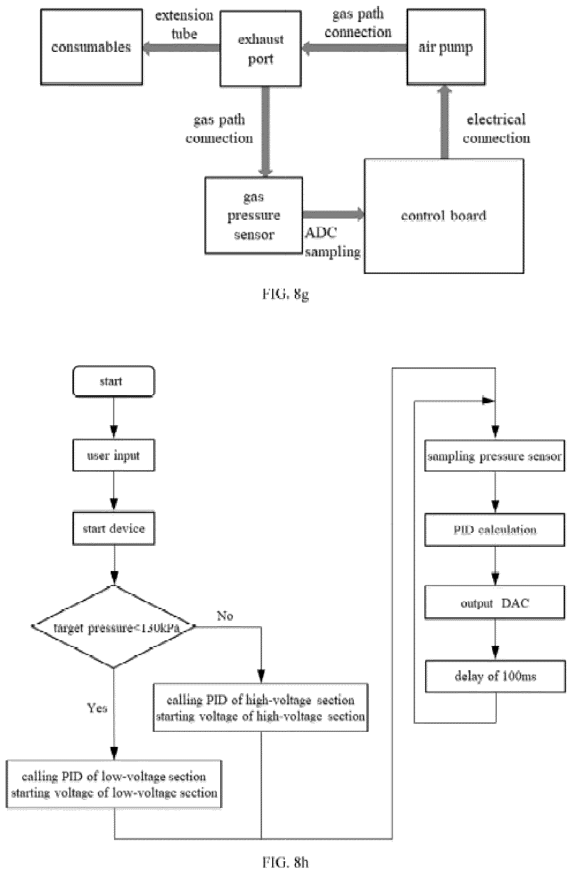

Referring to FIG. 8g and FIG. 8h, the interventional device outputs liquid-phase fluid and gas-phase fluid simultaneously, which are mixed in a certain ratio to output small droplets with a diameter of tens of micrometers. During mixing, it is necessary to control the output air pressure to remain stable. However, the air pump itself, the air path, and the load may all fluctuate, leading to unstable air pressure. Specific reasons may include motor overload and etc., which has a particularly significant impact when the air pressure output range is wide. The output air pressure generally range is 50Kpa ~ 200Kpa, and specifically for example may be 70Kpa ~ 200Kpa. The control unit controls the speed of the air pump to adjust the air pressure. The air pressure sensor collects the air pressure at an air outlet end, calculates it with the target air pressure value, and controls the output of the air pump.

As shown in FIG. 8g, the control unit is arranged in the control board, and obtains the sampling result of the air pressure sensor through the ADC sampling circuit. The air pressure sensor is arranged at the air outlet end of the air pump, wherein the air pump and the air outlet end are connected through an air path. The air pressure sensor obtains the air pressure detection result through the air path connection. The air outlet end is connected to the consumables through an extension tube air path. The air pressure sensor should be close to the consumables to obtain a more accurate air pressure value. Consumables may be understood as nebulization chips or interventional catheters.

In one embodiment, an interventional system control method is disclosed, including:
S1, preset a target air pressure and at least two groups of PID parameters, and associate each target air pressure with a corresponding PID parameter;
S2, the perfusion device first provides gas-phase fluid to the interventional catheter;
S3, the sampling device collects a first real-time air pressure value, compares the first real-time air pressure value with the target air pressure, and the controlling device calls the corresponding PID parameter and uses this PID parameter to adjust the pressure of the gas-phase fluid value of the perfusion device;
S4, the perfusion device provides liquid-phase fluid to the interventional catheter, so that the interventional catheter outputs nebulized fluid;
S5, the sampling device collects a second real-time air pressure value, compares the second real-time air pressure value with the target air pressure, and the controlling device calls the corresponding PID parameter and uses this PID parameter to adjust the pressure of the gas-phase fluid value of the perfusion device;
S6, repeat step S5 at preset time intervals.

By means of controlling the working process of the interventional system with at least two groups of PID parameters, the working stability can be effectively improved. For example, the target air pressure is the target air pressure value, and the controlling device determines the magnitude of the second real-time air pressure value and the target air pressure value to select the PID parameter that needs to be called; the target air pressure is the target air pressure range value, and the controlling device determines the target air pressure range value in which the second real-time air pressure value is located to select the PID parameter that needs to be called. In detail, using corresponding PID parameters to adjust the pressure of the gas-phase fluid of the perfusion device can prevent the liquid-phase fluid from entering the gas path of the interventional catheter, thereby improving safety.

Furthermore, the first real-time air pressure value is less than the second real-time air pressure value. Such setting prevents the liquid path in the interventional catheter from being cut off due to excessive air pressure. At least two voltage values are preset, and each group of PID parameters is associated with a corresponding voltage value. When the controlling device calls the corresponding PID parameter, the controlling device adjusts the starting voltage value of the perfusion device according to the voltage value corresponding to the PID parameter. Such setting can shorten the startup time of the air pump.

**In** other embodiments, the interventional system control method further includes:

S7, the perfusion device first stops providing liquid-phase fluid to the interventional catheter, and then stops providing gas-phase fluid to the interventional catheter. This step is mainly used to clean the liquid-phase fluid in the interventional catheter.

This embodiment provides a method for regulating the pressure of the gas-phase fluid in a segmented manner: the regulation target of the pressure of the gas-phase fluid is divided into at least two segments, and each segment is regulated using an independent PID control parameter. The control parameters may include, for example, the starting voltage value.

**In** this embodiment, the demarcation threshold of each segment is pre-set in the control unit, which may be, for example, 120Kpa or 130Kpa. By dividing the PID control parameters into multiple segments, stable and rapid air pressure regulation with small overshoot may be achieved in each segment, making the nebulization effect stable with less fluctuation and more precise control. For example, the target pressure of the gas-phase fluid is divided into a low pressure segment of 50KPa to 130KPa and a high pressure segment of 130KPa to 200KPa. The range of each PID control adjustment is usually between 20 and 80KPa. Based on the segmented PID control strategy (i.e., the above-mentioned method for adjusting the pressure of the gas-phase fluid), the interventional catheter needs to deliver the liquid-phase fluid first and then the gas-phase fluid to facilitate accurate sampling by the air pressure sensor.

Referring to FIG. 8h, the control process of the interventional device is described in detail. The interventional device includes a display device which may also be provided with a touch function accordingly, for human-computer interaction with a control unit arranged in the control panel. During use, the user inputs the target air pressure range, for example, 70Kpa to 200Kpa, through the display device, and starts the interventional device through the control unit.

The control unit calls corresponding control parameters according to a preset demarcation threshold. If the pressure is greater than or equal to the demarcation threshold, the control unit calls the PID control parameters of high-voltage segment, for example, calling high-voltage segment starting voltage value. If the pressure is less than the demarcation threshold, the control unit calls the PID control parameters of low-voltage segment, such as calling the low-voltage segment starting voltage value. The sampling air pressure sensor samples regularly, performs PID calculation based on the corresponding segmented PID control parameters, and adjusts the output of the air pump through the air pump speed control circuit (output DAC). The sampling is performed periodically, for example, once every 100ms. During the entire control process, a cyclic control process of "regular sampling of air pressure sensor - PID calculation - adjustment of air pump output" is carried out.

The control method of the present application also achieves safety control through multi-dimensional sensors. The interventional system includes a syringe, a position detector, an encoder and a motor. The encoder is arranged on the motor. The encoder records the stroke of the piston through the motor. The position detector is used to check the stroke of the piston to ensure that the piston is working normally. The interventional system further includes a limit position detector, which is used to limit the limit stroke of the piston.

Therefore, from another aspect, the present application further discloses an interventional system control method, including: presetting liquid medium delivery parameters, generating a liquid medium delivery map according to the preset liquid medium delivery parameters; generating a corresponding drive signal according to the liquid medium delivery map for driving a motor, wherein the motor is used to drive a piston of the syringe to realize liquid path delivery; collecting the signal of an encoder set on the motor to obtain the operating position of the motor and calculating the position of the piston through the operating position of the motor; recording the position of the piston and calculating the actual delivery map based on the position information of several pistons; comparing the liquid medium delivery map and the actual delivery map according to a preset period, if the difference is less than a preset value, retaining the original liquid medium delivery map; if the difference is greater than or equal to the preset value, generating a new liquid medium delivery map according to the preset rules to replace the original liquid medium delivery map; keeping updating the liquid medium delivery map until the liquid medium delivery is ended or paused. Similarly, the closed-loop control and dynamic adjustment of the liquid medium delivery may also be combined with the closed-loop control of the gas medium delivery, such as the comparison of air pressure values and the control method with at least two groups of PID mentioned above.

In one embodiment, a medication administration method for treating natural cavities of the human body is provided, wherein the interventional catheter in the above embodiments is inserted into the lesion site in the bronchus, and a fluid containing a therapeutic substance is nebulized through the interventional catheter and output to the lesion site. The lesion locations of the natural cavities of the human body include the bronchi, lungs, intestines, kidneys, bladder and blood vessels. Taking lung diseases as an example, lung diseases include sputum accumulation, tuberculosis, fungal infections and tumors. The therapeutic substance may be at least one of expectorant medications, anti-tuberculosis medications, anti-fungal infection medications, and anti-tumor medications. In specific applications, for example, it can be used for ICU patients to inhale aerosolized phlegm-removing medications, for tuberculosis patients to inhale aerosolized tuberculosis medications, for inhaled aerosolized medications to treat fungal infections, or for inhaled aerosolized medications to treat tumors, etc.

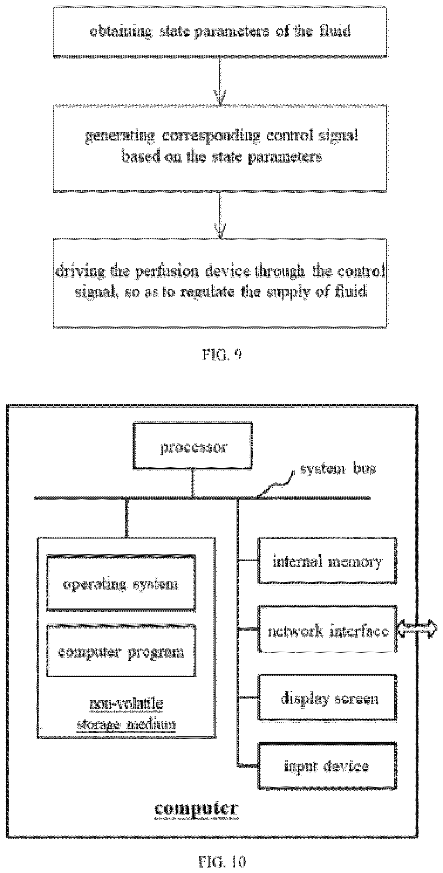

Referring to FIG. 9, the present application further provides a control method for a nebulized medication delivery system, wherein the nebulized medication delivery system is used to nebulize a fluid containing a therapeutic substance and output the fluid into the bronchus, wherein the control method includes:
obtaining state parameters of the fluid;
generating corresponding control signal according to the state parameters;
driving the perfusion device by the control signal to adjust the supply amount of the fluid.

In one embodiment, the nebulized medication delivery system further includes an endoscope, and the interventional catheter extends along a flow channel of the endoscope. When the nebulized medication delivery system is used, the fluid in the interventional catheter is filled until it reaches the distal end, and the flow mixing structure follows the endoscope and is delivered to the lesion. When the system is started, the gas-phase fluid is circulated first and then the liquid-phase fluid. When the system is shut down, the liquid-phase fluid is stopped first and then the gas-phase fluid, so as to avoid residual fluid in the interventional catheter as much as possible.

The fluid includes micron-sized liquid particles, and the state parameters of the fluid include state parameters of the micron-sized liquid particles;

The control signals is generated corresponding to the state parameters of micron-sized liquid particles, and the gas pressure and liquid flow rate of the perfusion device input into the interventional catheter are controlled by the control signals;

The sampling device collects the current gas pressure information and liquid flow rate information, and feeds back to the controlling device;

The controlling device determines whether it is necessary to adjust the gas pressure and/or liquid flow rate input into the interventional catheter by the perfusion device based on the current gas pressure information and liquid flow rate information.

It should be understood that, although the steps in the flowchart of FIG. 9 are displayed sequentially as indicated by the arrows, these steps are not necessarily executed sequentially in the order indicated by the arrows. Unless otherwise specified herein, there is no strict order limitation for the execution of these steps, and these steps may be executed in other orders. Moreover, at least part of the steps in FIG. 9 may include multiple sub-steps or multiple stages. These sub-steps or stages are not necessarily executed at the same time, but can be executed at different times. The execution order of these sub-steps or stages is not necessarily sequentially, but can be executed in turn or alternately with other steps or at least part of the sub-steps or stages of other steps.

**In** one embodiment, a computer is provided. The computer may be a terminal, and its internal structure may be as shown in FIG. 10. The computer includes a processor, a memory, a network interface, a display screen and an input device connected via a system bus. Among them, the processor of the computer is used to provide computing and control capabilities. The memory of the computer includes a non-volatile storage medium and an internal memory, wherein the non-volatile memory may include read-only memory (ROM), magnetic tape, floppy disk, flash memory, optical storage, high-density embedded non-volatile memory, resistive random access memory (ReRAM), magnetoresistive random access memory (MRAM), ferroelectric random access memory (FRAM), phase change memory (PCM), graphene memory, etc. The non-volatile storage medium stores an operating system and a computer program. The internal memory provides an environment for the operation of the operating system and computer programs in the non-volatile storage medium. The network interface of the computer is used to communicate with an external terminal via a network connection. When the computer program is executed by a processor, a control method for nebulized medication delivery system is implemented. The display screen of the computer may be a liquid crystal display screen or an electronic ink display screen, and the input device of the computer may be a touch layer covering the display screen, or a key, trackball or touchpad provided on the computer housing, or an external keyboard, touchpad or mouse, etc.

Similarly, the present application further discloses a program product and a corresponding storage medium. The program product includes one or more computer instructions, which, when executed by a computer, enable the computer to implement a control method for nebulized medication delivery system. When the computer program instructions are loaded and executed on the computer, the process described in the embodiment of the present application is generated in whole or in part. The computer may be a general purpose computer, a special purpose computer, a computer network, or other programmable device.

The computer can be configured in the nebulized medication delivery system, used to nebulize a fluid containing a therapeutic substance and output it into the bronchus. The computer includes a memory and a processor, wherein the memory stores a computer program, and the processor implements the following steps when executing the computer program:
obtaining state parameters of the fluid;
generating a corresponding control signal according to the state parameters;
driving the perfusion device to adjust the supply amount of the fluid by the control signal.

When the computer program is executed, the specific limitations of the implementation steps can be found in the above-mentioned limitations on the control method of the nebulized medication delivery system, which will not be repeated here.

Those skilled in the art will understand that the structure shown in FIG. 10 is merely a block diagram of a partial structure related to the solution of the present application, and does not constitute a limitation on the computer to which the solution of the present application is applied. The specific computer may include more or fewer components than shown in the figure, or combine certain components, or have a different arrangement of components.

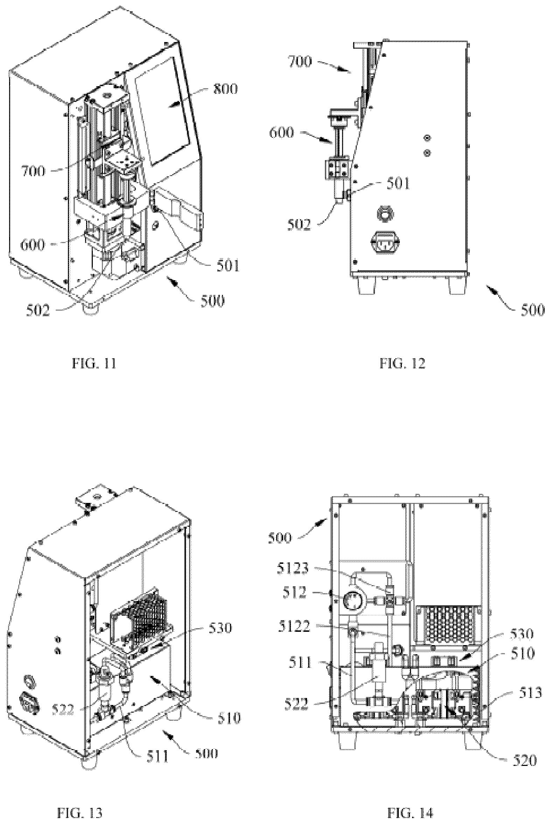

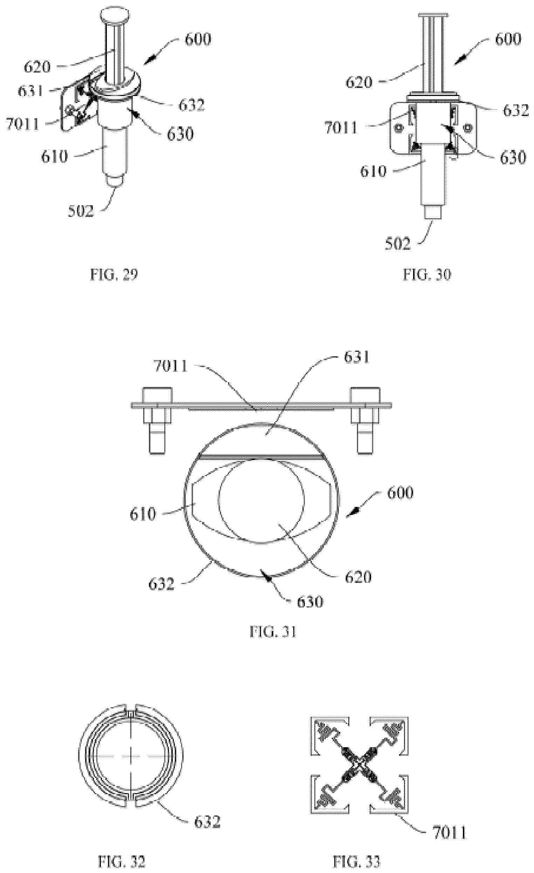

Based on the above description, with reference to the embodiments shown in FIG. 11 to FIG. 37, the present application further discloses a gas-liquid interventional device, wherein the interventional device includes:
a housing 500, on which a gas path connector 501 and a liquid path connector 502 are mounted, a pump chamber 510 being provided at a bottom of the housing 500;
an air pump 520 installed in the pump chamber 510, wherein the air pump 520 includes multiple pumps connected in parallel and is connected to the air path connector 501 through a main pipe 511;
a cooling assembly 530, thermally coupled to the air pump 520;
a syringe 600 including a cylinder 610 installed in the housing 500 and a piston 620 slidably installed in the cylinder 610, wherein the cylinder 610 has an outlet communicating with the liquid path connector 502;
a drive mechanism 700 linked to the piston 620 of the syringe 600.

The gas-liquid interventional device in the present application can be divided into a gas path part and a liquid path part, both of which enter the interventional catheter via the gas path connector 501 and the liquid path connector 502, respectively. In order to better understand the technical solution of the present application, the two parts will be explained separately below.

First, the structure of the gas path part will be described, referring to the interventional device shown in FIG. 11 to FIG.23, the gas path part includes:
a housing 500 with a gas path connector 501 installed thereon, a pump chamber 510 being provided at a bottom of the housing 500;
an air pump 520 installed in the pump chamber 510, wherein the air pump 520 includes multiple pumps connected in parallel and is connected to the air path connector 501 through a main pipe 511;
a pressure relief valve 512 installed on the main pipe 511, an outlet of the pressure relief valve 512 being connected to the pump chamber 510 through a pipeline;
a cooling fan 531 being thermally coupled to the air pump 520.

In this embodiment, the air source is provided by the air pump 520, thereby realizing the function of the air path part. In actual use, the inventors found that medical air pressure device needs to meet the oil-free requirement, which leads to dry grinding of the pump body in the air pump 520, so the heat generating is relatively serious, which affects the lifespan. At the same time, in order to reduce noise, a shielding cover is generally used, but this manner will result in the inability to form an effective air duct, or the air outlet is small, causing temperature rise. At the same time, when meeting certain special high pressure requirements of consumable instruments, the temperature rise of the air pump 520 is too large and the load is too large; the combination of the two cause excessive heating after long-term operation, affecting the outlet pressure of the air pump 520 or greatly affecting the life of the instrument. In order to overcome the above problems, the present application performs heat dissipation optimization. Referring to the embodiment shown in the drawings, a cooling fan 531 is arranged outside the pump chamber 510, a cooling window 532 is opened on a side wall of the pump chamber 510 for the cooling airflow of the cooling fan 531 to pass through, and a heat exchanger 533 thermally coupled to the air pump 520 is provided inside the pump chamber 510, wherein at least a portion of the heat exchanger 533 is aligned with the cooling window 532. In the drawings, there are multiple heat exchangers 533 arranged in groups and each group of heat exchangers 533 has at least two pieces with an air flow slit 534 formed therebetwee, wherein the air flow slit 534 is aligned with the cooling window 532 and the cooling fan 531. Optionally, the heat exchangers 533 in the same group correspond to different air pumps 520 or different pump heads of a same air pump 520. In the drawings, the air pump 520 is configured with two pump heads. Two heat exchangers 533 correspond to different pump heads, respectively, which can improve the heat exchange efficiency. The heat exchanger 533 may be made of a material with high thermal conductivity, or the heat exchange efficiency can be improved by increasing the surface area. Alternatively, referring to the embodiment shown in the drawings, the heat exchanger 533 is a thermoelectric cooler with a cold end thermally coupled to the pump head of the air pump 520 and a hot end exposed to the cooling window 532. The cold end is attached to the heat sink of the air pump 520, and the hot end may be in form of heat sink or heat dissipation to the air directly, and the heat of the thermoelectric cooler is carried away by blowing/sucking air. Closed-loop control of the heat dissipation may also be achieved by setting a temperature sensor. For example, a temperature sensor is installed on the air pump 520. When the temperature is too high, the working power of the thermoelectric cooler increases, that is, the current of the thermoelectric cooler is increased, and vice versa. Thus, a closed loop control is formed to control the temperature of the air pump 520 to be within a temperature range of efficient operation. If the temperature cannot drop and exceeds the preset value, an alarm will be given or the system will be instructed to stop the pump.

The flowing direction of the airflow of the cooling fan 531 needs to be adjusted according to the material of the heat exchangers 533.

For example, when the heat exchanger 533 is made of a material with a high thermal conductivity, the cooling fan 531 may be configured in a blowing manner to deliver cold air to the interior of the pump chamber 510, and after heat exchange with the heat exchanger 533 and/or cooling fins of the pump head of the air pump 520, it is discharged through an exhaust port 503 at the bottom of the pump chamber 510.

For another example, when the heat exchanger 533 is a thermoelectric cooler, if the cooling fan 531 continues to choose the blowing method, the heat from the hot end of the thermoelectric cooler may be delivered to the cold end, resulting in low heat exchange efficiency. At this time, the cooling fan 531 may be configured in a suction manner to extract the hot air from the pump chamber 510. The exhaust port 503 at the bottom of the pump chamber 510 acts as an air inlet. The cooling air enters the pump chamber 510 through the action of the negative pressure inside the pump chamber 510 (generated by the suction of the cooling fan 531), and is discharged through the cooling fan 531 after heat exchange with the heat exchanger 533 and/or cooling fins of the pump head of the air pump 520.

In addition to using a cooling fan, the establishment of an air duct may also refer to the following embodiments, which use a pressure relief duct. In the field of medical device, the amount of gas used is generally small, but in order to maintain a high pressure, the air pump 520 needs to be kept running, which is inconvenience. In this embodiment, the output air flow of the air pump 520 enters the pressure relief valve 512 via the main pipe 511, and the pressure relief valve 512 is at least connected to a pressure relief air duct 5122 which communicates with an output pipeline 5121 of the air path connector 501 and the pump chamber 510. By means of the pressure relief valve 512, excess gas is discharged through exhaust gas bypass, thereby utilizing the exhaust gas to dissipate heat from the air pump 520, which is without power supply, improving the miniaturization effect of the device. The airflow of the pressure relief air duct 5122 enters the pump chamber 510 to form a positive pressure, and the hot air inside the pump chamber 510 is discharged through the exhaust port 503 at the bottom of the device and/or the cooling fan 531 to take away the heat. In this embodiment, the cooling fan 531 may choose to blow air into the pump chamber 510 to assist in forming a positive pressure environment, or may choose to suck air from the pump chamber 510 to assist in gas outflow. The specific selection can be set according to the operating power of the cooling fan 531 and the air delivery volume of the pressure relief air duct 5122.

In this embodiment, a pressure relief air duct 5122 is used to form a positive pressure, and a cooling fan 531 is used to suck air. The main consideration of this manner is that, the medical pump used in this field is very small in size, and an outside of the pump is treated with sound insulation cotton and the like, resulting in a smaller space inside the isolation cover of the air pump 520 and a narrower air duct. Using an air duct to form an air channel with a small cross-sectional area and a fast flow rate under the axial flow fan meets the actual use requirements and the heat dissipation requirements. In addition, the magnitude of the wind is related to the working power of the pump, that is, related to the heat generated by the pump, which will also produce a certain linear relationship. In actual control, the pressure can be detected through the air pressure transmitter 522. The air pump 520 can be detected to determine whether the output pressure of the air pump 520 meets the standard, and the speed of the motor 702 is adjusted according to the pressure signal to perform closed-loop control of the pressure. The gas flows through the pressure relief valve 512, and the excess gas is discharged after silencing treatment at the outlet. A corresponding hose is connected here, and the pressure relief air duct 5122 is converged through a four-way joint 5123 (it can also be diverted to multiple places according to the matching degree of the air pump 520 and consumables) and connected to the pump chamber 510.

The pressure relief valve 512 can be controlled by elastic parts in its specific structure. For example, a spring can be used to press the valve. When the pressure is too high, the valve can be pushed open and the pressure can be released to achieve automatic over-control. Precise control can also be achieved through an electronically controlled valve.

**In** addition to the enhanced heat dissipation, in this embodiment, multiple air pumps 520 are connected in series to improve working efficiency and stability. There are many combinations in actual work. For example:
Two pumps work at low pressure at the same time to provide a high-pressure output state: Since the parameters of the consumables (interventional catheter) in the front section are variable, it is sometimes necessary to increase the upper limit pressure of the air pump 520, that is, close to 0.2mPa at full load. The dual pumps are connected in parallel and each provides a certain low flow rate, which are aggregated to the main pipe 511 by the three-way one-way valve at the rear end, so as to meet the working pressure requirements and greatly reduce the utilization coefficient of each unit;
The two pumps work separately to extend the service life: when the working pressure is low, one air pump is used for one operation, which will not cause the impact of continuous temperature increase on the device. In addition, if one pump is damaged, the other can be used as a backup to prevent downtime and alarm prompts.

The control of the air pump 520 can be achieved through the detection of the air pressure transmitter 522, which controls the speed of the brushless motor 702 of each air pump 520 to control the pressure generated by the movement of the pump body within a required temperature range, thereby extending the service life.

In this embodiment, the stability of the operation of the gas path is effectively improved through the optimized heat dissipation settings and the optimization of the control logic of the online operation of multiple air pumps 520.

In addition to the operational stability of the air pump 520 mentioned above, the noise and vibration of the air pump 520 also present problems in actual use. Referring to the embodiment shown in FIG. 14, a soundproof partition 513 is provided on an inner sidewall of the pump chamber 510, attached to the sidewall of the pump chamber 510 or spaced apart from the sidewall of the pump chamber 510. The soundproof partition 513 can be made of sound-absorbing materials with high elasticity or complex surfaces (for example, environmentally friendly sponges, made of sound-insulating materials with multiple fibers and porous structures, such as rock wool, plant fiber spraying, etc., to absorb noise), or high-density sound-insulating materials, or high-damping shock-absorbing materials, or even composited into the sidewall of the pump chamber 510 (for example, the sidewall of the pump chamber 510 is treated with double layers and vacuumed, and a composite absorber composed of barium aluminum alloy and zirconium aluminum is added between the double layers to reduce the medium for sound transmission).

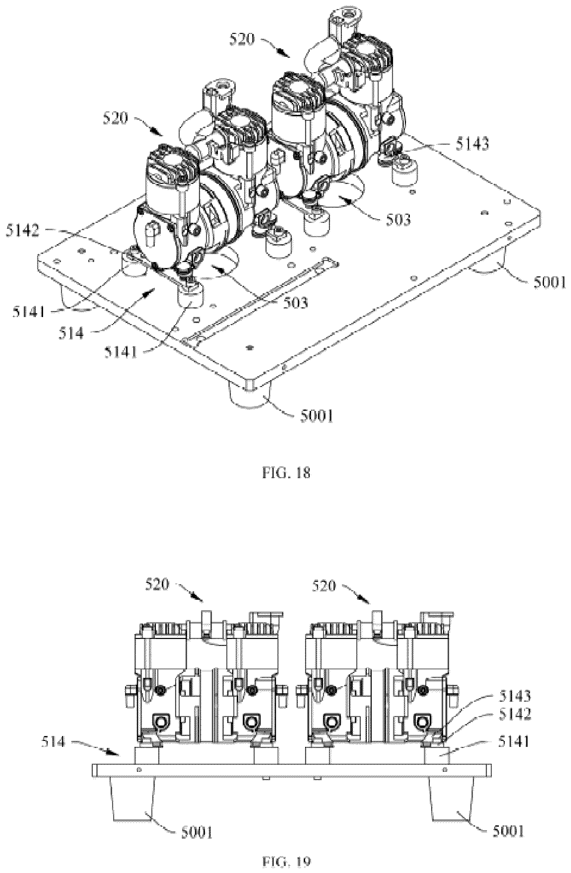

In addition, the above problems can be overcome by setting a damping device. Referring to the embodiment shown in FIG. 18 to FIG. 22, the air pump 520 is connected to the housing 500 via a two-stage damping device 514, wherein the two-stage damping device 514 includes:
a plurality of first buffer blocks 5141 arranged on the housing 500;
a primary damping platform 5142 supported by the first buffer blocks 5141;
a plurality of second buffer blocks 5143 disposed on the primary damping platform 5142 and connected to the air pump 520.

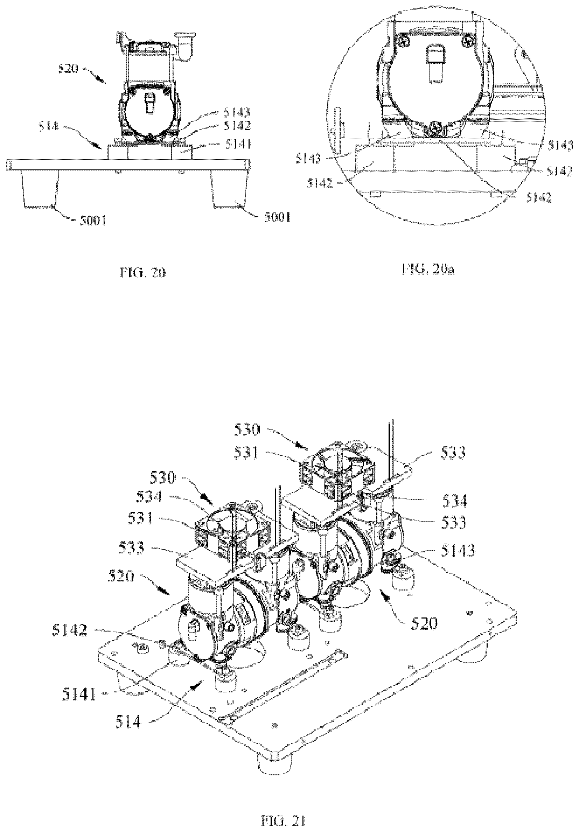

The second buffer block 5143 has a lower hardness than the first buffer block 5141, and can effectively block the vibration transmission between the air pump 520 and the housing 500. In terms of specific structure, referring to FIG. 20a, the second buffer block 5143 is a thin, cylindrical-shaped as a whole, and is provided with a first mounting groove 5144 and a second mounting groove 5145 for clamping the primary damping platform 5142 and the machine foot of the air pump 520 at its top and bottom. In terms of overall size, the diameter of an outer wall of the first buffer block 5141 is about 11mm, a buffer spacing between the first mounting groove 5144 and the second mounting groove 5145 is about 8mm, and a wall thickness of the cylindrical body between the first mounting groove 5144 and the second mounting groove 5145 is about 1mm. From the overall shape, the second buffer block 5143 is configured as an elongated cylinder, that is, an axial length thereof is slightly greater than a radial length, thereby providing a larger deformation space to absorb vibration effects.

In the drawings, the second buffer block 5143 may be tilted relative to the primary damping platform 5142. In principle, a line between the center of the upper surface of the second buffer block 5143 and the middle of the lower surface is the working line. When the second buffer block 5143 is tilted, the working line will form an angle with the direction of gravity. In the above setting, the angle can reach 20 degrees or even 30 degrees, thereby effectively releasing the vibration energy of the air pump 520.

However, excessive inclination of the second buffer block 5143 may reduce the installation stability of the air pump 520. Therefore, the above problem can be avoided by adjusting the structural parameters of the second buffer block 5143. For example, each second buffer block 5143 has directional characteristics, thereby ensuring the spatial stability of the air pump through force coupling in different directions. Ideally, the second buffer block 5143 should only receive its own axial load. In order to avoid unexpected situations and improve robustness, a buffer zone may be provided between the air pump 520 and surrounding components to avoid component interference caused by the tilt of the second buffer block 5143. Especially when two air pumps are arranged side by side, the distance between the air pumps should be ensured to avoid interference.

In actual products, the first buffer block 5141 can adopt a column-shaped rubber pad, the material of which can be NR, SBR and CR. Due to the viscoelastic properties of rubber, the first buffer block 5141 has good shock absorption, sound insulation and buffering properties. According to tests, it can effectively reduce 60% of the vibration transmitted to the housing 500. A thicker and denser rubber material is used to fix and support the primary damping platform 5142 and the second buffer block 5143. The second buffer block 5143 adopts a hollow rubber column-shaped structure, and the wall thickness is about 2mm when unloaded. In one embodiment, the hardness of the second buffer block 5143 is less than that of the first buffer block 5141, and the second buffer block 5143 has a higher degree of freedom of spatial movement than the first buffer block 5141. The specific material of the second buffer block 5143 may be softer rubber material, so as to increase flexibility. Two sides are fixed by inserting, with no restrictions on freedom, which can eliminate 80% of vibrations.

After actual tests, the shock absorbing effect of the two-stage damping device 514 in this embodiment can be reduced to 8% (40%*20%=8%) of the original situation, and the noise can also be mostly eliminated.

Furthermore, a third buffer block 5001 may be provided at the bottom of the housing 500 to further reduce noise and vibration.

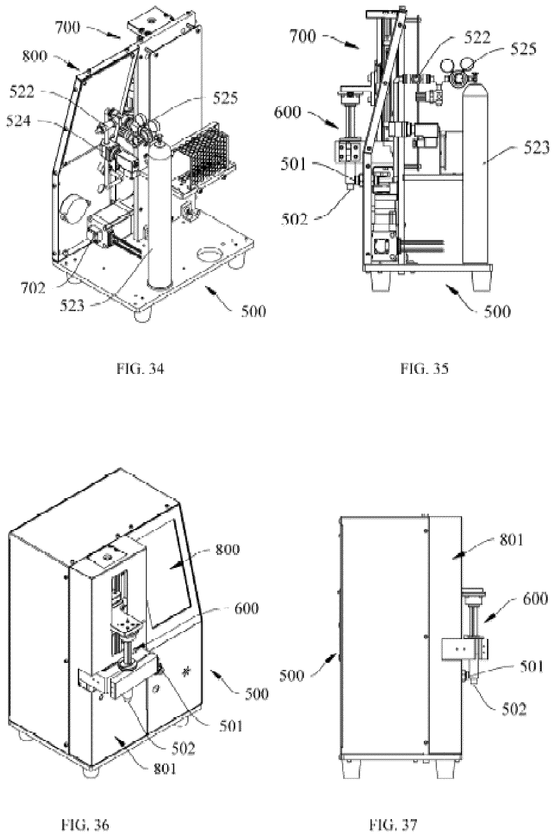

With reference to FIG. 34 and FIG. 35, it can be seen that in this embodiment, a gas source can also be provided by setting up a gas cylinder 523. Compared with the solution of the air pump 520, the gas source in this embodiment has less noise and lower energy consumption, and the controlling device can control the air pressure and airflow through a solenoid valve 524. The gas cylinder 523 can be optionally connected to a pressure regulating valve 525 to achieve pressure regulation. At the same time, the controlling device can detect the amount of gas in the gas cylinder 523, thereby reminding to replace the gas cylinder 523 when the gas content is insufficient. Other settings may refer to the nebulization device with an air pump described above, and will not be described in detail here.

The structural optimization of the air pump 520 and the pump chamber 510 is to provide a good working foundation for the operation of the gas path part. The function of the gas path part is to provide a stable and reliable gas source to the gas path part so that it can work in coordination with the liquid path part. In the embodiment shown in the drawings, a liquid path connector 502 and a fixing seat 701 are also installed on the housing 500; an adapter 630 is detachably connected to the fixing seat 701 for movably inserting a cylinder 610 of a syringe 600 therein, wherein the syringe 600 includes the cylinder 610 and a piston 620 slidably installed in the cylinder 610, the cylinder 610 has an outlet and is connected to the liquid path connector 502; the piston 620 of the syringe 600 is linked to a drive mechanism 700. The liquid-phase fluid output by the liquid path connector 502 and the gas-phase fluid output by the gas path connector 501 are mixed with each other to form a gas-liquid flow which is nebulized to achieve medication administration.

In combination with the above, it can be seen that the present application discloses an interventional device, including:
a housing 500, on which a gas path connector 501 and a liquid path connector 502 are mounted, a pump chamber 510 being provided at a bottom of the housing 500;
an air pump 520 installed in the pump chamber 510, the air pump 520 including multiple pumps connected in parallel and connected to the air path connector 501 through a main pipe 511;
a pressure relief valve 512 installed on the main pipe 511, and an outlet of the pressure relief valve 512 being connected to the pump chamber 510 through a pipeline;
a cooling fan 531, thermally coupled to the air pump 520;
a syringe 600 including a cylinder 610 installed in the housing 500 and a piston 620 slidably installed in the cylinder 610, wherein the cylinder 610 has an outlet and is connected to the liquid path connector 502;
a drive mechanism 700 linked to the piston 620 of the syringe 600.

Similarly, the present application discloses an interventional system, including:
a housing 500 with a gas path connector 501 provided thereon, a pump chamber 510 being provided at a bottom of the housing 500;
an air pump 520 installed in the pump chamber 510, the air pump 520 including multiple pumps connected in parallel and connected to the air path connector 501 through a main pipe 511;
a pressure relief valve 512 installed on the main pipe 511, an outlet of the pressure relief valve 512 being connected to the pump chamber 510 through a pipeline;
a cooling fan 531, thermally coupled to the air pump 520;
a syringe 600 including a cylinder 610 installed in the housing 500 and a piston 620 slidably installed in the cylinder 610, wherein the cylinder 610 has an outlet and is connected to the liquid path connector 502;
a drive mechanism 700, linked to the piston 620 of the syringe 600;
an interventional catheter comprising a catheter body, wherein one end of the catheter body is a proximal end, and another end of the catheter body is a distal end that can extend into the bronchus, the proximal end is connected to the gas path connector and the liquid path connector, an interior of the catheter body is configured with a flow channel that can transport fluid from the proximal end to the distal end, the distal end of the catheter body is provided with a flow mixing structure, a microfluidic chip is provided in the flow mixing structure, and the fluid in the flow channel is output after being nebulized through the microfluidic chip.

In the embodiment shown in the drawings, the gas-phase fluid and liquid-phase fluid are used to be delivered to an interventional catheter, which includes a catheter body with one end thereof being a proximal end and another end thereof being a distal end that can extend into the bronchus, an interior of the catheter body is provided with a flow channel that can deliver fluid from the proximal end to the distal end, the distal end of the catheter body is provided with a fluid mixing structure, a microfluidic chip is provided in the fluid mixing structure, and the fluid in the flow channel is output after being nebulized through the microfluidic chip;
a distribution structure capable of acting on at least a portion of the fluid is arranged inside the microfluidic chip.

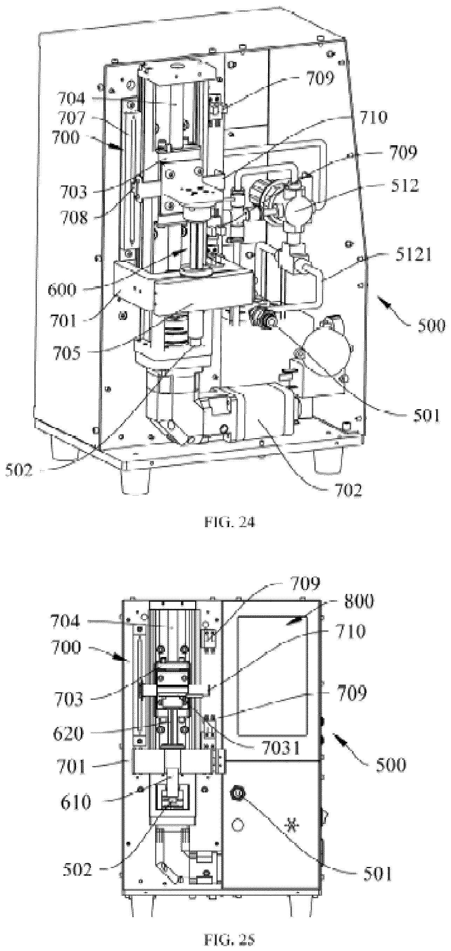

Next, the structure of the liquid path part will be described, referring to the interventional device disclosed in FIG. 24 to FIG.33, including:
a housing 500, on which a liquid path connector 502 is mounted;
a fixing base 701 fixed on the housing 500;
an adapter 630 detachably connected to the fixing base 701, and electronic identification components which cooperate with each other are provided between the adapter 630 and the fixing base 701;
a syringe 600 including a cylinder 610 being movably inserted into the adapter 630 and a piston 620 slidably mounted in the cylinder 610, wherein the cylinder 610 has an outlet and is connected to the liquid path connector 502;
a drive mechanism 700 to the piston 620 of the syringe 600.

In this embodiment, the movement of the piston 620 of the syringe 600 is achieved by the movement of the drive mechanism 700, thereby achieving the liquid medium output of the liquid path connector 502, realizing the function of the liquid path part. In terms of specific configuration, referring to the embodiment shown in the drawings, the drive mechanism 700 includes:
a motor 702;
a sliding seat 703 driven by the motor 702 to slide and installed in the housing 500, wherein the sliding seat 703 cooperates with the piston 620 of the syringe 600. The sliding seat 703 can improve its own movement accuracy, so as to accurately output a preset amount of liquid medium. In a specific embodiment, the motor 702 is linked with a screw rod 704, and the sliding seat 703 and the screw rod 704 are threadedly matched with each other. In order to further improve the accuracy of the output, the sliding seat 703 abuts against the piston 620 of the syringe 600, and a pressure sensor 7031 for detecting an interaction force is provided at the abutting portion.

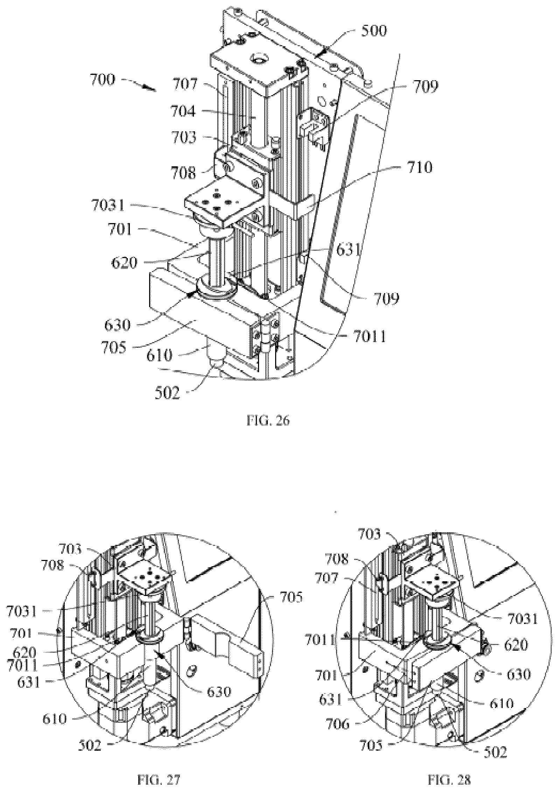

**In** addition to the pressure sensor 7031, the drive mechanism 700 can also be controlled by stroke detection. Referring to the embodiment shown in FIG. 26 to FIG. 28, a sliding position sensor 707 is provided on one of the sliding seat 703 and the housing 500, and a sliding trigger member 708 for adapting to the sliding position sensor 707 is fixed on the other one of the sliding seat 703 and the housing 500. The controlling device uses the sliding position sensor 707 to read the position information of the sliding seat 703. A limit position sensor 709 is provided on one of the sliding seat 703 and the housing 500, and a limit triggering member 710 for adapting to the limit position sensor 709 is fixed on the other one of the sliding seat 703 and the housing 500. The drive mechanism 700 is controlled by the limit position sensor 709. In the specific product, the sliding seat 703, the housing 500 and the screw rod 704 cooperatively constitute a linear module. The sliding position sensor 707 is configured in the form of a sliding potentiometer and is linked to the sliding seat 703. The stroke of the sliding potentiometer is greater than the maximum stroke adapted for the nebulized medication delivery device. Through the information of different sensors, the controlling device can accurately obtain the relative position/absolute position of the sliding seat 703, so as to calculate the delivery amount of the liquid medium. The specific control logic will be described in detail in the method part below.

It is not difficult to find that the driving effect of the drive mechanism 700 on the piston 620 depends on the stable installation of the cylinder 610. In terms of the specific installation method, referring to the embodiment shown in FIG. 26 to FIG.28, a hinged pressing plate 705 is provided on the fixing seat 701, wherein the pressing plate 705 has a working state which cooperates with the fixing seat 701 to enclose and clamp the adapter 630 and/or the syringe 600 (refer to FIG.28) and a corresponding release state (refer to FIG. 27). The syringe 600 can be easily assembled and disassembled through the movement of the pressing plate 705. A locking device 706 is provided between the fixing seat 701 and the pressing plate 705 for maintaining the pressing plate 705 in the working state.

In order to adapt to syringes 600 of different volumes, shapes and sizes, the syringe 600 cooperates with the fixing seat 701 and the pressing plate 705 through the adapter 630. Referring to the embodiment shown in the drawings, the adapter 630 is cylindrical-shaped and has a position limit portion 631 protruding radially and outwardly from one end thereof. The position limit portion 631 cooperates with the cylinder 610 of the syringe 600 to limit the movement of the cylinder 610 in the circumferential direction. In a specific embodiment, the position limit portion 631 is a bump protruding axially, and cooperates with a commonly used clamping portion of the syringe 600, which has high adaptability, and can adapt to syringes 600 of different sizes. In terms of specific shapes, referring to the embodiment shown in the drawings, the adapter 630 is a cylindrical-shaped structure, which is adapted to the shape of the opening in on the fixing seat 701. In a specific product, the adapter 630 may also be a multi-layer structure that is nested inside and outside or nested up and down.

In addition to adapting to the shapes and sizes of different syringes 600, the adapter 630 can also perform an identification function. Referring to the embodiment shown in FIG. 27 to FIG.33, an adapter identification label 632 is provided on the adapter 630, and an adapter identification circuit 7011 adapted to the adapter identification label 632 is provided on the fixing seat 701. The adapter identification circuit 7011 determines the parameters of the syringe 600 by reading the adapter identification label 632 or uploads a data packet containing the parameters of the syringe 600. Referring to the embodiment shown in the drawings, the adapter identification circuit 7011 is provided inside the fixing seat 701, and spaced from the adapter identification label 632, completing the identification in a non-contact manner. In a specific product, the adapter identification circuit 7011 is an antenna and an antenna PCB arranged on a side of the fixing base 701 facing away from the adapter 630, and the adapter identification label 632 is a ring circuit mounted around the adapter 630. During the assembly process of the adapter identification circuit 7011 and the adapter identification label 632, the adapter identification label 632 and the adapter 630 cooperate with each other in a manner of transition fit, thereby configured as a whole. When the adapter 630 is matched with the fixing seat 701, especially when the pressing plate 705 and the fixing seat 701 surround the adapter 630, elastic materials such as EVA or rubber plastic may be filled to ensure that the pressing plate 705 does not damage the adapter 630 after closing. The locking device maybe configured to use a mechanical structure or an electronically controlled structure to achieve locking, or it can be simply locked and closed by means of bolts.

In the above-mentioned identification process of the adapter identification circuit 7011 and the adapter identification label 632, there are several manners as follows:
1) If the device is not turned on, it will automatically detect the adapter identification label 632 at the beginning and adjust the parameters accordingly;
2) If the device is already turned on, the user can use the operation screen 800 directly to perform the action of identifying the adapter identification label 632;
3) If the parameters have been set, press the start button to perform an automatic check, and then automatically run subsequent operations after adjusting the corresponding parameters.

After the syringe 600 is identified, the parameters need to be adjusted, and the corresponding parameters are closed-loop fed back by the corresponding components;
1) the pressure of the liquid is detected by the pressure sensor 7031;
2) the speed is detected by the sliding potentiometer;
3) the pressure of the gas is detected by the pressure transmitter at the rear end and adjusted by the speed, and etc. of the air pump 520.

Electronic labels RFID or photoelectric switches can identify different syringes 600 at that time. The principle of electronic label identification is briefly described below:
1) The electronic label stores corresponding information through the circuit board and software;
2) The identifier or reader runs on the PCB, and the antenna sends a radio frequency signal of a certain frequency;
3) When the electronic label is within the covering range of the radio frequency signal and the antenna sends the radio frequency signal of the certain frequency, an induced current is generated to obtain energy, and thus corresponding information is sent to the identifier, so that the corresponding syringe 600 can be determined;
4) After determining the syringe 600, the corresponding parameters are amended and corresponding adjustments are made in the application software.

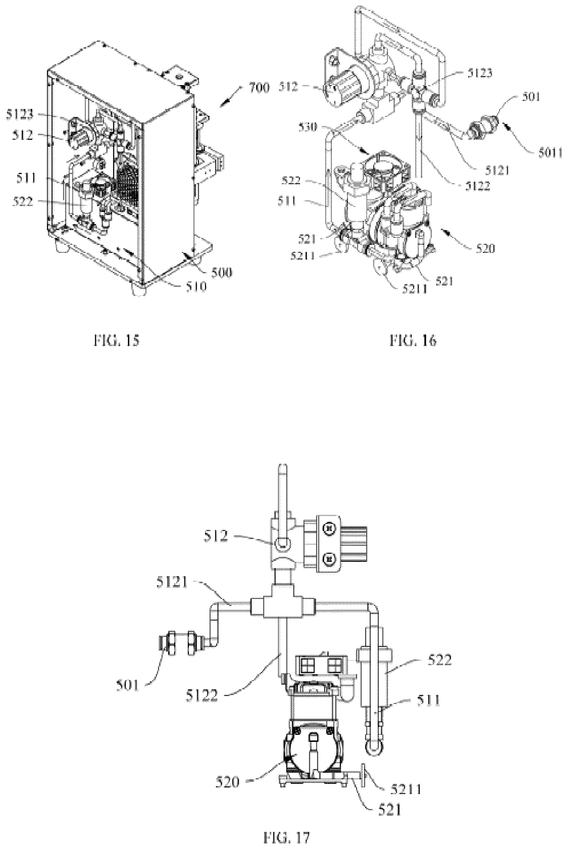

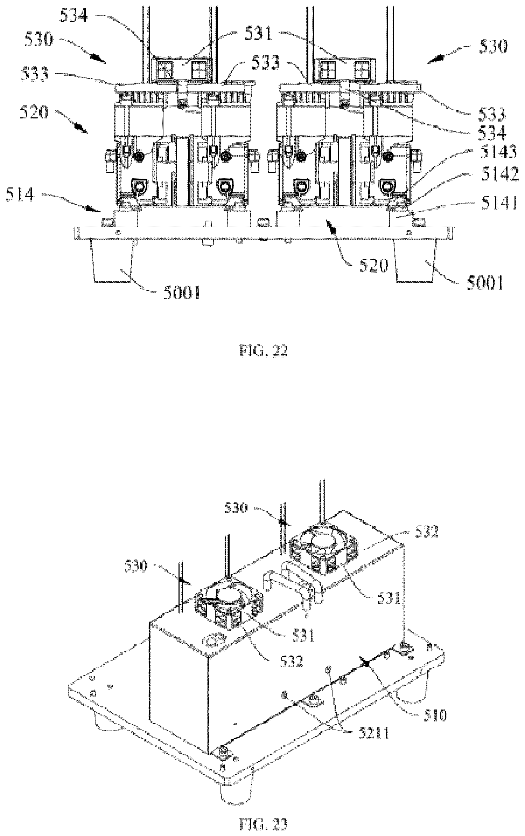

Similar to the above, in addition to the automatic identification of the adapter 630, the technical solution disclosed in the present application may also achieve multi-dimensional self-control through the setting of other identification labels. Referring to the embodiment shown in FIG. 16 and FIG. 23, the air pump 520 is provided with an air inlet pipe 521 connected to the outside of the pump chamber 510, a first filter 5211 is provided on the air inlet pipe 521, and a first filter identification label is provided on the first filter 5211. The interventional device is provided with a controlling device, which determines the parameters of the first filter 5211 by reading the first filter identification label. Referring to the embodiment shown in the drawings, a second filter 5011 is provided on the gas path connector 501, and a second filter identification label is provided on the second filter 5011. The interventional device is provided with a controlling device, which determines the parameters of the second filter 5011 by reading the second filter identification label. Through the corresponding identification labels, the controlling device can identify the status of the filter and record the usage time, thereby controlling the replacement cycle of the consumables as a whole. The specific identification process can be found in the description above and will not be repeated here.

The optimized setting of the liquid path part is to provide a good working basis for the operation of the liquid path part. Referring to the embodiment shown in the drawings, an air path connector 501 is installed on the housing 500, and a pump chamber 510 is provided at the bottom of the housing 500; an air pump 520 is installed in the pump chamber 510, and includes multiple pumps connected in parallel and is connected to the air path connector 501 through a main pipe 511; a pressure relief valve 512 is installed on the main pipe 511, and the outlet of the pressure relief valve 512 is connected to the pump chamber 510 through a pipeline; a cooling fan 531 thermally coupled to the air pump 520 is installed on the pump chamber 510; the liquid-phase fluid output by the liquid path connector 502 and the gas-phase fluid output by the air path connector 501 are mixed with each other to form a gas-liquid flow which is nebulized to achieve medication administration.

In combination with the above, it can be seen that the present application discloses an interventional device, including:
a housing 500, on which a liquid path connector 502 and a gas path connector 501 are mounted, a pump chamber 510 being provided at a bottom of the housing 500;
a fixing base 701 being fixed on the housing 500;
an adapter 630 being detachably connected to the fixing base 701, wherein electronic identification components cooperating with each other are provided between the adapter 630 and the fixing base 701;
a syringe 600 including a cylinder 610 being movably inserted into the adapter 630 and a piston 620 being slidably mounted in the cylinder 610, wherein the cylinder 610 has an outlet and is connected to the liquid path connector 502;
a drive mechanism 700 linked to the piston 620 of the syringe 600;
an air pump 520 being installed in the pump chamber 510, wherein the air pump 520 includes multiple pumps connected in parallel and is connected to the air path connector 501 through a main pipe 511;
a cooling assembly 530 being thermally coupled to the air pump 520.

Similarly, the present application discloses an interventional system, including:
a housing 500, on which a liquid path connector 502 and a gas path connector 501 are installed, a pump chamber 510 being provided at a bottom of the housing 500;
a fixing base 701 being fixed on the housing 500;
an adapter 630 being detachably connected to the fixing base 701, wherein electronic identification components cooperating with each other are provided between the adapter 630 and the fixing base 701;
a syringe 600 including a cylinder 610 being movably inserted into the adapter 630 and a piston 620 being slidably mounted in the cylinder 610, wherein the cylinder 610 has an outlet and is connected to the liquid path connector 502;
a drive mechanism 700 linked to the piston 620 of the syringe 600;
an air pump 520 being installed in the pump chamber 510, wherein the air pump 520 includes a plurality of pumps connected in parallel and is connected to the air path connector 501 through a main pipe 511;
a cooling assembly 530 being thermally coupled to the air pump 520;
an interventional catheter including a catheter body, wherein one end of the catheter body is the proximal end, and another end of the catheter body is a distal end that can extend into the bronchus, the proximal end is connected to the air path connector and the liquid path connector, an interior of the catheter body has a flow channel that can transport fluid from the proximal end to the distal end, the distal end of the catheter body is provided with a flow mixing structure, and a microfluidic chip is configured in the flow mixing structure, and the fluid in the flow channel is output after being nebulized through the microfluidic chip.

Referring to the embodiment shown in the drawings, the gas-liquid flow is used to be delivered to the interventional catheter, which includes a catheter body, one end of which is a proximal end and another end of which is a distal end that can extend into the bronchus, an interior of the catheter body is provided with a flow channel that can deliver fluid from the proximal end to the distal end, the distal end of the catheter body is provided with a fluid mixing structure, a microfluidic chip is configured in the fluid mixing structure, and the fluid in the flow channel is output after being nebulized through the microfluidic chip;
a distribution structure capable of acting on at least a portion of the fluid is arranged inside the microfluidic chip.

In combination with the above, it can be seen that the gas path part and the liquid path part of the interventional device disclosed in this application can be implemented separately or in combination with each other to effectively improve the treatment effect. In other aspects, referring to the embodiment shown in FIG. 1, the interventional device is provided with a controlling device connected to the drive mechanism 700, the controlling device is connected to an operating screen 800, the operating screen 800 is arranged on the outside of the housing 500 and is used to input control information and output parameter status.

Referring to the embodiment shown in FIG. 36 and FIG. 37, a shielding cover 801 is installed on the housing 500. The shielding cover 801 can shield the drive mechanism and provide an installation basis for a movable door. The movable door has a closed state for sealing the syringe 600 and/or the drive mechanism 700 and a corresponding open state. The interventional device is provided with a controlling device connected to the drive mechanism 700. The controlling device reads different states of the movable door through a sensor and responds according to preset rules.

A group of circuit elements is provided on the replaceable component in the interventional device. Detectable signals which can be detected by the group of circuit elements are used as identification marks. The controlling device reads these identification marks and responds according to preset rules. The replaceable components in the interventional device are provided with circuit elements as identification marks, wherein the circuit elements include multiple resistors that constitute a circuit topology structure, at least one position in the circuit topology structure serves as a sampling point, and the controlling device distinguishes different components according to the electrical signals measured at the sampling points.

With reference to the embodiments shown in the drawings, it can be seen that the drive mechanism, except for the motor, is located on a front panel of the housing and as a whole at a side of the operating screen. Such arrangement is convenient for installation and observation of the syringe. The motor of the drive mechanism is arranged inside the housing, which is beneficial for controlling noise and vibration. The components of the gas-phase part are located inside the housing, which is convenient to control noise and set up pipelines. Overall, the drive mechanism is configured in shape of "7", with the electrodes located at the bottom of the housing. Correspondingly, the gas path portion as a whole is located in a blank space of the 7-shaped drive mechanism, wherein the gas pump is arranged adjacent to the electrodes. The above arrangement is conducive to lowering the center of gravity, increasing the layout density, thereby controlling the product volume. The gas path connector and the liquid path connector are close to each other and arranged below the operation screen. Such arrangement can realize the centralized setting of the operation area, thereby facilitating the operation of medical staff.

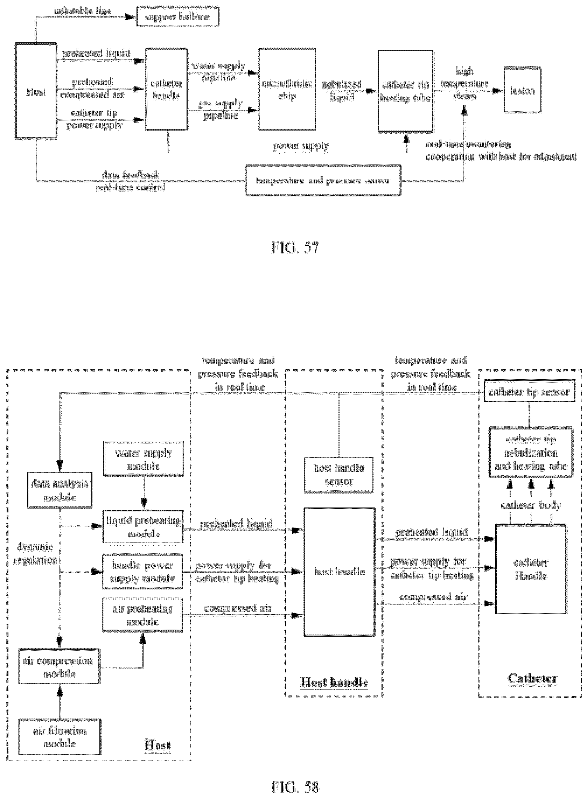

In addition to the parameter control mentioned above, the nebulized medication delivery system may also be set to closed-loop temperature control. As shown in FIG. 57 and FIG. 58, the nebulized medication delivery system includes a host, a host handle connected to the host, and a catheter provided on the host handle;
wherein the host includes:
   an air filtration module, an air compression module and an air preheating module connected in series, configured for providing a gas source;
   a data analysis module;
   a handle power supply module being controlled by the data analysis module;
   a water supply module and a liquid preheating module connected in series, , configured for providing a liquid-phase fluid;
wherein the host handle includes:
   a host handle sensor, configured for providing signals to the data analysis module;
   a host handle body, configured for receiving and delivering preheated liquid-phase fluid, preheated compressed air and energy from the handle power supply module;
wherein the catheter includes:
   a catheter tip sensor configured for providing a signal source for the data analysis module;
   a catheter handle and a catheter body, configured for receiving and delivering preheated liquid-phase fluid, preheated compressed air and energy from the handle power supply module;
   a catheter tip nebulization assembly, configured for receiving and transporting the preheated liquid-phase fluid and preheated compressed air and realizing nebulization;
   a catheter tip heating module, configured for receiving energy from the handle power supply module and heating the nebulized medium.

In this embodiment, a multi-stage heating method is adopted. The liquid is first preheated in the host, and then transported to the catheter tip through the host handle and the catheter. At the catheter tip, the liquid is first nebulized through a microfluidic chip, and then heated in multiple stages to generate hot steam, thereby improving the heating efficiency and reducing tissue damage along the entire path. And, through real-time monitoring, dynamic adjustment of the steam energy value is achieved by adjusting parameters such as water pressure, air pressure and heating temperature. The traditional method of directly heating water into steam is eliminated. The host first transports room temperature water to the liquid preheating module. After the water is preheated to a predetermined temperature, it is transported to the microfluidic chip at the catheter tip through the host handle and the catheter body. At the same time, the air filtered by the air filtration module passes through the air compression module to generate compressed air, and then the compressed air is also preheated through the air preheating module and transported to the microfluidic chip at the catheter tip through the host handle and the catheter. The microfluidic chip converts water into water mist through the compressed air, and then the water mist is heated again through the catheter tip heating tube to achieve rapid nebulization. At the same time, the water temperature and water pressure of the host handle and the steam temperature and pressure at the catheter tip can be monitored in real time through the catheter tip sensor, and the steam can be precisely regulated by adjusting the water supply flow of the host and the pressure of the compressed air. It overcomes the disadvantages of existing ablation methods, such as large energy loss; releasing a large amount of heat to the outside by the entire catheter and thus posing a risk of burns; a single output temperature and pressure of the ablation device and thus cannot adapt to a variety of surgical environments; it cannot detect the outlet temperature in real time, and thus cannot provide real-time information feedback to the surgeon.

Furthermore, a corresponding heating module may be provided on the catheter handle to heat the liquid-phase medium and/or gas-phase medium passing through the catheter handle, thereby compensating for the heat loss caused by long-distance transportation. In terms of temperature control, the nebulized medication delivery system in this application can achieve three-stage heating through the host, the catheter handle, and the catheter tip heating module, which can achieve more precise closed-loop temperature control. The heating module is mainly used for the nebulization of medications or water, and the cells, viruses and hydrogels are usually not heated.

Therefore, the present application further discloses a multi-stage heating nebulization system, including:
a perfusion device, configured for supplying liquid-phase fluid and gas-phase fluid, wherein the perfusion device includes a primary heating device for heating at least one of the liquid-phase fluid and gas-phase fluid;
an interventional catheter, a proximal end of which is in communication with the perfusion device for receiving the gas-phase fluid and the liquid-phase fluid respectively, a secondary heating device being provided at a distal end of the interventional catheter;
a sampling device, configured for collecting state parameters of the liquid-phase fluid and gas-phase fluid;
a controlling device connected to the sampling device, configured for receiving the state parameters and controlling the perfusion device according to the state parameters.

In this embodiment, the secondary heating device of the interventional catheter can be implemented by the heating module mentioned above. The primary heating device of the perfusion device and the secondary heating device of the interventional catheter provide the nebulization system with a two-stage heating capability, effectively overcoming the technical defects existing in the prior art.

On this basis, the interventional catheter further includes a tertiary heating device located at the proximal end. The tertiary heating device may be provided on the operating handle of the interventional catheter (such as the host handle in the attached drawings), so that the nebulization system realizes segmented heating capability with three-stage heating. Compared with the existing method of directly heating water into steam, the present embodiment preheats the liquid-phase fluid and the gas-phase fluid to a predetermined temperature through the first-stage heating device of the perfusion device, and then transmits them to the interventional catheter. The interventional catheter can compensate for the heat loss or the preset temperature difference during the transportation process through the secondary heating device at the proximal end. The interventional catheter transports the liquid-phase fluid and the gas-phase fluid to the distal end, and the water is converted into water mist through the microfluidic chip, and then the water mist is heated again by the tertiary heating device to achieve rapid vaporization. At the same time, the steam temperature and pressure at the distal end can be monitored in real time by the sensor at the distal end and transmitted back to the perfusion device, so that the steam can be precisely regulated by adjusting the transportation parameters of the liquid-phase fluid and the gas-phase fluid (such as the flow rate of the liquid-phase fluid and the gas pressure of the gas-phase fluid). By means of setting the three-stage heating device, the temperature index of the fluid at each stage can be effectively control, providing a structural basis for stable energy transmission.

It is not difficult to understand from the above that the present application further discloses an interventional system control method, including:
Step S100, resetting the drive mechanism 700;
Step S200, pre-injecting the liquid-phase fluid into the interventional catheter under the condition that the syringe 600 is correctly installed,
Step S300, pre-infusing the interventional catheter with a gas-phase fluid;
Step S400: driving the liquid-phase fluid and the gas-phase fluid according to preset parameters, so that the fluid is atomized through the intervention catheter and output.

Referring to the embodiment shown in the drawings, the preset parameters include at least one of the following:
type of the liquid-phase fluid, dosage of the liquid-phase fluid, flow rate of the liquid-phase fluid, type of gas-phase fluid, pressure of gas-phase fluid, model of the syringe 600, duration of nebulization operation;
temperature threshold, pressure threshold, flow rate threshold, and flow velocity threshold of each fluid; and
force threshold between the drive mechanism 700 and the piston 620.

In one embodiment, the interventional system control method further includes:
Step S000, self-checking of the hardware;
determining whether the identification mark and/or sensor information is consistent with the input information received by the controlling device, and if inconsistent, giving an alarm or stopping the current step.

In one embodiment, the step S000 is performed before the step S100.

In one embodiment, in step S000, it is determined whether the adapter identification mark is consistent with the parameters of the syringe 600 in the input information received by the controlling device.

In one embodiment, the sliding seat 703 has a first limit position away from the syringe 600 and a second limit position close to the syringe 600 along its own movement direction. When the drive mechanism 700 is reset, the sliding seat 703 moves toward the first limit position. The movement result may approach the first limit position or reach the limit position. There needs to be enough space to install the corresponding model of syringe.

The pre-infusion of liquid-phase fluid and gas-phase fluid can be performed simultaneously, or the start time can be slightly different, but the order is not strictly limited. For example, gas-phase fluid can be introduced into the interventional catheter first, and the liquid-phase fluid can be introduced during this period.

In one embodiment, the pre-infusion of liquid-phase fluid includes:
driving the piston 620 and monitoring an acting force between the drive mechanism 700 and the piston 620 in real time until the acting force reaches a threshold value.

In one embodiment, the threshold value of the acting force is 200N.

In the initial stage of movement, the drive mechanism 700 (sliding seat 703) has not yet contacted the piston 620 or has insufficient pre-tightening force, so the force value obtained by the thrust detector is zero or in a low value range;
when the drive mechanism 700 and the piston 620 begin to act with each other, the acting force increases. During this process, the liquid-phase fluid is gradually perfused into the corresponding flow channel of the interventional catheter. However, due to the change in resistance at different positions in the flow channel, the acting force is not stable. When the perfusion is complete, the resistance is generally stable and the acting force does not change significantly. At this time, the pre-perfusion can be regarded as completed. The monitoring of the change in this process may be applicable to the pre-perfusion process of gas-phase fluid.

Of course, due to the limited space in the interventional catheter, the duration of the pre-perfusion may be used alone to determine whether the process is complete, or the duration of the pre-perfusion may be combined with the change in the pressure of the fluid for mutual verification.

Liquid-phase fluid can be measured more accurately, so it is also possible to determine whether the pre-perfusion is complete by using only or in combination with the consumption of the pre-perfused liquid.

In one embodiment, the pre-perfusion of gas-phase fluid includes:
turning on the air pump 520 to input gas-phase fluid until the time and/or pressure meet the preset value. The preset time value may preferably be 1~5 minutes, and the preset pressure value may refer to the pressure value of the gas-phase fluid mentioned below, and may also preferably be 0.1MPa to 0.7MPa.

Step S400 starts nebulization. In order to stabilize the output and ensure the nebulization effect, closed-loop control can be adopted. For example, step S400 further includes:
Step S410, obtaining current state parameters of the fluid;
Step S420, generating a corresponding control signal according to the state parameters;
Step S430, driving the perfusion device through the control signal to adjust the supply of the fluid (e.g., adjust the pressure, flow rate, etc.).

In one embodiment, the liquid-phase fluid is loaded with micron-sized particles, and the particle size of the micron-sized particles may be 20 to 100µm.

Referring to the embodiment shown in the drawings, the current state parameters of the fluid in step S410 may include:
a flow rate or a pressure of the liquid-phase fluid (for example, the flow rate threshold is set to 3 ml/min, and the pressure threshold is set to 0.4MPa, the threshold may also be a numerical range);
a pressure of the gas-phase fluid (for example, the pressure threshold is set to 0.2MPa, and the threshold may also be a numerical range).

In step S420, the control unit obtains the state parameters and compares it with the threshold. If they match or the error is within the permitted range, a corresponding control signal is generated to maintain the status quo. When any state parameter to be monitored does not match the threshold or deviates from the threshold range, a corresponding control signal is generated for adjustment.

For the liquid-phase fluid, the thrust applied to the piston 620 can be changed to increase or decrease the flow rate and pressure accordingly. For the gas-phase fluid, the pressure can be adjusted through pipeline devices such as pressure reducing valves and regulating valves, or be adjusted by directly controlling the air pump 520, for example, using PWM to adjust the output of the motor 702 of the air pump 520 to increase or decrease the air pressure.

Combined with the specific application of liquid-phase fluid loaded with micron-sized particles, an ideal nebulization effect can be obtained when the pressure of the liquid-phase fluid is 0.2~0.76MPa and the pressure of the gas-phase fluid is 0.15~0.4MPa, while both the application efficiency and efficacy can be taken into account.

In step S400, in order to monitor the progress as a whole, the amount of liquid-phase fluid used can be detected in real time (it can be determined by the stroke that the piston 620 has moved in combination with the model of the syringe 600, such as the product of the cross-section of the cylinder 610 and the stroke of the piston 620). When the amount reaches a preset value, step S400 is finished.

In one embodiment, when step S400 is finished, the drive mechanism 700 stops running first, and after a delay (for example, 3~10 seconds), the air pump 520 stops running, which can avoid residual liquid in the interventional catheter as much as possible and reduce the waste of therapeutic substances in the liquid-phase.

Similarly, in one embodiment, a nebulization method is provided, including:
inputting a fluid into an interventional catheter and outputting the fluid after being nebulized through the interventional catheter, wherein the fluid comprises a liquid-phase fluid and a gas-phase fluid, and at least one of the liquid-phase fluid and the gas-phase fluid carries a therapeutic substance;
obtaining current state parameters of the fluid during the nebulization process, and generating corresponding control signal based on the state parameters;
regulating the supply of the fluid accordingly to the control signal.

In another embodiment, a method for treating and administering medications to a natural cavity of a human body is provided, including:
providing an interventional catheter with opposed distal end and proximal end;
inserting the distal end of the interventional catheter to a lesion site, wherein a fluid is input into the proximal end of the interventional catheter, nebulized during flowing through the interventional catheter and then output to the lesion site, wherein the fluid includes a liquid-phase fluid and a gas-phase fluid, and at least one of the liquid-phase fluid and the gas-phase fluid carries a therapeutic substance;
generating a control signal to adjust the supply of the fluid accordingly during the nebulization process, wherein the control signal may be generated in one or more of the following manners:
   manner a): obtaining the current state parameters of the fluid and generating corresponding control signals according to the state parameters;
   manner b): observing the nebulization and application effects through an endoscope and generating corresponding control signals.

The state parameters mentioned above include at least the pressure of the liquid-phase fluid and the pressure of the gas-phase fluid. Unless otherwise emphasized, the pressure in this application is expressed as a physical quantity related to pressure or having a conversion relationship, and is strictly limited to the definition of pressure in physical concepts.

In another embodiment, a cell spraying method is provided, including:
providing an interventional catheter with opposed distal end and proximal end;
inserting the distal end of the interventional catheter into the lesion site;
supplying a fluid loaded with cells to the proximal end of the interventional catheter, wherein the fluid is nebulized during flowing through the interventional catheter and then output to the lesion site;
wherein a particle size of the cells is 15~120µm, a pressure of the liquid-phase fluid is 0.2~0.76MPa, and a pressure of the gas-phase fluid is 0.1~0.4MPa.

For the parameters, preferably, the cells include one or more of lung stem cells, hematopoietic stem cells, mesenchymal stem cells, neural stem cells, exosomes, and lung precursor cells, and the cell content in the liquid-phase fluid is 1*10⁶~8*10⁶ cells/ml. The amount of the liquid-phase fluid used is 10~40ml, and the flow rate of the liquid-phase fluid is less than or equal to 4 ml/min. The particle size of the cells is 15~30µm, and the particle size of the droplets is 15~40µm. The pressure of the gas-phase fluid is 0.1~0.35MPa, and the medium of the gas-phase fluid is one of the follows: air, air and hydrogen, and oxygen.

For the parameters, preferably, the concentration of the therapeutic medium in the liquid-phase fluid is 2*10⁶ cells/ml.

Hematopoietic stem cells are used to treat hematological diseases, mesenchymal stem cells are used to treat cardiovascular diseases, cirrhosis, and neurological diseases, neural stem cells are used to replenish damaged nerve cells, lung precursor cells are used to treat chronic obstructive pulmonary disease, and extracellular vesicles include small molecule active peptides and protein cells. Extracellular vesicles can participate in the body's immune response, antigen presentation, cell migration, cell differentiation, tumor invasion, etc.

In another embodiment, a method for spraying inactivated viruses is provided, including:
providing an interventional catheter with opposed distal end and proximal end;
inserting the distal end of the interventional catheter to a lesion site;
supplying a fluid loaded with viruses into the proximal end of the interventional catheter, wherein the fluid is nebulized through the interventional catheter and then output to the lesion site;
wherein a particle size of the virus is less than 21µm, a pressure of the liquid-phase fluid is 0.2~0.76MPa, and a pressure of the gas-phase fluid is 0.2~0.6MPa.

For the parameters, preferably, the amount of the liquid-phase fluid is 5~10ml, and the flow rate of the liquid-phase fluid is less than or equal to 4 ml/min. The particle size of the virus is 50-350nm, and the particle size of the droplets is 20~300µm.

Inactivated viruses include inactivated polio vaccine, inactivated Japanese encephalitis vaccine, influenza vaccine, rabies vaccine, inactivated hepatitis A vaccine, EV71 hand, foot and mouth disease vaccine, and inactivated COVID-19 vaccine.

In another embodiment, a medication spraying method is provided, including:
providing an interventional catheter with opposed distal end and proximal end;
inserting the distal end of the interventional catheter to a lesion site;
supplying a fluid loaded with nano-drugs into the proximal end of the interventional catheter, wherein the fluid is nebulized through the interventional catheter and then output to the lesion site;
wherein a particle size of the nano-drug is less than 69µm, a pressure of the liquid-phase fluid is 0.2~0.76MPa, and a pressure of the gas-phase fluid is 0.2~0.6MPa.

For the parameters, preferably, the amount of liquid-phase fluid is 5~10 ml, and the flow rate of the liquid-phase fluid is less than or equal to 4 ml/min. The particle size of nanomedication is 50-350nm, and the particle size of droplets is 20~300µm.

Drugs include ribavirin, acyclovir, ganciclovir, oseltamivir, vidarabine, amantadine, voriconazole, amphotericin B, isoniazid, dexamethasone, sildenafil citrate, isoniazid, and N-acetylcysteine. Among them, ribavirin is used to treat respiratory syncytial virus, influenza virus, hepatitis A virus, and adenovirus, acyclovir is used to treat herpes virus and varicella virus, ganciclovir is used to treat cytomegalovirus, oseltamivir is used to treat influenza A and influenza B, adenosine is used to treat chronic hepatitis B, herpes virus, and varicella virus, amantadine is used to treat influenza virus, dexamethasone is used for new coronary pneumonia, asthma, chronic obstructive pulmonary disease, croup, and cerebral edema, sildenafil citrate is used to treat erectile dysfunction, voriconazole is used to treat aspergillosis, Candida, Actinomyces, and Fusarium, amphotericin B is used to treat visceral or systemic infections caused by Cryptococcus, Coccidioides, Capsular Histoplasma, Blastomyces, Sporothrix, Candida, Mucor, Aspergillus, etc., isoniazid is used to treat Mycobacterium tuberculosis, and N-acetylcysteine is used to treat expectorant.

In this embodiment, ribavirin, acyclovir, ganciclovir, oseltamivir, vidarabine, and amantadine are used to treat viral infections, and voriconazole, amphotericin B, isoniazid, and N-acetylcysteine are used to treat fungal infections.

| Parameter | Spraying of cells | Spraying of inactivated virus | Spraying of medications | Spraying of high viscosity fluid |
|---|---|---|---|---|
| Specific types of therapeutic media | Lung stem cells; Hematopoietic stem cells; Mesenchymal stem cells; Neural stem cells; Extracellular vesicles; Pulmonary precursor cells | Inactivated polio vaccine; Inactivated Japanese encephalitis vaccine; Influenza vaccine; Rabies vaccine; Inactivated hepatitis A vaccine; EV71 hand, foot and mouth disease vaccine; Inactivated COVID-19 vaccine | Ribavirin; Acyclovir; Ganciclovir; Oseltamivir; Vidarabine; Amantadine; Fluconazole; Bisamphotericin B nuclide; Dexamethasone; Sildenafil Citrate; Isoniazid; Acetylcysteine | High viscosity fluid microsphere; Hydrogel |
| Particle size of therapeutic medium | 5~120µm | 50~21000nm | 50~69000nm | / |
| Concentration of therapeutic medium in liquid-phase fluid | 1*10⁶~8*10⁶ cells/ml | / | / | / |
| amount of liquid-phase fluid used | 10~40 ml | 5~10 ml | 5~10 ml | 5~10 ml |
| Flow rate of liquid-phase fluid | 1~4 ml/min | 1~4 ml/min | 1~4 ml/min | 1~4 ml/min |
| Pressure threshold of liquid-phase fluid | 0.2~0.76 MPa | 0.2~0.76 MPa | 0.2~0.76 MPa | 0.2~0.76 MPa |
| Flow rate threshold (maximum injection volume) | 4 ml/min | 4 ml/min | 4 ml/min | 4 ml/min |
| Force threshold between drive mechanism and piston | < 200N | < 200N | < 200N | < 200N |
| Types of gas-phase fluid | air; or air + hydrogen; or oxygen | air; or air + hydrogen; or oxygen | air; or air + hydrogen; or oxygen | air; or air + hydrogen; or oxygen |
| Pressure of gas-phase fluid | 0.1~0.4 MPa | 0.2~0.6 MPa | 0.2~0.6 MPa | 0.2~0.6 MPa |
| Temperature threshold of fluid | < 37°C | < 37°C | < 37°C | < 37°C |
| Particle size range of droplets | 5~120µm | 5~300µm | 10~300µm | 20~60µm |
| Preferred particle size of droplets | 5~50µm | 5~15µm | 10~30µm | 40~60µm |
| Time for nebulization process | 5~15min | 5~15min | 5-15min | 5-15min |
| Embodiment of preferred application | FIG. 6 | FIG. 9a~FIG. 10c | FIG. 2 and FIG. 5 | FIG. 8a~FIG. 8b |

In combination with the above, the present application further discloses a method for spraying a high-viscosity fluid in a natural cavity of a human body, wherein the fluid includes a first fluid and a second fluid, one of the first fluid and the second fluid is a liquid-phase fluid and the other is a gas-phase fluid, and the high-viscosity fluid is loaded on the liquid-phase fluid;
wherein the first fluid and the second fluid are nebulized into droplets with a particle size not larger than 60µm after at least two collisions, wherein the droplets contain inactivated viruses, and move to the lesion location and infiltrate the lesion.

The high-viscosity fluid is loaded on the liquid-phase fluid, the droplets size after nebulization is greater than 20µm, the pressure of the liquid-phase fluid is 0.2-0.76MPa, and the pressure of the gas-phase fluid is 0.1-0.4MPa.

Among them, the high viscosity fluid includes hydrogel.

The present application further discloses a high-viscosity fluid spraying system for natural cavity of a human body, including an interventional catheter and a perfusion device for supplying a first fluid and a second fluid to the interventional catheter, respectively;
wherein the interventional catheter includes a catheter body 900, one end of the catheter body 900 is a proximal end 901, and another end of the catheter body 900 is a distal end 902 that can extend into the bronchus, a flow channel is configured in the catheter body 900 and capable of transporting fluid from the proximal end 901 to the distal end 902, a flow mixing structure 910 is provided at the distal end 902 of the catheter body 900, and the fluid in the flow channel is output after being mixed through the flow mixing structure 910;
wherein the flow mixing structure 910 at least includes a first fluid inlet 111, a second fluid inlet 112 and an outlet 120, and each fluid inlet 110 is independently configured with a flow channel in the catheter body 900;
wherein a microfluidic chip 920 is provided in the fluid mixing structure 910, the first fluid inlet 111 is located at a proximal end side of the microfluidic chip 920, and the second fluid inlet 112 is located at a lateral side of the microfluidic chip 920, an interior of the microfluidic chip 920 is configure as a chip channel structure interconnecting the outlet 120 and each fluid inlet 110, wherein the chip channel structure includes:
   a main channel 130 with one end thereof connected to the first fluid inlet 111 at the proximal end side and another end thereof connected to the outlet 120;
   a lateral channel 140 with one end thereof connected to the second fluid inlet 112 at the lateral side and another end thereof connected to the main channel 130;
   a distribution structure capable of acting on at least a portion of the fluid being provided in the chip channel structure;
   wherein a particle size of the nebulized droplets is greater than 20µm, one of the first fluid and the second fluid is a liquid-phase fluid, and the other of the first fluid and the second fluid is a gas-phase fluid, a pressure of the liquid-phase fluid is 0.2~0.76MPa, and a pressure of the gas-phase fluid is 0.1~0.4MPa.

The present application further discloses a method for spraying cells into natural cavity of a human body, including a first fluid and a second fluid, wherein one of the first fluid and the second fluid is a liquid-phase fluid and the other is a gas-phase fluid, the cells are loaded in the liquid-phase fluid, and a particle size of the cells is less than or equal to 120µm;
wherein the first fluid and the second fluid are nebulized into droplets with a particle size not larger than 120µm after at least two collisions, wherein the droplets contain inactivated viruses, and move to the lesion location and infiltrate the lesion.

The cells are loaded in the liquid-phase fluid, the particle size of the cells is 15~120µm, the pressure of the liquid-phase fluid is 0.20.76MPa, and the pressure of the gas-phase fluid is 0.1~0.4MPa.

The present application further discloses a cell spraying system for natural cavity of a human body, including an interventional catheter and a perfusion device for supplying a first fluid and a second fluid to the interventional catheter, respectively;
wherein the interventional catheter includes a catheter body 900, one end of the catheter body 900 is a proximal end 901, and another end of the catheter body 900 is a distal end 902 that can extend into the bronchus, a flow channel is configured inside the catheter body 900 and can transport fluid from the proximal end 901 to the distal end 902. A flow mixing structure 910 is provided at the distal end 902 of the catheter body 900, and the fluid in the flow channel is output after being mixed through the flow mixing structure 910;
wherein the flow mixing structure 910 at least includes a first fluid inlet 111, a second fluid inlet 112 and an outlet 120, and each fluid inlet 110 is independently configured with a flow channel in the catheter body 900;
wherein a microfluidic chip 920 is provided in the fluid mixing structure 910, the first fluid inlet 111 is located at the proximal end side of the microfluidic chip 920, and the second fluid inlet 112 is located at a lateral side of the microfluidic chip 920, an interior of the microfluidic chip 920 is configured as a chip channel structure interconnecting the outlet 120 and each fluid inlet 110, wherein the chip channel structure includes:
   a main channel 130 with one end thereof connected to the first fluid inlet 111 at the proximal end side and another end thereof connected to the outlet 120;
   a lateral channel 140 with one end thereof connected to the second fluid inlet 112 at the lateral side and another end thereof connected to the main channel 130;
   a distribution structure capable of acting on at least a portion of the fluid being provided in the chip channel structure;
   wherein a particle size of the cells is 15~120µm, one of the first fluid and the second fluid is a liquid-phase fluid and the other is a gas-phase fluid, a pressure of the liquid-phase fluid is 0.2~0.76MPa, and a pressure of the gas-phase fluid is 0.1~0.4MPa.

The present application further discloses a method for spraying medication into natural cavity of a human body, including a first fluid and a second fluid, wherein one of the first fluid and the second fluid is a liquid-phase fluid and the other is a gas-phase fluid, the medication is loaded in the liquid-phase fluid, and a particle size of the medication is not larger than 69000nm;
wherein the first fluid and the second fluid are nebulized into droplets with a particle size not larger than 300µm after at least two collisions, wherein the droplets contain inactivated viruses, and move to the lesion location and infiltrate the lesion.

The medication is loaded in a liquid-phase fluid, the particle size of the medication is less than 69µm, the pressure of the liquid-phase fluid is 0.2~0.76MPa, and the pressure of the gas-phase fluid is 0.2~0.6MPa. Furthermore, the particle size of the medication is less than 600nm.

The present application further discloses a medication spraying system for natural cavity of a human body, including an interventional catheter and a perfusion device for supplying a first fluid and a second fluid to the interventional catheter, respectively;
wherein the interventional catheter includes a catheter body 900, one end of the catheter body 900 is a proximal end 901, and another end of the catheter body 900 is a distal end 902 that can extend into the bronchus, a flow channel is configure inside the catheter body 900 and can transport fluid from the proximal end 901 to the distal end 902, a flow mixing structure 910 is provided at the distal end 902 of the catheter body 900, and the fluid in the flow channel is output after being mixed through the flow mixing structure 910;
Wherein the flow mixing structure 910 at least includes a first fluid inlet 111, a second fluid inlet 112 and an outlet 120, and each fluid inlet 110 is independently configured with a flow channel in the catheter body 900;
Wherein a microfluidic chip 920 is provided in the fluid mixing structure 910, the first fluid inlet 111 is located at the proximal end side of the microfluidic chip 920, and the second fluid inlet 112 is located the lateral side of the microfluidic chip 920, an interior of the microfluidic chip 920 is configured as a chip channel structure which interconnects the outlet 120 and each fluid inlet 110, wherein the chip channel structure includes:
   a main channel 130 with one end thereof connected to the first fluid inlet 111 at the proximal end side and another end thereof connected to the outlet 120;
   a lateral channel 140 with one end thereof connected to the second fluid inlet 112 at the lateral side and another end thereof connected to the main channel 130;
   a distribution structure capable of acting on at least a portion of the fluid being provided in the chip channel structure;
   wherein one of the first fluid and the second fluid is a liquid-phase fluid, and the other of the first fluid and the second fluid is a gas-phase fluid, the medication is loaded in the liquid-phase fluid, a particle size of the medication is less than 69µm, a pressure of the liquid-phase fluid is 0.2~0.76MPa, and a pressure of the gas-phase fluid is 0.2~0.6MPa.

Furthermore, the particle size of the medication is less than 600nm.

The present application further discloses a method for spraying inactivated viruses into natural cavity of a human body, including a first fluid and a second fluid, wherein one of the first fluid and the second fluid is a liquid-phase fluid and the other is a gas-phase fluid, the inactivated virus is loaded in the liquid-phase fluid, wherein a particle size of the inactivated virus is not larger than 21000nm;
wherein the first fluid and the second fluid are nebulized into droplets with a particle size not larger than 300µm after at least two collisions, wherein the droplets contain inactivated viruses, and move to the lesion location and infiltrate the lesion

The inactivated virus is loaded in the liquid-phase fluid, the particle size of the inactivated virus is less than 21µm, the pressure of the liquid-phase fluid is 0.2~0.76MPa, and the pressure of the gas-phase fluid is 0.2~0.6MPa. Furthermore, the particle size of the inactivated virus is less than 600nm.

The present application further discloses a inactivated virus spraying system for natural cavity of a human body, including an interventional catheter and a perfusion device for supplying a first fluid and a second fluid to the interventional catheter, respectively;
wherein the interventional catheter includes a catheter body 900, one end of the catheter body 900 is a proximal end 901, and another end of the catheter body 900 is a distal end 902 that can extend into the bronchus, a flow channel is provided inside the catheter body 900 and can transport fluid from the proximal end 901 to the distal end 902, a flow mixing structure 910 is provided at the distal end 902 of the catheter body 900, and the fluid in the flow channel is output after being mixed through the flow mixing structure 910;
wherein the flow mixing structure 910 at least includes a first fluid inlet 111, a second fluid inlet 112 and an outlet 120, and each fluid inlet 110 is independently configured with a flow channel in the catheter body 900;
wherein a microfluidic chip 920 is provided in the fluid mixing structure 910, the first fluid inlet 111 is located at the proximal end side of the microfluidic chip 920, and the second fluid inlet 112 is located at the lateral side of the microfluidic chip 920, an interior of the microfluidic chip 920 is configured as a chip channel structure interconnecting the outlet 120 and each fluid inlet 110, wherein the chip channel structure includes:
   a main channel 130 with one end thereof connected to the first fluid inlet 111 at the proximal end side and another end thereof connected to the outlet 120;
   a lateral channel 140 with one end thereof connected to the second fluid inlet 112 at the lateral side, and another end connected to the main channel 130;
   a distribution structure capable of acting on at least a portion of the fluid being provided in the flow channel of the chip;
   wherein one of the first fluid and the second fluid is a liquid-phase fluid and the other is a gas-phase fluid, the inactivated virus is loaded in the liquid-phase fluid, wherein a particle size of the inactivated virus is less than 21µm, a pressure of the liquid-phase fluid is 0.2~0.76MPa, and a pressure of the gas-phase fluid is 0.2~0.6MPa. Optionally, the particle size of the inactivated virus is less than 600nm.

It is not difficult to understand that the interventional catheter and the nebulization system in each of the embodiments mentioned above can respectively implement each of the methods mentioned above. Among them, the high-viscosity fluid spraying method is preferably the embodiment shown in FIG. 46 to FIG. 47, the cell spraying method is preferably the embodiment shown in FIG. 44 to FIG. 45 and FIG. 48 to FIG. 53, the inactivated virus spraying method is preferably the embodiment shown in FIG. 51 to FIG. 53, and the medication spraying method is preferably the embodiment shown in FIG. 40 to FIG. 43.

It is not difficult to understand that the above-mentioned spraying system further includes:
a sampling device, configured for collecting state parameters of the fluid;
a controlling device connected to the sampling device and configured for receiving the state parameters and controlling the perfusion device according to the state parameters.

Similarly, the above-mentioned spraying system further includes a housing, on which a gas path connector and a liquid path connector are installed, wherein a pump chamber is provided at a bottom of the housing;
wherein he perfusion device includes:
a first perfusion device including a syringe and a drive mechanism, wherein the syringe includes a cylinder installed in the housing and a piston slidably installed in the cylinder, the cylinder has an outlet and is connected to the liquid path connector, and the drive mechanism is linked to the piston;
a second perfusion device including an air pump installed in the pump chamber, wherein the air pumps includes multiple pump connected in parallel and is connected to the air path connector through a main pipe. Furthermore, the spraying system further includes a cooling assembly thermally coupled to the air pump.

The term "spraying system" specifically refers to one or more of the following:
high viscosity fluid spraying system, inactivated virus spraying system, cell spraying system, medication spraying system.

In combination with the above description, the present application further discloses a nebulization method based on an interventional catheter, wherein a microfluidic chip 920 is disposed in the distal end 902 of the interventional catheter, and the nebulization method includes:
supplying a first fluid and a second fluid into the interventional catheter respectively, wherein at least one of the first fluid and second fluid is loaded with a therapeutic substance;
the first fluid and second fluid entering the microfluidic chip 920 from the proximal end side and the lateral side of the microfluidic chip 920, respectively, wherein at least one of the first fluid and second fluid is pre-dispersed in the microfluidic chip 920 and then mixed with the other one of the first fluid and second fluid, so as to nebulize and then output to the lesion location;
obtaining the current state parameters of the fluid during the nebulization process, and generating corresponding control signal based on the state parameters;
regulating supply of the fluids accordingly to the control signal.

The present application further discloses a nebulized medication delivery system, including an interventional catheter and a perfusion device for supplying a first fluid and a second fluid to the interventional catheter, respectively;
wherein the interventional catheter includes a catheter body 900, one end of the catheter body 900 is a proximal end 901, and another end of the catheter body 900 is a distal end 902 that can extend into the bronchus, a flow channel being configured inside the catheter body 900 and can transport fluid from the proximal end 901 to the distal end 902, a flow mixing structure 910 is provided at the distal end 902 of the catheter body 900, the fluid in the flow channel is output after being mixed through the flow mixing structure 910.
wherein the flow mixing structure 910 at least includes a first fluid inlet 111, a second fluid inlet 112 and an outlet 120, and each fluid inlet 110 is independently configured with a flow channel in the catheter body 900;
wherein a microfluidic chip 920 is provided in the fluid mixing structure 910, the first fluid inlet 111 is located at the proximal end side of the microfluidic chip 920, and the second fluid inlet 112 is located at the lateral side of the microfluidic chip 920, an interior of the microfluidic chip 920 is configured as a chip channel structure which interconnects the outlet 120 and each fluid inlet 110, wherein the chip channel structure includes:
   a main channel 130 with one end thereof connected to the first fluid inlet 111 at the proximal end side and another end thereof connected to the outlet 120;
   a lateral channel 140 with one end thereof connected to the second fluid inlet 112 at the lateral side and another end connected to the main channel 130;
   a distribution structure capable of acting on at least a portion of the fluid being arranged in the chip channel structure.

The present application further discloses a control method for a nebulized medication delivery system, which includes an interventional catheter, wherein a microfluidic chip 920 is disposed in a distal end 902 of the interventional catheter, and the control method includes:
Step S510, supplying a gas-phase fluid firstly when the nebulized medication delivery system is started, wherein the adjustment target of the pressure of the gas-phase fluid is divided into at least two stages, each stage is independently configured with a PID parameter, and collecting a first real-time pressure of the gas-phase fluid when the gas-phase fluid is supplied, and determining the adjustment target stage to which the first real-time pressure belongs, and adjusting the pressure of the gas-phase fluid with the PID parameter corresponding to this adjustment target stage;
Step S520, supplying a gas-phase fluid with the therapeutic substance, wherein the liquid-phase fluid and the gas-phase fluid enter the microfluidic chip 920 from the proximal end side and the lateral side of the microfluidic chip 920, respectively, at least one of the liquid-phase fluid and gas-phase fluid is pre-dispersed in the microfluidic chip 920 and then mixed with the other one of the liquid-phase fluid and gas-phase fluid and nebulized and then output to a lesion location;
Step S530: collecting a second real-time pressure of the gas-phase fluid during nebulization and output, determining the adjustment target stage to which the second real-time pressure belongs, and adjusting the pressure of the gas-phase fluid with the PID parameter corresponding to this adjustment target stage;
Step S540, looping step S530 at a preset interval.

The present application further discloses a control method of the nebulized medication delivery system, including:
pre-setting delivery parameters of a liquid medium and generating a liquid medium delivery map according to the preset delivery parameters of the liquid medium;
generating a corresponding driving signal according to the liquid medium delivery map for driving a motor 702 which is used to drive a piston 620 of a syringe 600 to move to realize liquid delivery;
collecting an encoder signal set on the motor 702, obtaining a running position of the motor 702, and calculating the moving position of the piston 620 through the running position of the motor 702;
recording the moving position of the piston 620 and calculating an actual delivery map based on the moving position information of a plurality of pistons 620;
comparing the liquid medium delivery map with the actual delivery map according to a preset period, if the difference is less than a preset value, retaining the original liquid medium delivery map; if the difference is greater than or equal to the preset value, correcting the original liquid medium delivery map according to preset rules and generating a new liquid medium delivery map to replace the original liquid medium delivery map;
updating the liquid medium delivery map continuously until the liquid medium delivery is finished or paused.

The embodiments disclosed in the present application also include how to monitor and process abnormal information in real time. In one embodiment, in at least one of steps S100 to S400, abnormal signals are monitored in real time, and an alarm is issued or the current step is paused when an abnormal signal occurs.

The abnormal signals include at least one of the following:
a movable door on the housing 500 is in an open state;
whether the consumables are assembled correctly;
the number of times or time of use of consumables exceeds a threshold, wherein the consumables at least include filters and interventional catheters;
the model of the syringe 600 does not match the preset parameters;
the pressure or temperature of the air pump 520 exceeds a threshold;
the resistance of the drive mechanism 700 exceeds a threshold value;
the piston 620 reaches the limit position;
the position of the piston 620 is abnormal, that is, the deviation between the encoder of the motor 702 and the signal of the position sensor is large, for example, the position deviation of the piston 620 corresponding to the encoder of the motor 702 and the signal of the position sensor reaches 5mm.

On the basis of the above functions, the embodiments of the present application may also be configured as follows. In one embodiment, an operation screen 800 and/or physical keys are installed on the housing 500, which are respectively used to send control instructions, wherein the control instructions at least include:
a first signal, configured to trigger step S100;
a second signal, configured to trigger step S200;
a third signal, configured to trigger step S300;
a fourth signal, configured to trigger step S400;
a fifth signal, configured to suspend step S200 and/or step S300 and/or step S400;
a sixth signal, configured to resume step S200 and/or step S300 and/or step S400;
a seventh signal, configured to terminate any of the above steps.

In one embodiment, a display screen (or the above-mentioned operation screen 800) is installed on the housing 500. During the execution of step S400, the display screen virtually displays the progress of step 400, which may be in the form of a progress bar or percentage.

Referring to the embodiment shown in the drawings, the stroke L1 of the piston 620 is collected in real time, and the preset total amount of liquid-phase fluid corresponds to the stroke L2 of the piston 620, and the progress of step 400 is converted by the ratio of L1 to L2.

In one embodiment, the interventional device includes a controlling device and stores log files of device operation. The housing 500 is provided with a data interface that is in communication with the controlling device and is used to transmit the log files.

In one embodiment, the log file records at least the number of times the consumable is used and/or the usage time.

In one embodiment, a clock circuit is independently configured in the controlling device to provide clock signals to related circuit components.

In one embodiment, one or more indicator lights are installed on the housing 500 to indicate the device status in different ways, at least indicating abnormal signals.

In one embodiment, the indication method is flashing frequency, color change, light and dark state, or a combination thereof.

The technical features of the above-described embodiments may be arbitrarily combined. To make the description concise, not all possible combinations of the technical features in the above-described embodiments are described. However, as long as there is no contradiction in the combination of these technical features, they should be considered to be within the scope of this specification. When technical features in different embodiments are shown in the same figure, it can be regarded that the figure also discloses a combination embodiment of the various embodiments involved.

The above-described embodiments merely express several implementation manners of the present application, and the description thereof is relatively specific and detailed, but it should not be construed as limiting the scope of the patent application. It should be pointed out that, for ordinary technicians in this field, modifications and improvements can be made without departing from the concept of the present application, which all fall within the scope of protection of the present application.

## Claims

1. An interventional catheter, comprising a catheter body, one end of the catheter body being configured as a proximal end, another end of the catheter body being configured as a distal end that is capable of extending into a bronchus, an interior of the catheter body being configured with a flow channel that is capable of transporting a fluid from the proximal end to the distal end, a mixing structure being provided at the distal end of the catheter body, wherein the fluid in the flow channel is output after being mixed by the mixing structure;
the fluid mixing structure at least comprising a first fluid inlet, a second fluid inlet and an outlet, wherein each fluid inlet is independently configured with a flow channel in the catheter body;
a microfluidic chip being provided in the fluid mixing structure, wherein the first fluid inlet is located at a proximal end side of the microfluidic chip, the second fluid inlet is located at a lateral side of the microfluidic chip, an interior of the microfluidic chip is configured with a chip channel structure which interconnects the outlet and each fluid inlet, and wherein the chip channel structure comprises:
a main channel with one end thereof being connected to the first fluid inlet at the proximal end side and another end thereof being connected to the outlet;
a lateral channel with one end thereof being connected to the second fluid inlet at the lateral side and another end thereof being connected to the main channel; and
a distribution structure capable of acting on at least a portion of the fluid being arranged in the chip channel structure.

2. The interventional catheter according to claim 1, wherein the distribution structure is located at an intersection position of the main channel and the lateral channel.

3. The interventional catheter according to claim 1, wherein a channel width of the lateral channel comprises a narrowing part.

4. The interventional catheter according to claim 1, wherein the lateral channel has an extension trend line, and a channel width of the lateral channel gradually narrows along the extension trend line.

5. The interventional catheter according to claim 1, wherein a width of the lateral channel decreases continuously from upstream to downstream.

6. The interventional catheter according to claim 1, wherein intersection positions of the lateral channel and the main channel are configured as junctions, and an orientation of at least one junction is perpendicular to an extending direction of the main channel.

7. The interventional catheter according to claim 1, wherein the distribution structure is configured as at least one of the following manners:
Manner a: a plurality of branch channels connected to the lateral channel, wherein the lateral channel intersects with the main channel via the plurality of branch channels;
Manner b: a plurality of distribution members which are arranged at intervals or are separately provided in the main channel and/or the lateral channel.

8. The interventional catheter according to claim 7, wherein the distribution structure is configured as the distribution members located in the main channel and/or the lateral channel, and the distribution members guide a flowing direction of the fluid of one of the main channel and lateral channel in which the distribution members are located to be perpendicular to a flowing direction of the fluid in another one of the main channel and lateral channel.

9. The interventional catheter according to claim 8, wherein a portion of the main channel is configured as a widened expansion section, and at least a portion of the lateral channel intersects with the main channel at the expansion section, the distribution members are located in the expansion section and comprise at least a first distribution surface and a second distribution surface facing to a flowing direction of the fluid in the main channel.

10. The interventional catheter according to claim 1, wherein a fluid acceleration structure is provided in the main channel and/or the lateral channel, the fluid acceleration structure is located upstream of at least one intersection position of the main channel and the lateral channel in which at least one narrow opening is provided, and the main channel intersects with the lateral channel through the narrow opening.

11. The interventional catheter according to claim 10, wherein a sudden expansion section is provided in the main channel and/or the lateral channel and downstream of the fluid acceleration structure, an internal volume of the sudden expansion section is at least 15% larger than that of the fluid acceleration structure, and the intersection position with the narrow opening is arranged in the sudden expansion section.

12. The interventional catheter according to claim 11, wherein the sudden expansion section is arranged close to the fluid acceleration structure, and a chamber diameter of the sudden expansion section is at least 10% larger than that at an outlet of the fluid acceleration structure.

13. The interventional catheter according to claim 12, wherein there are two lateral channels arranged at two sides of the main channel, respectively, the fluid acceleration structure and the sudden expansion section are configured in groups and arranged in the corresponding lateral channels, respectively, and two of the sudden expansion sections are configured to intersect in the main channel.

14. The interventional catheter according to claim 13, wherein a distribution member is provided in the main channel, and at least two of the narrow openings are formed by the distribution member and side walls of the main channel and correspond to two expanded sections, respectively.

15. A high-viscosity fluid spraying system for natural cavity of human body, comprising an interventional catheter and a perfusion device for supplying a first fluid and a second fluid into the interventional catheter, respectively, wherein
the interventional catheter comprises a catheter body, one end of the catheter body is configured as a proximal end and another end of the catheter body is configured as a distal end that can extend into a bronchus, an interior of the catheter body is provided with a flow channel that is capable of transporting fluid from the proximal end to the distal end, a mixing structure is provided at the distal end of the catheter body, the fluid in the flow channel is output after being mixed by the mixing structure;
a flow mixing structure at least comprises a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure comprises:
a main channel, one end of which is connected to the first fluid inlet at the proximal end side and another end of which is connected to the outlet;
a lateral channel, one end of which is connected to a second fluid inlet at the lateral side and another end of which is connected to the main channel; and
a distribution structure capable of acting on at least a portion of the fluid being arranged in the chip channel structure.

16. The high-viscosity fluid spraying system according to claim 15, wherein a droplets size of the nebulized fluid is larger than or equal to 20µm, one of the first fluid and the second fluid is a liquid-phase fluid, and the other one of the first fluid and the second fluid is a gas-phase fluid, a pressure of the liquid-phase fluid is 0.2~0.76MPa, and a pressure of the gas-phase fluid is 0.1~0.4MPa.

17. The high-viscosity fluid spraying system according to claim 15, wherein the lateral channel is configured with a fluid acceleration structure which is located upstream of at least one intersection position of the main channel and the lateral channel.

18. The high-viscosity fluid spraying system according to claim 3, wherein the fluid in the lateral channel is gas, and the fluid acceleration structure is a Laval nozzle that is configured to converge gradually and expand gradually.

19. The high-viscosity fluid spraying system according to claim 18, wherein a portion of the main channel is a widened expansion section, at least a portion of the lateral channel intersects with the expansion section, a distribution member is provided in the expansion section, and the distribution member is provided with at least a first distribution surface and a second distribution surface facing towards a flowing direction of the fluid in the main channel.

20. The high-viscosity fluid spraying system according to claim 15, wherein the main channel is provided with a throat that is narrower than other portion of the main channel at the upstream and downstream of the throat, and a width W7 of the throat of the main channel is 0.1mm to 0.2mm.

21. The high-viscosity fluid spraying system according to claim 15, wherein a width W8 of a droplets outlet is 0.15mm to 0.3mm.

22. The high-viscosity fluid spraying system according to claim 15, wherein an etched depth H3 is 0.1mm to 0.25mm.

23. The high-viscosity fluid spraying system according to claim 15, wherein a thickness H1 of a unit chip with etched flow channel is 0.25mm to 0.55mm.

24. The high-viscosity fluid spraying system according to claim 15, wherein the high-viscosity fluid comprises a hydrogel.

25. A high-viscosity fluid spraying method for natural cavity of human body, wherein the fluid comprises a first fluid and a second fluid, one of the first fluid and the second fluid is a liquid-phase fluid, and the other one of the first fluid and the second fluid is a gas-phase fluid, and the high-viscosity fluid is in the liquid-phase fluid;
wherein the first fluid and the second fluid are nebulized into droplets with a particle size less than or equal to 60µm after at least two collisions, and the droplets contain inactivated viruses and move to a lesion location to infiltrate the lesion.

26. The high-viscosity fluid spraying method according to claim 25, wherein the particle size of nebulized droplets is larger than or equal to 20µm, a pressure of the liquid-phase fluid is 0.2~0.76MPa, and a pressure of the gas-phase fluid is 0.1~0.4MPa.

27. The high-viscosity fluid spraying method according to claim 26, wherein the particle size of nebulized droplets is in range of 20~60µm.

28. A cell spraying system for natural cavity of human body, comprising an interventional catheter and a perfusion device for supplying a first fluid and a second fluid to the interventional catheter, respectively, wherein
the interventional catheter comprises a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least comprises a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure comprises:
a main channel, one end of which is connected to the first fluid inlet at the proximal end side and another end of which is connected to the outlet;
a lateral channel, one end of which is connected to a second fluid inlet at the lateral side and another end of which is connected to the main channel; and
a distribution structure capable of acting on at least a portion of the fluid being arranged in the chip channel structure.

29. The cell spraying system according to claim 28, wherein a particle size of the cells is 15~120µm, one of the first fluid and the second fluid is a liquid-phase fluid and the other one of the first fluid and the second fluid is a gas-phase fluid, a pressure of the liquid-phase fluid is 0.2~0.76MPa, and a pressure of the gas-phase fluid is 0.1~0.4MPa.

30. The cell spraying system according to claim 28, wherein a fluid acceleration structure and a sudden expansion section are provided in the main channel and/or the lateral channel and arranged in sequence along a flowing direction of the fluid, an internal volume of the sudden expansion section is at least 15% larger than that of the fluid acceleration structure, and the fluids in the main channel and lateral channel meet and are nebulized in the sudden expansion section.

31. The cell spraying system according to claim 30, wherein the lateral channel is configured for transporting liquid, a distribution structure is provided at an end of the lateral channel close to the main channel, the distribution structure is configured as distribution members arranged in an array, and a cross-sectional size of each distribution member gradually increases along the flowing direction of the fluid.

32. The cell spraying system according to claim 28, wherein an etched depth H3 is 0.08mm to 0.12mm.

33. The cell spraying system according to claim 28, wherein a width W8 of a droplets outlet is 0.1mm to 0.2mm.

34. The cell spraying system according to claim 28, wherein a width W1 of the main channel is 0.2mm to 0.4mm.

35. The cell spraying system according to claim 28, wherein a particle size of droplets ranges from 5µm to 50µm.

36. The cell spraying system according to claim 28, wherein the cells comprise exosomes.

37. A cell spraying method for natural cavity of human body, comprising a first fluid and a second fluid, wherein one of the first fluid and the second fluid is a liquid-phase fluid and the other one of the first fluid and the second fluid is a gas-phase fluid, the cells are in the liquid-phase fluid, and a particle size of the cells is less than or equal to120µm;
the first fluid and the second fluid are nebulized into droplets with a particle size less than or equal to 120µm after at least two collisions, and the droplets contain inactivated viruses and move to a lesion location to infiltrate the lesion.

38. The cell spraying method according to claim 37, wherein the particle size of the cells is 15~120µm, a pressure of the liquid-phase fluid is 0.2~0.76MPa, and a pressure of the gas-phase fluid is 0.1~0.4MPa.

39. The cell spraying method according to claim 37, wherein a particle size of the droplets is in ranges of 20µm~50µm.

40. A medication spraying system for natural cavity of human body, comprising an interventional catheter and a perfusion device for supplying a first fluid and a second fluid to the interventional catheter, respectively, wherein
the interventional catheter comprises a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least comprises a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure comprises:
a main channel, one end of which is connected to the first fluid inlet at the proximal end side and another end of which is connected to the outlet;
a lateral channel, one end of which is connected to a second fluid inlet at the lateral side and another end of which is connected to the main channel; and
a distribution structure capable of acting on at least a portion of the fluid being arranged in the chip channel structure.

41. The medication spraying system according to claim 40, wherein one of the first fluid and the second fluid is a liquid-phase fluid, and the other one of the first fluid and the second fluid is a gas-phase fluid, the medication is in the liquid-phase fluid, a particle size of the medication is less than 69µm, a pressure of the liquid-phase fluid is 0.2~0.76MPa, and a pressure of the gas-phase fluid is 0.2~0.6MPa.

42. The medication spraying system according to claim 40, wherein the lateral channel and the main channel intersect with each other at least twice narrower than other portion of the main channel at the upstream and downstream of the throat.

43. The medication spraying system according to claim 42, wherein a portion of the main channel is configured as a widened expansion section, the lateral channel intersects with the main channel at least once at the expansion section, and the lateral channel intersects with the main channel at least once downstream of the expansion section.

44. The medication spraying system according to claim 40, wherein a width W1 of the main channel is 0.2 to 0.5mm.

45. The medication spraying system according to claim 40, wherein the main channel is provided with a throat that is narrower than the upstream and downstream, and a width W7 of the throat of the main channel is less than the width W1 of the main channel, and the width W7 of the throat of the main channel is preferably 0.05~0.15mm.

46. The medication spraying system according to claim 45, wherein a width W8 of a droplets outlet deviates from the width W7 of the throat of the main channel by 5%~15%.

47. A medication spraying method for natural cavity of human body, comprising a first fluid and a second fluid, wherein one of the first fluid and the second fluid is a liquid-phase fluid and the other one of the first fluid and the second fluid is a gas-phase fluid, the medication is in the liquid-phase fluid, and a particle size of the medication is less than or equal to 69000nm;
wherein the first fluid and the second fluid are nebulized into droplets with a particle size less than or equal to 300µm after at least two collisions, and the droplets contain inactivated viruses and move to a lesion location to infiltrate the lesion.

48. The medication spraying method according to claim 47, wherein a pressure of the liquid-phase fluid is 0.2~0.76MPa, a pressure of the gas-phase fluid is 0.2~0.6MPa; and the particle size of nebulized droplets is in range of 8~30µm.

49. An inactivated virus spraying system for natural cavity of human body, comprising an interventional catheter and a perfusion device for supplying a first fluid and a second fluid to the interventional catheter, respectively, wherein
the interventional catheter comprises a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least comprises a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure comprises:
a main channel, one end of which is connected to the first fluid inlet at the proximal end side and another end of which is connected to the outlet;
a lateral channel, one end of which is connected to a second fluid inlet at the lateral side and another end of which is connected to the main channel; and
a distribution structure capable of acting on at least a portion of the fluid being arranged in the chip channel structure.

50. The inactivated virus spraying system according to claim 49, wherein one of the first fluid and the second fluid is a liquid-phase fluid and the other one of the first fluid and the second fluid is a liquid-phase fluid is a gas-phase fluid, the inactivated virus is in the liquid-phase fluid, a particle size of the inactivated virus is less than 21µm, a pressure of the liquid-phase fluid is 0.2~0.76MPa, and a pressure of the gas-phase fluid is 0.2~0.6MPa.

51. The inactivated virus spraying system according to claim 49,wherein there are two lateral channels provided at two sides of the main channel, respectively, each lateral channel is provided with an independent branch channel which comprises multiple sub-channels arranged in an array, the multiple sub-channels of two branch channels are arranged opposite to each other and connected to the main channel, and wherein the multiple sub-channels of the two branch channels are arranged at intervals and staggered.

52. The inactivated virus spraying system according to claim 49, wherein a width W8 of a droplets outlet is 0.1~0.2mm.

53. An inactivated viruses spraying method for natural cavity of human body, comprising a first fluid and a second fluid, wherein one of the first fluid and the second fluid is a liquid-phase fluid and the other one of the first fluid and the second fluid is a gas-phase fluid, the inactivated virus is in the liquid-phase fluid, and a particle size of the inactivated virus is less than or equal to 21000nm;
the first fluid and the second fluid are nebulized into droplets with a particle size less than or equal to 300µm after at least two collisions, and the droplets contain inactivated viruses and move to a lesion location to infiltrate the lesion.

54. The inactivated virus spraying method according to claim 53, wherein a pressure of the liquid-phase fluid is 0.2~0.76MPa, and a pressure of the gas-phase fluid is 0.2~0.6MPa.

55. The inactivated virus spraying method according to claim 53, wherein a particle size of nebulized droplets is in range of 5~40µm.

56. A nebulization method based on interventional catheter, wherein a microfluidic chip is provided at a distal end of the interventional catheter, and the nebulization method comprises:
supplying a first fluid and a second fluid into the interventional catheter respectively, wherein at least one of the first fluid and second fluid is loaded with a therapeutic substance;
flowing of the first fluid and second fluid into the microfluidic chip from the proximal end side and the lateral side of the microfluidic chip, respectively, wherein at least one of the first fluid and second fluid is pre-dispersed in the microfluidic chip and then mixed with the other one of the first fluid and second fluid and nebulized, and then output to a lesion position;
obtaining current state parameters of the fluid during the nebulization process and generating a corresponding control signal based on the state parameters;
regulating a supply of the fluid accordingly the control signal.

57. A nebulized medication delivery system, comprising an interventional catheter and a perfusion device for supplying a first fluid and a second fluid to the interventional catheter, respectively, wherein
the interventional catheter comprises a catheter body, one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least comprises a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure comprises:
a main channel, one end of which is connected to the first fluid inlet at the proximal end side and another end of which is connected to the outlet;
a lateral channel, one end of which is connected to a second fluid inlet at the lateral side and another end of which is connected to the main channel; and
a distribution structure capable of acting on at least a portion of the fluid being arranged in the chip channel structure.

58. A control method for nebulized medication delivery system, wherein the nebulized medication delivery system comprises an interventional catheter, a microfluidic chip is provided at a distal end of the interventional catheter, and the control method comprises:
Step S510, supplying a gas-phase fluid first when the nebulized medication delivery system is started, wherein an adjustment target of a pressure of the gas-phase fluid is divided into at least two stages, each stage is independently configured with a PID parameter; collecting a first real-time pressure of the gas-phase fluid when the gas-phase fluid is supplied, determining the adjustment target stage to which the first real-time pressure belongs, and adjusting the pressure of the gas-phase fluid based on the PID parameter corresponding to the adjustment target stage to which the first real-time pressure belongs;
Step S520, supplying a gas-phase fluid with a therapeutic substance, wherein the liquid-phase fluid and the gas-phase fluid enter the microfluidic chip from a proximal end side and a lateral side of the microfluidic chip, respectively, at least one of the liquid-phase fluid and gas-phase fluid is pre-dispersed in the microfluidic chip and then mixed with another one of the liquid-phase fluid and gas-phase fluid and nebulized, and then output to a lesion position;
Step S530, collecting a second real-time pressure of the gas-phase fluid during nebulization and output of the fluid, determining the adjustment target stage to which the second real-time pressure belongs, and adjusting the pressure of the gas-phase fluid based on the PID parameter corresponding to the adjustment target stage to which the second real-time pressure belongs; and
Step S540, looping step S530 at a preset interval.

59. An interventional catheter, comprising a catheter body, wherein one end of the catheter body is configured as a proximal end and another end of the catheter body is configured as a distal end that is capable of extending into a bronchus, an interior of the catheter body is configured with a flow channel that is capable of transporting fluid from the proximal end to the distal end, a mixing structure is provided at the distal end of the catheter body, and the fluid in the flow channel is output after being mixed by the mixing structure;
a fluid mixing structure at least comprises a first fluid inlet, a second fluid inlet and an outlet, and each fluid inlet is independently configured with a flow channel in the catheter body;
a microfluidic chip is arranged in the fluid mixing structure, the first fluid inlet is located at a proximal end side of the microfluidic chip, the second fluid inlet is located at a lateral side of the microfluidic chip, an interior of the microfluidic chip is configured as a chip channel structure that interconnects the outlet and each fluid inlet, wherein the chip channel structure comprises:
a main channel, one end of which is connected to the first fluid inlet at the proximal end side and another end of which is connected to the outlet;
a lateral channel, one end of which is connected to a second fluid inlet at the lateral side and another end of which is connected to the main channel; and
a fluid acceleration structure and a sudden expansion section being provided in the main channel and/or the lateral channel and arranged in sequence along a flowing direction of the fluid, an internal volume of the sudden expansion section being at least 15% larger than that of the fluid acceleration structure, and the fluids in the main channel and lateral channel meeting and nebulized in the sudden expansion section.

60. The interventional catheter according to claim 59, wherein the sudden expansion section is formed by an expansion of a flow channel in which the sudden expansion section is located, and a smooth transition or a stepped transition is formed between the sudden expansion section and an outlet of the fluid acceleration structure.

61. The interventional catheter according to claim 59, wherein, in a flowing direction of a corresponding flow channel, an expansion trend of the sudden expansion section increases, maintains or shrinks compared to an expansion trend of the fluid acceleration structure.

62. The interventional catheter according to claim 59, wherein the sudden expansion section is arranged close to the fluid acceleration structure, and a chamber diameter of the sudden expansion section is at least 10% larger than that of the outlet of the fluid acceleration structure.

63. The interventional catheter according to claim 59, wherein at least one narrow opening is provided at an intersection position of the main channel and the lateral channel, and the main channel and the lateral channel intersect with each other through the narrow opening.

64. The interventional catheter according to claim 59, wherein there are two lateral channels arranged at two sides of the main channel, respectively, the fluid acceleration structure and the sudden expansion section are configured in groups and arranged in corresponding lateral channels, respectively, and two of the sudden expansion sections are configured to intersect in the main channel.

65. The interventional catheter according to claim 59, wherein a distribution structure capable of acting on at least a portion of the fluid is provided in the chip channel structure, wherein the distribution structure is configured as distribution members located in the main channel and/or the lateral channel, and the distribution members guide a flowing direction of the fluid of one of the main channel and lateral channel in which the distribution members are located to be perpendicular to a flowing direction of the fluid in another one of the main channel and lateral channel.

66. The interventional catheter according to claim 65, wherein the distribution members are provided in the main channel, and at least two of the narrow openings are formed by the distribution members and side walls of the main channel and correspond to two expanded sections, respectively.

67. The interventional catheter according to claim 59, wherein there are two lateral channels provided at two sides of the main channel, respectively, each lateral channel is provided with an independent branch channel which comprises multiple sub-channels arranged in an array.

68. The interventional catheter according to claim 67, wherein the multiple sub-channels of two branch channels are arranged opposite to each other and connected to the main channel.

69. The interventional catheter according to claim 67, wherein the multiple sub-channels of two branch channels are arranged in a staggered manner.

70. An interventional catheter, comprising:
a catheter body with one end thereof being configured as a proximal end and another end thereof being configured as a distal end and capable of extending into a bronchus;
a microfluidic chip being configured with a chip channel structure therein, wherein the chip channel structure comprises a main channel and a lateral channel, and fluids in the main channel and the lateral channel cooperate with each other to output nebulized fluid;
a heating module, located in the catheter body and at a distal end of the microfluidic chip, for adjusting a temperature of the nebulized fluid;
a balloon being deformably arranged outside the catheter body.

71. The interventional catheter according to claim 70, wherein the balloon and the heating module are positioned correspondingly in an axial direction of the catheter body.

72. The interventional catheter according to claim 70, wherein the distal end of the microfluidic chip is provided with an outlet for outputting the nebulized fluid, and the heating module is a heating tube, wherein an interior of the heating tube is a temperature control channel connected to the outlet.

73. The interventional catheter according to claim 72, wherein a ratio of an inner diameter and an axial length of the heating tube ranges from 0.05 to 0.3.

74. The interventional catheter according to claim 70, wherein a fluid acceleration structure and a sudden expansion section are provided in the main channel and/or the lateral channel and arranged in sequence along a flowing direction of the fluid, and fluids in the main channel and the lateral channel interact with each other in the sudden expansion section to output nebulized fluid.

75. The interventional catheter according to claim 70, wherein a liquid medium configured for nebulization in the microfluidic chip is water or saline and without any therapeutic medium.

76. The interventional catheter according to claim 70, wherein a fluid acceleration structure and a sudden expansion section are provided in the main channel and/or the lateral channel and arranged in sequence along a flowing direction of the fluid, and fluids in the main channel and the lateral channel interact with each other in the sudden expansion section to output nebulized fluid.

77. The interventional catheter according to claim 76, wherein the microfluidic chip comprises:
a first fluid inlet located at a proximal end side of the microfluidic chip;
a second fluid inlet located at a lateral side the microfluidic chip; and
an outlet located at a distal end side of the microfluidic chip;
wherein one end of the main channel is connected to the first fluid inlet and another end of the main channel is connected to the outlet; one end of the lateral channel is connected to the second fluid inlet and another end of the lateral channel is connected to the main channel; and a distribution structure which is capable of acting on at least a portion of the fluid to pre-disperse this portion of the fluid is provided in the chip channel structure.

78. The interventional catheter according to claim 76, wherein an internal volume of the sudden expansion section is at least 15% larger than that of the fluid acceleration structure, and the main channel and the lateral channel are connected in the sudden expansion section with a connection position being adjacent to the fluid acceleration structure.

79. The interventional catheter according to claim 76, wherein the fluid acceleration structure is formed by a gradually contracting and expanding of a flow channel in which the fluid acceleration structure is located.

80. The interventional catheter according to claim 76, wherein the sudden expansion section is formed by an expansion of the flow channel in which the sudden expansion section is located, and the sudden expansion section and an outlet of the fluid acceleration structure have a smooth transition or a step transition.

81. The interventional catheter according to claim 76, wherein the sudden expansion section is arranged close to the fluid acceleration structure, and a chamber diameter of the sudden expansion section is at least 10% larger than that of the outlet of the fluid acceleration structure.

82. The interventional catheter according to claim 70, wherein the main channel and the lateral channel intersect with each other at least twice along an extending direction of the main channel, wherein a first intersection is at the middle and upper reaches of the main channel for implementing turbulence, and a second intersection is adjacent to the outlet for implementing nebulization.

83. The interventional catheter according to claim 82, wherein there are two lateral channels respectively provided at two sides of the main channel, and a distribution structure is provided in the main channel, and the distribution structure and the side wall of the main channel form two narrow openings, and each narrow opening corresponds to the expanded section connecting the two lateral channels to form the second intersection.

84. The interventional catheter according to claim 76, wherein there are two lateral channels arranged at two sides of the main channel, respectively, the fluid acceleration structure and the sudden expansion section are configured in groups and arranged in corresponding lateral channels, respectively, and two of the sudden expansion sections are configured to intersect in the main channel.

85. A nebulized medication delivery system, comprising:
a perfusion device for supplying a fluid containing therapeutic substance, wherein the fluid comprises a liquid-phase fluid and a gas-phase fluid, and at least one of the liquid-phase fluid and gas-phase fluid contains the therapeutic substance;
an interventional catheter according to any one of claims 70 to 84, wherein a proximal end of the interventional catheter is connected to a perfusion device for receiving the gas-phase fluid and the liquid-phase fluid, respectively;
a sampling device for collecting state parameters of the fluids;
a controlling device connected to the sampling device, configured for receiving the state parameters and controlling the perfusion device according to the state parameters.

86. A gas-liquid interventional device, comprising:
a housing, on which a gas path connector and a liquid path connector are installed, a pump chamber being provided at a bottom of the housing;
an air pump installed in the pump chamber, wherein the air pump comprises a plurality of pumps connected in parallel and is connected to the gas path connector through a main pipe;
a cooling assembly thermally coupled to the air pump;
a syringe comprising a cylinder installed in a housing and a piston slidably installed in the cylinder, wherein the cylinder has an outlet and is connected to the liquid path connector;
a drive mechanism, linked to the piston of the syringe;
an interventional catheter according to any one of claims 70 to 84, wherein a proximal end of the interventional catheter is respectively connected to the gas path connector and the liquid path connector, for respectively receiving the gas-phase fluid and the liquid-phase fluid.

87. An interventional catheter, comprising a catheter body, wherein one end of the catheter body is configured as a proximal end, and another end of the catheter body is configured as a distal end that is capable of extending into a bronchus, an interior of the catheter body is provided with a microfluidic chip, wherein the microfluidic chip comprises:
a first fluid inlet located at a proximal end side of the microfluidic chip;
a second fluid inlet located at a lateral side of the microfluidic chip; and
an outlet located at a distal end side of the microfluidic chip;
wherein an interior of the microfluidic chip is configured as a chip channel structure which interconnects the outlet and each fluid inlet, and the chip channel structure comprises:
a main channel, one end of which is connected to the first fluid inlet and another end of which is connected to the outlet; and
a lateral channel with one end thereof being connected to the second fluid inlet and another end thereof being connected to the main channel, wherein the lateral channel is provided with a branch channel and intersects with the main channel via the branch channel.

88. The interventional catheter according to claim 87, wherein the lateral channel comprises:
a trunk channel extending from the second fluid inlet towards the distal end until it intersects with the main channel;
the branch channel with one end thereof being connected to the trunk channel at a position being adjacent to the second fluid inlet and another end thereof extending toward the proximal end and branching into a plurality of sub-channels, distal ends of the sub-channels intersecting with each other at the middle and upper reaches of the main channel.

89. The interventional catheter according to claim 88, wherein the branch channel has a bending portion at the second fluid inlet and extends toward the distal end through the bending portion, and one end of the branch channel is connected to a proximal end side of the bending portion.

90. The interventional catheter according to claim 88, wherein the branch channel is configured as a comb in whole, and distal ends of the sub-channels are vertically connected to the main channel.

91. The interventional catheter according to claim 90, wherein there are two lateral channels arranged at two sides of the main channel, respectively, and distal ends of the sub-channels of the two lateral channels are staggered.

92. The interventional catheter according to claim 88, wherein there are two lateral channels arranged at two sides of the main channel, respectively, the trunk channels of the two lateral channels each intersect with the main channel to form a first intersection position and a second intersection position, and the two lateral channels each independently extend from the first intersection position and the second intersection position towards the outlet and intersect with each other at a position adjacent to the outlet.

93. The interventional catheter according to claim 92, wherein a distribution structure is provided in the main channel and adjacent to an outlet portion, and the distribution structure closes a middle portion of the main channel along a width direction of the main channel, narrow openings are formed on two sides between the distribution structure and side walls of the main channel, and the first intersection position and the second intersection position are located at corresponding narrow openings, respectively.

94. The interventional catheter according to claim 93, wherein the lateral channel is configured with a fluid acceleration structure and a sudden expansion section which are arranged in sequence along a flowing direction of the fluid, wherein the narrow opening is connected to the sudden expansion section at a position adjacent to the fluid acceleration structure.

95. The interventional catheter of claim 93, wherein a cross-section of the distribution member is triangular-shaped.

96. The interventional catheter according to claim 93, wherein the main channel extends straightly from the first fluid inlet to the distribution member, with a constant cross-section.

97. The interventional catheter according to claim 92, wherein an obtuse angle is formed between trend lines of the two lateral channels extending from the first intersection position and the second intersection position towards the outlet.

98. The interventional catheter according to claim 97, wherein the outlet has a tendency to expand, and an expansion angle corresponds to an angle between the trend lines.

99. A nebulization method based on microfluidic chip, comprising:
receiving a liquid-phase fluid through the microfluidic chip;
receiving a gas-phase fluid through the microfluidic chip, wherein the gas-phase fluid is divided into two paths and each path is independently guided to mix with the liquid-phase fluid to form two nebulized flows;
guiding the two nebulized flows to mix inside the microfluidic chip and then output.

100. The nebulization method according to claim 99, wherein at least a portion of each path of the gas-phase fluid is pre-diverted and mixed with the liquid-phase fluid at a relatively upstream location to cause turbulence; and a remaining portion of each path of the gas-phase fluid is mixed with the liquid-phase fluid at a relatively downstream location to cause nebulization.

101. The nebulization method according to claim 100, wherein an angle between flow trend lines of two nebulized flows before intersection is an obtuse angle.

102. A nebulized medication delivery system, comprising:
a perfusion device for supplying a fluid containing therapeutic substance, wherein the fluid comprises a liquid-phase fluid and a gas-phase fluid, and at least one of the liquid-phase fluid and gas-phase fluid contains the therapeutic substance;
an interventional catheter according to any one of claims 87 to 98, wherein a proximal end of the interventional catheter is connected to the perfusion device for receiving the gas-phase fluid and the liquid-phase fluid respectively;
a sampling device for collecting state parameters of the fluids;
a controlling device connected to the sampling device, configured for receiving the state parameters and controlling the perfusion device according to the state parameters.

103. A gas-liquid interventional device, comprising:
a housing, on which a gas path connector and a liquid path connector are installed, a pump chamber being provided at a bottom of the housing;
an air pump is installed in the pump chamber, wherein the air pump comprises a plurality of pumps connected in parallel and is connected to the gas path connector through a main pipe;
a cooling assembly thermally coupled to the air pump;
a syringe comprising a cylinder installed in a housing and a piston slidably installed in the cylinder, wherein the cylinder has an outlet and is connected to the liquid path connector;
a drive mechanism, linked to the piston of the syringe;
an interventional catheter according to any one of claims 87 to 98, wherein a proximal end of the interventional catheter is respectively connected to the gas path connector and the liquid path connector, for respectively receiving the gas-phase fluid and the liquid-phase fluid.

104. An interventional catheter, comprising a catheter body, wherein one end of the catheter body is configured as a proximal end, and another end of the catheter body is configured as a distal end that is capable of extending into a bronchus, an interior of the catheter body is provided with a microfluidic chip, wherein the microfluidic chip comprises:
a first fluid inlet located at a proximal end side of the microfluidic chip;
two second fluid inlets arranged at two opposite sides of the microfluidic chip, respectively; and
an outlet located at a distal end side of the microfluidic chip;
wherein an interior of the microfluidic chip is configured with a chip channel structure connecting the outlet and each fluid inlet, wherein the chip channel structure comprises:
a main channel, one end of which is connected to the first fluid inlet and another end of which is connected to the outlet; and
a lateral channel with one end thereof being connected to the second fluid inlet and another end thereof being connected to the main channel, wherein a plurality of spaced distribution members are provided in the lateral channel.

105. The interventional catheter according to claim 104, wherein there are two lateral channels arranged at two sides of the main channel, respectively, and each lateral channel is connected to the middle and downstream of the main channel.

106. The interventional catheter according to claim 105, wherein the lateral channel has a bending portion pointing to the distal end at the second fluid inlet, and a side at an end of the bending portion facing the main channel is connected to the main channel through an intersection section; the distribution members are sequentially arranged in the intersection section along a length direction of the main channel.

107. The interventional catheter according to claim 106, wherein a fluid acceleration structure and a sudden expansion section are provided in the main channel and arranged in sequence along a flowing direction of the fluid, wherein the intersection section is connected to the sudden expansion section at a position adjacent to the fluid acceleration structure.

108. The interventional catheter according to claim 105, wherein the intersection section comprises multiple stages of narrowing parts in sequence, and the distribution structure is located between two adjacent stages of narrowing parts.

109. The interventional catheter according to claim 108, wherein the narrowing parts comprise three stages, and the distribution member is located between the first and second stages of narrowing parts.

110. The interventional catheter according to claim 108, wherein an overall extending direction of the intersection section is along a width of the main channel, each narrowing part has two opposite side walls, and at least one of the side walls is inclined relative to the extending direction, so that the narrowing part gradually shrinks.

111. The interventional catheter according to claim 110, wherein one of the two side walls is parallel to the extending direction, and the other one of the two side walls is inclined relative to the extending direction.

112. The interventional catheter according to claim 111, wherein, in the two most downstream stages, the inclined side walls are on different sides of the flow channel.

113. The interventional catheter according to claim 110, wherein a variation in the width of the flow channel between the front and rear of each narrowing part is 20~60%.

114. The interventional catheter according to claim 104, wherein the distribution members are configured in shape of pointed teeth, and a cross-sectional size of each distribution member gradually increases along the flowing direction of the fluid.

115. The interventional catheter according to claim 114, wherein the distribution members occupy 30-70% of the width of the flow channel at a downstream side of the distribution members.

116. A nebulized medication delivery system, comprising:
a perfusion device for supplying a fluid containing therapeutic substance, wherein the fluid comprises a liquid-phase fluid and a gas-phase fluid, and at least one of the liquid-phase fluid and gas-phase fluid contains the therapeutic substance;
an interventional catheter according to any one of claims 104 to 115, wherein a proximal end of the interventional catheter is connected to a perfusion device for receiving the gas-phase fluid and the liquid-phase fluid respectively;
a sampling device for collecting state parameters of the fluids;
a controlling device connected to the sampling device, configured for receiving the state parameters and controlling the perfusion device accordingly.

117. A gas-liquid interventional device, comprising:
a housing, on which a gas path connector and a liquid path connector are installed, and a pump chamber being provided at a bottom of the housing;
an air pump installed in the pump chamber, wherein the air pump comprises a plurality of pumps connected in parallel and is connected to the gas path connector through a main pipe;
a cooling assembly thermally coupled to the air pump;
a syringe comprising a cylinder installed in a housing and a piston slidably installed in the cylinder, wherein the cylinder has an outlet and is connected to the liquid path connector;
a drive mechanism, linked to the piston of the syringe;
an interventional catheter according to any one of claims 104 to 115, wherein a proximal end of the interventional catheter is connected to the gas path connector and the liquid path connector, respectively, for receiving the gas-phase fluid and the liquid-phase fluid, respectively.

118. A nebulized medication delivery system, comprising:
a perfusion device for supplying a fluid containing therapeutic substance, wherein the fluid comprises a liquid-phase fluid and a gas-phase fluid, at least one of the liquid-phase fluid and gas-phase fluid contains the therapeutic substance, and the perfusion device comprises a primary heating device for heating at least one of the liquid-phase fluid and gas-phase fluid;
an interventional catheter, a proximal end of which is in connected to the perfusion device for receiving the gas-phase fluid and the liquid-phase fluid respectively, and the interventional catheter is provided with a secondary heating device at a distal end thereof;
a sampling device for collecting state parameters of the fluids;
a controlling device connected to the sampling device, configured for receiving the state parameters and controlling the perfusion device according to the state parameters.

119. The nebulizer medication delivery system according to claim 118, wherein the interventional catheter further comprises a tertiary heating device located at the proximal end thereof.
